# EUROPEAN PATENT SPECIFICATION

(11) **EP 2 908 814 B1**
(45) Date of publication and mention of the grant of the patent: **25.12.2019**
(21) Application number: 13824539.4
(22) Date of filing: 17.10.2013
(51) Int. Cl.: A61K 31/4985, A61P 35/00

(54) **TREATMENT OF PROSTATE CANCER WITH TOR KINASE INHIBITORS**
BEHANDLUNG VON PROSTATAKREBS MIT TOR-KINASE-HEMMERN
TRAITEMENT DU CANCER DE LA PROSTATE AVEC DES INHIBITEURS DE KINASE TOR

(30) Priority: 18.10.2012 US 201261715510 P
(43) Date of publication of application: 26.08.2015
(73) Proprietor: Signal Pharmaceuticals, LLC, San Diego, CA 92121 (US)
(72) Inventor: MORTENSEN, Deborah, San Diego, CA 92117 (US); RAYMON, Heather, San Diego, CA 92117 (US); NARLA, Rama, K., San Diego, CA 92130 (US); HEGE, Kristen, Mae, Burlingame, CA 94010 (US); FULTZ, Kimberly, Elizabeth, San Diego, CA 92130 (US); TSUJI, Toshiya, San Diego, CA 92117 (US)
(74) Representative: Jones Day
(86) International application number: PCT/US2013/065363
(87) International publication number: WO 2014/062878

(56) References cited:
- US-A1- 2007 238 745
- US-B2- 7 981 893
- US-B2- 8 110 578
- YU K ET AL: "DEREGULATED P13K/AKT/TOR PATHWAY IN PTEN-DEFICIENT TUMOR CELLS CORRELATES WITH AN INCREASED GROWTH INHIBITION SENSITIVITY TO A TOR KINASE INHIBITOR CCI-779", AMERICAN ASSOCIATION FOR CANCER RESEARCH. PROCEEDINGS OF THE ANNUAL MEETING, AMERICAN ASSOCIATION FOR CANCER RESEARCH, US, vol. 42, 24 March 2001 (2001-03-24), page 802, XP001204671, ISSN: 0197-016X

## Description

### FIELD

Disclosed herein are methods for treating or preventing prostate cancer, comprising administering an effective amount of a TOR kinase inhibitor to a patient having prostate cancer.

### BACKGROUND

The connection between abnormal protein phosphorylation and the cause or consequence of diseases has been known for over 20 years. Accordingly, protein kinases have become a very important group of drug targets. *See* Cohen, Nature, 1:309-315 (2002). Various protein kinase inhibitors have been used clinically in the treatment of a wide variety of diseases, such as cancer and chronic inflammatory diseases, including diabetes and stroke. *See* Cohen, Eur. J. Biochem., 268:5001-5010 (2001), Protein Kinase Inhibitors for the Treatment of Disease: The Promise and the Problems, Handbook of Experimental Pharmacology, Springer Berlin Heidelberg, 167 (2005).

The protein kinases are a large and diverse family of enzymes that catalyze protein phosphorylation and play a critical role in cellular signaling. Protein kinases may exert positive or negative regulatory effects, depending upon their target protein. Protein kinases are involved in specific signaling pathways which regulate cell functions such as, but not limited to, metabolism, cell cycle progression, cell adhesion, vascular function, apoptosis, and angiogenesis. Malfunctions of cellular signaling have been associated with many diseases, the most characterized of which include cancer and diabetes. The regulation of signal transduction by cytokines and the association of signal molecules with protooncogenes and tumor suppressor genes have been well documented. Similarly, the connection between diabetes and related conditions, and deregulated levels of protein kinases, has been demonstrated. *See e.g.,* Sridhar et al. Pharmaceutical Research, 17(11):1345-1353 (2000). Viral infections and the conditions related thereto have also been associated with the regulation of protein kinases. Park et al. Cell 101 (7): 777-787 (2000).

Because protein kinases regulate nearly every cellular process, including metabolism, cell proliferation, cell differentiation, and cell survival, they are attractive targets for therapeutic intervention for various disease states. For example, cell-cycle control and angiogenesis, in which protein kinases play a pivotal role are cellular processes associated with numerous disease conditions such as but not limited to cancer, inflammatory diseases, abnormal angiogenesis and diseases related thereto, atherosclerosis, macular degeneration, diabetes, obesity, and pain.

Protein kinases have become attractive targets for the treatment of cancers. Fabbro et al., Pharmacology & Therapeutics 93:79-98 (2002). It has been proposed that the involvement of protein kinases in the development of human malignancies may occur by: (1) genomic rearrangements (*e.g.,* BCR-ABL in chronic myelogenous leukemia), (2) mutations leading to constitutively active kinase activity, such as acute myelogenous leukemia and gastrointestinal tumors, (3) deregulation of kinase activity by activation of oncogenes or loss of tumor suppressor functions, such as in cancers with oncogenic RAS, (4) deregulation of kinase activity by over-expression, as in the case of EGFR and (5) ectopic expression of growth factors that can contribute to the development and maintenance of the neoplastic phenotype. Fabbro et al., Pharmacology & Therapeutics 93:79-98 (2002). US 8,110,578 relates to certain pyrazino[2,3-b]pyrazine mTOR kinase inhibitors for treating or preventing cancer, inflammatory conditions, immunological conditions, neurodegenerative diseases, diabetes, obesity, neurological disorders, age-related diseases, or cardiovascular conditions.

The elucidation of the intricacy of protein kinase pathways and the complexity of the relationship and interaction among and between the various protein kinases and kinase pathways highlights the importance of developing pharmaceutical agents capable of acting as protein kinase modulators, regulators or inhibitors that have beneficial activity on multiple kinases or multiple kinase pathways. Accordingly, there remains a need for new kinase modulators.

The protein named mTOR (mammalian target of rapamycin), which is also called FRAP, RAFTI or RAPT1), is a 2549-amino acid Ser/Thr protein kinase, that has been shown to be one of the most critical proteins in the mTOR/PI3K/Akt pathway that regulates cell growth and proliferation. Georgakis and Younes Expert Rev. Anticancer Ther. 6(1):131-140 (2006). mTOR exists within two complexes, mTORC1 and mTORC2. While mTORC1 is sensitive to rapamycin analogs (such as temsirolimus or everolimus), mTORC2 is largely rapamycin-insensitive. Notably, rapamycin is not a TOR kinase inhibitor. Several mTOR inhibitors have been or are being evaluated in clinical trials for the treatment of cancer. Temsirolimus was approved for use in renal cell carcinoma in 2007 and sirolimus was approved in 1999 for the prophylaxis of renal transplant rejection. Everolimus was approved in 2009 for renal cell carcinoma patients that have progressed on vascular endothelial growth factor receptor inhibitors, in 2010 for subependymal giant cell astrocytoma (SEGA) associated with tuberous sclerosis (TS) in patients who require therapy but are not candidates for surgical resection, and in 2011 for progressive neuroendocrine tumors of pancreatic origin (PNET) in patients with unresectable, locally advanced or metastatic disease. There remains a need for additional TOR kinase inhibitors.

Citation or identification of any reference in this application is not to be construed as an admission that the reference is prior art to the present application.

### SUMMARY

Provided herein is a TOR kinase inhibitor for use in methods for treating prostate cancer as defined in the claims.

In certain examples, there are methods for improving the Prostate-Specific Antigen Working Group 2 (PSAWG2) Criteria for prostate cancer of a patient, comprising administering an effective amount of a TOR kinase inhibitor to a patient having prostate cancer.

The TOR kinase inhibitor for uses of the present invention is 1-ethyl-7-(2-methyl-6-(1H-1,2,4-triazol-3-yl)pyridin-3-yl)-3,4-dihydropyrazino[2,3-b]pyrazin-2(1H)-one, the prostate cancer for the purpose of the invention is not ETS overexpressing castration-resistant prostate cancer and the dosage for administration for the purpose of the invention is 0.5 mg/day to 128 mg/day.

The present embodiments can be understood more fully by reference to the detailed description and examples, which are intended to exemplify non-limiting embodiments.

### BRIEF DESCRIPTION OF THE DRAWINGS

FIG. 1 shows the cell cycle distribution of untreated HeLa and PC3 cells. (as reference)
FIG. 2 shows the effects of Compound 1 treatment on cell cycle distribution in PC3 cells. (as reference)

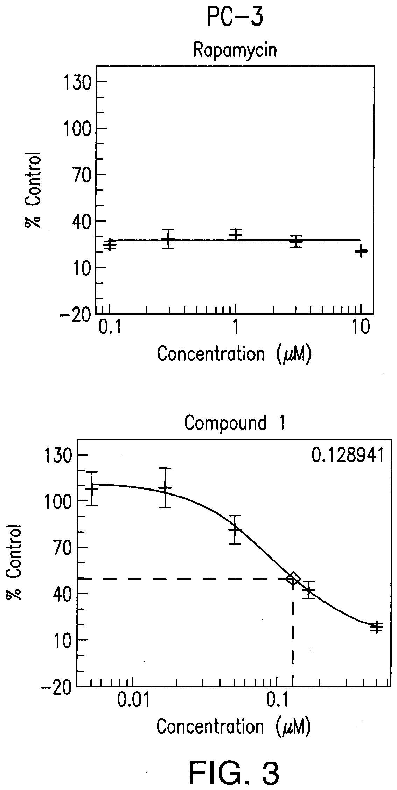

FIG. 3 shows growth inhibition curves for the PC3 cell line in response to either rapamycin or Compound 1 (as reference)
FIG. 4 shows the phospho-biomarker inhibition curve for the PC3 cell line in response to either rapamycin or Compound 1 treatment. (as reference)
FIG. 5A shows the antitumor activity of Compound 6 in PC3 xenograft model with various dosing schedules. (as reference)
FIG. 5B shows the antitumor activity of Compound 6 in PC3 xenograft model with once daily dosing at various dose levels. (as reference)
FIG. 6 shows the PK/PD relationship of Compound 6 in mice with PC3 tumors with a single oral dose of 30 mg/kg. The inhibition of pS6 and pAkt was correlated with the compounds levels in both plasma and tumors. (as reference)
FIG. 7 shows a growth inhibition curve for PC-3 in response to Compound 2.
FIG. 8 shows substrate phosphorylation inhibition curves in PC-3 in response to Compound 2 treatment.
FIG. 9 shows the antitumor activity of Compound 1 in the PC3 xenograft model with once daily dosing. (as reference)
FIG. 10 shows the comparison of Compound 1 (10 mg/kg) and rapamycin (4 mg/kg) exposure and relationship to pS6 and pAkt levels in the PC3 xenograft model following 21 days of dosing. (as reference)
FIG. 11 shows the antitumor activity of Compound 1 in the PC3 xenograft model with intermittent dosing. (as reference)
FIG. 12 shows the antitumor activity of Compound 1 in the PC3 xenograft model with twice daily dosing. (as reference)
FIG. 13 shows PC3 tumor regression with Compound 1 in the PC3 xenograft model with various dosing schedules. (as reference)
FIG. 14 shows Compound 1 exposure in PC3 tumor-bearing mice following a single oral dose of 25 mg/kg and the relationship to pS6 and pAkt levels. (as reference)
FIG. 15 shows Compound 1 exposure in PC3 tumor-bearing mice following a single oral dose of 10 mg/kg and the relationship to pS6 and pAkt levels. (as reference)
FIG. 16 shows Compound 1 exposure in PC3 tumor-bearing mice following a single oral dose of 1 mg/kg and the relationship to pS6 and pAkt levels. (as reference)
FIG. 17 shows the quantitation of apopotosis staining in PC3 tumors in response to either rapamycin or Compound 1 treatment. (as reference)
FIG. 18 shows the antiproliferative and anti-angiogenic effects as measured by quantitation of the Ki-67 and CD-31 staining in PC3 tumors in response to either rapamycin or Compound 1 treatment. (as reference)
FIG. 19 shows the antitumor activity of Compound 2 in the PC3 xenograft model with twice daily dosing.
FIG. 20 shows the antitumor activity of Compound 2 in the PC3 xenograft model with daily dosing at various dose levels.
FIG. 21 shows Compound 2 exposure in PC3 tumor-bearing mice following a single oral dose of 1 and 10 mg/kg and the relationship to pS6 and pAkt levels.
FIG. 22 shows effect on p-DNA PK levels in response to Compound 2 in PC3 tumor-bearing mice following daily dosing at 5 mg/kg for 6 days.

### DETAILED DESCRIPTION

### DEFINITIONS

An "alkyl" group is a saturated, partially saturated, or unsaturated straight chain or branched non-cyclic hydrocarbon having from 1 to 10 carbon atoms, typically from 1 to 8 carbons or, in some embodiments, from 1 to 6, 1 to 4, or 2 to 6 or carbon atoms. Representative alkyl groups include -methyl, -ethyl, -n-propyl, -n-butyl, -n-pentyl and -n-hexyl; while saturated branched alkyls include -isopropyl, -*sec*-butyl, -isobutyl, *-tert*-butyl, -isopentyl, 2-methylpentyl, 3-methylpentyl, 4-methylpentyl, 2,3-dimethylbutyl and the like. Examples of unsaturared alkyl groups include, but are not limited to, vinyl, allyl, -CH=CH(CH₃), -CH=C(CH₃)₂, -C(CH₃)=CH₂, -C(CH₃)=CH(CH₃), -C(CH₂CH₃)=CH₂, -C≡CH, -C≡C(CH₃), -C≡C(CH₂CH₃), -CH₂C≡CH,-CH₂C≡C(CH₃) and -CH₂C≡C(CH₇CH₃), among others. An alkyl group can be substituted or unsubstituted. In certain examples, when the alkyl groups described herein are said to be "substituted," they may be substituted with any substituent or substituents as those found in the exemplary compounds and examples disclosed herein, as well as halogen (chloro, iodo, bromo, or fluoro); hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonato; phosphine; thiocarbonyl; sulfonyl; sulfone; sulfonamide; ketone; aldehyde; ester; urea; urethane; oxime; hydroxyl amine; alkoxyamine; aralkoxyamine; N-oxide; hydrazine; hydrazide; hydrazone; azide; isocyanate; isothiocyanate; cyanate; thiocyanate; B(OH)₂, or O(alkyl)aminocarbonyl.

An "alkenyl" group is a straight chain or branched non-cyclic hydrocarbon having from 2 to 10 carbon atoms, typically from 2 to 8 carbon atoms, and including at least one carbon-carbon double bond. Representative straight chain and branched (C₂-C₈)alkenyls include -vinyl, -allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, -2-octenyl, -3-octenyl and the like. The double bond of an alkenyl group can be unconjugated or conjugated to another unsaturated group. An alkenyl group can be unsubstituted or substituted.

A "cycloalkyl" group is a saturated, partially saturated, or unsaturated cyclic alkyl group of from 3 to 10 carbon atoms having a single cyclic ring or multiple condensed or bridged rings which can be optionally substituted with from 1 to 3 alkyl groups. In some examples, the cycloalkyl group has 3 to 8 ring members, whereas in other embodiments the number of ring carbon atoms ranges from 3 to 5, 3 to 6, or 3 to 7. Such cycloalkyl groups include, by way of example, single ring structures such as cyclopropyl, cyclobutyl, cyclopentyl, cyclohexyl, cycloheptyl, cyclooctyl, 1-methylcyclopropyl, 2-methylcyclopentyl, 2-methylcyclooctyl, and the like, or multiple or bridged ring structures such as adamantyl and the like. Examples of unsaturared cycloalkyl groups include cyclohexenyl, cyclopentenyl, cyclohexadienyl, butadienyl, pentadienyl, hexadienyl, among others. A cycloalkyl group can be substituted or unsubstituted. Such substituted cycloalkyl groups include, by way of example, cyclohexanone and the like.

An "aryl" group is an aromatic carbocyclic group of from 6 to 14 carbon atoms having a single ring (*e.g.,* phenyl) or multiple condensed rings (*e.g.,* naphthyl or anthryl). In some examples, aryl groups contain 6-14 carbons, and in others from 6 to 12 or even 6 to 10 carbon atoms in the ring portions of the groups. Particular aryls include phenyl, biphenyl, naphthyl and the like. An aryl group can be substituted or unsubstituted. The phrase "aryl groups" also includes groups containing fused rings, such as fused aromatic-aliphatic ring systems (e.g., indanyl, tetrahydronaphthyl, and the like).

A "heteroaryl" group is an aryl ring system having one to four heteroatoms as ring atoms in a heteroaromatic ring system, wherein the remainder of the atoms are carbon atoms. In some examples, heteroaryl groups contain 5 to 6 ring atoms, and in others from 6 to 9 or even 6 to 10 atoms in the ring portions of the groups. Suitable heteroatoms include oxygen, sulfur and nitrogen. In certain examples, the heteroaryl ring system is monocyclic or bicyclic. Non-limiting examples include but are not limited to, groups such as pyrrolyl, pyrazolyl, imidazolyl, triazolyl, tetrazolyl, oxazolyl, isoxazolyl, thiazolyl, pyrolyl, pyridyl, pyridazinyl, pyrimidinyl, pyrazinyl, thiophenyl, benzothiophenyl, furanyl, benzofuranyl (for example, isobenzofuran-1,3-diimine), indolyl, azaindolyl (for example, pyrrolopyridyl or 1H-pyrrolo[2,3-b]pyridyl), indazolyl, benzimidazolyl (for example, 1H-benzo[d]imidazolyl), imidazopyridyl (for example, azabenzimidazolyl, 3H-imidazo[4,5-b]pyridyl or 1H-imidazo[4,5-b]pyridyl), pyrazolopyridyl, triazolopyridyl, benzotriazolyl, benzoxazolyl, benzothiazolyl, benzothiadiazolyl, isoxazolopyridyl, thianaphthalenyl, purinyl, xanthinyl, adeninyl, guaninyl, quinolinyl, isoquinolinyl, tetrahydroquinolinyl, quinoxalinyl, and quinazolinyl groups.

A "heterocyclyl" is an aromatic (also referred to as heteroaryl) or non-aromatic cycloalkyl in which one to four of the ring carbon atoms are independently replaced with a heteroatom from the group consisting of O, S and N. In some examples, heterocyclyl groups include 3 to 10 ring members, whereas other such groups have 3 to 5, 3 to 6, or 3 to 8 ring members. Heterocyclyls can also be bonded to other groups at any ring atom (*i.e.,* at any carbon atom or heteroatom of the heterocyclic ring). A heterocyclylalkyl group can be substituted or unsubstituted. Heterocyclyl groups encompass unsaturated, partially saturated and saturated ring systems, such as, for example, imidazolyl, imidazolinyl and imidazolidinyl groups. The phrase heterocyclyl includes fused ring species, including those comprising fused aromatic and non-aromatic groups, such as, for example, benzotriazolyl, 2,3-dihydrobenzo[1,4]dioxinyl, and benzo[1,3]dioxolyl. The phrase also includes bridged polycyclic ring systems containing a heteroatom such as, but not limited to, quinuclidyl. Representative examples of a heterocyclyl group include, but are not limited to, aziridinyl, azetidinyl, pyrrolidyl, imidazolidinyl, pyrazolidinyl, thiazolidinyl, tetrahydrothiophenyl, tetrahydrofuranyl, dioxolyl, furanyl, thiophenyl, pyrrolyl, pyrrolinyl, imidazolyl, imidazolinyl, pyrazolyl, pyrazolinyl, triazolyl, tetrazolyl, oxazolyl, isoxazolyl, thiazolyl, thiazolinyl, isothiazolyl, thiadiazolyl, oxadiazolyl, piperidyl, piperazinyl, morpholinyl, thiomorpholinyl, tetrahydropyranyl (for example, tetrahydro-2H-pyranyl), tetrahydrothiopyranyl, oxathiane, dioxyl, dithianyl, pyranyl, pyridyl, pyrimidinyl, pyridazinyl, pyrazinyl, triazinyl, dihydropyridyl, dihydrodithiinyl, dihydrodithionyl, homopiperazinyl, quinuclidyl, indolyl, indolinyl, isoindolyl, azaindolyl (pyrrolopyridyl), indazolyl, indolizinyl, benzotriazolyl, benzimidazolyl, benzofuranyl, benzothiophenyl, benzthiazolyl, benzoxadiazolyl, benzoxazinyl, benzodithiinyl, benzoxathiinyl, benzothiazinyl, benzoxazolyl, benzothiazolyl, benzothiadiazolyl, benzo[1,3]dioxolyl, pyrazolopyridyl, imidazopyridyl (azabenzimidazolyl; for example, 1H-imidazo[4,5-b]pyridyl, or 1H-imidazo[4,5-b]pyridin-2(3H)-onyl), triazolopyridyl, isoxazolopyridyl, purinyl, xanthinyl, adeninyl, guaninyl, quinolinyl, isoquinolinyl, quinolizinyl, quinoxalinyl, quinazolinyl, cinnolinyl, phthalazinyl, naphthyridinyl, pteridinyl, thianaphthalenyl, dihydrobenzothiazinyl, dihydrobenzofuranyl, dihydroindolyl, dihydrobenzodioxinyl, tetrahydroindolyl, tetrahydroindazolyl, tetrahydrobenzimidazolyl, tetrahydrobenzotriazolyl, tetrahydropyrrolopyridyl, tetrahydropyrazolopyridyl, tetrahydroimidazopyridyl, tetrahydrotriazolopyridyl, and tetrahydroquinolinyl groups. Representative substituted heterocyclyl groups may be monosubstituted or substituted more than once, such as, but not limited to, pyridyl or morpholinyl groups, which are 2-, 3-, 4-, 5-, or 6-substituted, or disubstituted with various substituents such as those listed below.

A "cycloalkylalkyl" group is a radical of the formula: -alkyl-cycloalkyl, wherein alkyl and cycloalkyl are defined above. Substituted cycloalkylalkyl groups may be substituted at the alkyl, the cycloalkyl, or both the alkyl and the cycloalkyl portions of the group. Representative cycloalkylalkyl groups include but are not limited to cyclopentylmethyl, cyclopentylethyl, cyclohexylmethyl, cyclohexylethyl, and cyclohexylpropyl. Representative substituted cycloalkylalkyl groups may be mono- substituted or substituted more than once.

An "aralkyl" group is a radical of the formula: -alkyl-aryl, wherein alkyl and aryl are defined above. Substituted aralkyl groups may be substituted at the alkyl, the aryl, or both the alkyl and the aryl portions of the group. Representative aralkyl groups include but are not limited to benzyl and phenethyl groups and fused (cycloalkylaryl)alkyl groups such as 4-ethyl-indanyl.

A "heterocyclylalkyl" group is a radical of the formula: -alkyl-heterocyclyl, wherein alkyl and heterocyclyl are defined above. Substituted heterocyclylalkyl groups may be substituted at the alkyl, the heterocyclyl, or both the alkyl and the heterocyclyl portions of the group. Representative heterocylylalkyl groups include but are not limited to 4-ethyl-morpholinyl, 4-propylmorpholinyl, furan-2-yl methyl, furan-3-yl methyl, pyrdine-3-yl methyl, (tetrahydro-2H-pyran-4-yl)methyl, (tetrahydro-2H-pyran-4-yl)ethyl, tetrahydrofuran-2-yl methyl, tetrahydrofuran-2-yl ethyl, and indol-2-yl propyl.

A "halogen" is fluorine, chlorine, bromine or iodine.

A "hydroxyalkyl" group is an alkyl group as described above substituted with one or more hydroxy groups.

An "alkoxy" group is -O-(alkyl), wherein alkyl is defined above.

An "alkoxyalkyl" group is -(alkyl)-O-(alkyl), wherein alkyl is defined above.

An "amino" group is a radical of the formula: -NH₂.

An "alkylamino" group is a radical of the formula: -NH-alkyl or -N(alkyl)₂, wherein each alkyl is independently as defined above.

A "carboxy" group is a radical of the formula: -C(O)OH.

An "aminocarbonyl" group is a radical of the
formula: -C(O)N(R^{#})₂, -C(O)NH(R^{#}) or -C(O)NH₂, wherein each R^{#} is independently a substituted or unsubstituted alkyl, cycloalkyl, aryl, aralkyl, heterocyclyl or heterocyclyl group as defined herein.

An "acylamino" group is a radical of the formula: -NHC(O)(R^{#}) or -N(alkyl)C(O)(R^{#}), wherein each alkyl and R^{#} are independently as defined above.

An "alkylsulfonylamino" group is a radical of the formula: -NHSO₂(R^{#}) or -N(alkyl)SO₂(R^{#}), wherein each alkyl and R^{#} are defined above.

A "urea" group is a radical of the
formula: -N(alkyl)C(O)N(R^{#})₂, -N(alkyl)C(O)NH(R^{#}),-N(alkyl)C(O)NH₂, -NHC(O)N(R^{#})₂, -NHC(O)NH(R^{#}), or -NH(CO)NHR^{#}, wherein each alkyl and R^{#} are independently as defined above.

When the groups described herein, with the exception of alkyl group are said to be "substituted," they may be substituted with any appropriate substituent or substituents. Illustrative examples of substituents are those found in the exemplary compounds and embodiments disclosed herein, as well as halogen (chloro, iodo, bromo, or fluoro); alkyl; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonato; phosphine; thiocarbonyl; sulfonyl; sulfone; sulfonamide; ketone; aldehyde; ester; urea; urethane; oxime; hydroxyl amine; alkoxyamine; aralkoxyamine; N-oxide; hydrazine; hydrazide; hydrazone; azide; isocyanate; isothiocyanate; cyanate; thiocyanate; oxygen (=O); B(OH)₂, O(alkyl)aminocarbonyl; cycloalkyl, which may be monocyclic or fused or non-fused polycyclic (e.g., cyclopropyl, cyclobutyl, cyclopentyl, or cyclohexyl), or a heterocyclyl, which may be monocyclic or fused or non-fused polycyclic (e.g., pyrrolidyl, piperidyl, piperazinyl, morpholinyl, or thiazinyl); monocyclic or fused or non-fused polycyclic aryl or heteroaryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothiophenyl, or benzofuranyl) aryloxy; aralkyloxy; heterocyclyloxy; and heterocyclyl alkoxy.

As used herein, the term "pharmaceutically acceptable salt(s)" refers to a salt prepared from a pharmaceutically acceptable non-toxic acid or base including an inorganic acid and base and an organic acid and base. Suitable pharmaceutically acceptable base addition salts of the TOR kinase inhibitors include, but are not limited to metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts. Others are well-known in the art, see for example, Remington's Pharmaceutical Sciences, 18th eds., Mack Publishing, Easton PA (1990) or Remington: The Science and Practice of Pharmacy, 19th eds., Mack Publishing, Easton PA (1995).

As used herein and unless otherwise indicated, the term "clathrate" means a TOR kinase inhibitor, or a salt thereof, in the form of a crystal lattice that contains spaces (*e.g.,* channels) that have a guest molecule (*e.g.,* a solvent or water) trapped within or a crystal lattice wherein a TOR kinase inhibitor is a guest molecule.

As used herein and unless otherwise indicated, the term "solvate" means a TOR kinase inhibitor, or a salt thereof, that further includes a stoichiometric or non-stoichiometric amount of a solvent bound by non-covalent intermolecular forces. In one embodiment, the solvate is a hydrate.

As used herein and unless otherwise indicated, the term "hydrate" means a TOR kinase inhibitor, or a salt thereof, that further includes a stoichiometric or non-stoichiometric amount of water bound by non-covalent intermolecular forces.

As used herein and unless otherwise indicated, the term "prodrug" means a TOR kinase inhibitor derivative that can hydrolyze, oxidize, or otherwise react under biological conditions (*in vitro* or *in vivo*) to provide an active compound, particularly a TOR kinase inhibitor. Examples of prodrugs include, but are not limited to, derivatives and metabolites of a TOR kinase inhibitor that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. In certain examples, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger's Medicinal Chemistry and Drug Discovery 6th ed. (Donald J. Abraham ed., 2001, Wiley) and Design and Application of Prodrugs (H. Bundgaard ed., 1985, Harwood Academic Publishers Gmfh).

As used herein and unless otherwise indicated, the term "stereoisomer" or "stereomerically pure" means one stereoisomer of a TOR kinase inhibitor that is substantially free of other stereoisomers of that compound. For example, a stereomerically pure compound having one chiral center will be substantially free of the opposite enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, greater than about 90% by weight of one stereoisomer of the compound and less than about 10% by weight of the other stereoisomers of the compound, greater than about 95% by weight of one stereoisomer of the compound and less than about 5% by weight of the other stereoisomers of the compound, or greater than about 97% by weight of one stereoisomer of the compound and less than about 3% by weight of the other stereoisomers of the compound. The TOR kinase inhibitors can have chiral centers and can occur as racemates, individual enantiomers or diastereomers, and mixtures thereof. All such isomeric forms are included within the embodiments disclosed herein, including mixtures thereof. The use of stereomerically pure forms of such TOR kinase inhibitors, as well as the use of mixtures of those forms are encompassed by the embodiments disclosed herein. For example, mixtures comprising equal or unequal amounts of the enantiomers of a particular TOR kinase inhibitor may be used in methods and compositions disclosed herein. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

It should also be noted the TOR kinase inhibitors can include E and Z isomers, or a mixture thereof, and cis and trans isomers or a mixture thereof. In certain examples, the TOR kinase inhibitors are isolated as either the cis or trans isomer. In other examples, the TOR kinase inhibitors are a mixture of the cis and trans isomers.

"Tautomers" refers to isomeric forms of a compound that are in equilibrium with each other. The concentrations of the isomeric forms will depend on the environment the compound is found in and may be different depending upon, for example, whether the compound is a solid or is in an organic or aqueous solution. For example, in aqueous solution, pyrazoles may exhibit the following isomeric forms, which are referred to as tautomers of each other:

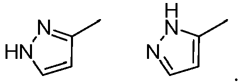

As readily understood by one skilled in the art, a wide variety of functional groups and other stuctures may exhibit tautomerism and all tautomers of the TOR kinase inhibitor for uses of the present invention are within the scope of the present invention.

It should also be noted the TOR kinase inhibitors can contain unnatural proportions of atomic isotopes at one or more of the atoms. For example, the compounds may be radiolabeled with radioactive isotopes, such as for example tritium (³H), iodine-125 (¹²⁵I), sulfur-35 (³⁵S), or carbon-14 (¹⁴C), or may be isotopically enriched, such as with deuterium (²H), carbon-13 (¹³C), or nitrogen-15 (¹⁵N). As used herein, an "isotopologue" is an isotopically enriched compound. The term "isotopically enriched" refers to an atom having an isotopic composition other than the natural isotopic composition of that atom. The term "isotopically enriched" may also refer to a compound containing at least one atom having an isotopic composition other than the natural isotopic composition of that atom. The term "isotopic composition" refers to the amount of each isotope present for a given atom. Radiolabeled and isotopically encriched compounds are useful as therapeutic agents, *e.g*., cancer and inflammation therapeutic agents, research reagents, *e.g*., binding assay reagents, and diagnostic agents, *e.g.,* in vivo imaging agents. All isotopic variations of the TOR kinase inhibitor for uses of the present invention, whether radioactive or not, are intended to be encompassed within the scope of the embodiments provided herein. In some embodiments, there are provided isotopologues of the TOR kinase inhibitor, for example, the isotopologues are deuterium, carbon-13, or nitrogen-15 enriched TOR kinase inhibitor.

"Treating" as used herein, means an alleviation, in whole or in part, of prostate cancer, or a symptom thereof, or slowing, or halting of further progression or worsening of prostate cancer.

"Preventing" as used herein, means the prevention of the onset, recurrence or spread, in whole or in part, of prostate cancer, or a symptom thereof.

The term "effective amount" in connection with an TOR kinase inhibitor means an amount capable of alleviating, in whole or in part, symptoms associated with prostate cancer, or slowing or halting further progression or worsening of those symptoms, or treating or preventing prostate cancer. The effective amount of the TOR kinase inhibitor, for example in a pharmaceutical composition, may be at a level that will exercise the desired effect; for example, about 0.005 mg/kg of a subject's body weight to about 100 mg/kg of a patient's body weight in unit dosage for both oral and parenteral administration. As will be apparent to those skilled in the art, it is to be expected that the effective amount of a TOR kinase inhibitor disclosed herein may vary depending on the severity of the indication being treated.

In the context of prostate cancer, treatment may be assessed by inhibition of disease progression, inhibition of tumor growth, reduction of primary and/or secondary tumor(s), relief of tumor-related symptoms, improvement in quality of life, inhibition of tumor secreted factors (including prostate specific antigen or PSA), reduction in circulating tumor cells, delayed appearance of primary and/or secondary tumor(s), slowed development of primary and/or secondary tumor(s), decreased occurrence of primary and/or secondary tumor(s), slowed or decreased severity of secondary effects of disease, arrested tumor growth and/or regression of tumors, among others.

The terms "patient" and "subject" as used herein include an animal, including, but not limited to, an animal such as a cow, monkey, horse, sheep, pig, chicken, turkey, quail, cat, dog, mouse, rat, rabbit or guinea pig, in one embodiment a mammal, in another embodiment a human. In one embodiment, a "patient" or "subject" is a human having prostate cancer. In one embodiment, a patient is a human having prostate cancer, including subjects who have progressed on (or not been able to tolerate) standard anticancer therapy or for whom no standard anticancer therapy exists.

In the context of prostate cancer, treatment may be assessed by inhibition of disease progression, inhibition of tumor growth, reduction of primary and/or secondary tumor(s), relief of tumor-related symptoms, improvement in quality of life, inhibition of tumor secreted factors (including prostate specific antigen or PSA), delayed appearance of primary and/or secondary tumor(s), slowed development of primary and/or secondary tumor(s), decreased occurrence of primary and/or secondary tumor(s), slowed or decreased severity of secondary effects of disease, arrested tumor growth and/or regression of tumors, among others. In certain embodiments, treatment of prostate cancer may be assessed by the inhibition of phosphorylation of S6RP, 4E-BP1 and/or AKT in circulating blood and/or tumor cells and/or skin biopsies or tumor biopsies/aspirates, before, during and/or after treatment with a TOR kinase inhibitor. In other embodiments, treatment of prostate cancer may be assessed by the inhibition of DNA-dependent protein kinase (DNA-PK) activity in skin samples and/or tumor biopsies/aspirates, such as by assessment of the amount of pDNA-PK S2056 as a biomarker for DNA damage pathways before, during, and/or after TOR kinase inhibitor treatment. In one embodiment, the skin sample is irradiated by UV light. In the extreme, complete inhibition, is referred to herein as prevention or chemoprevention. In this context, the term "prevention" includes either preventing the onset of clinically evident prostate cancer altogether or preventing the onset of a preclinically evident stage of prostate cancer. Also intended to be encompassed by this definition is the prevention of transformation into malignant cells or to arrest or reverse the progression of premalignant cells to malignant cells. This includes prophylactic treatment of those at risk of developing prostate cancer.

### TOR KINASE INHIBITORS

The compounds disclosed herein are generally referred to as "TOR kinase inhibitor(s)." In a specific example, the TOR kinase inhibitors do not include rapamycin or rapamycin analogs (rapalogs).

As disclosed herein, the TOR kinase inhibitors include compounds having the following formula (I): 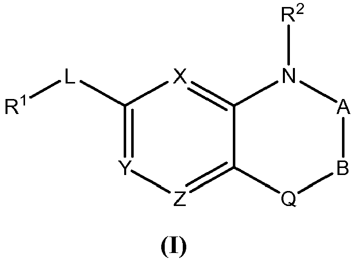 and pharmaceutically acceptable salts, clathrates, solvates, stereoisomers, tautomers, and prodrugs thereof, wherein:
X, Y and Z are at eac h occurrence independently N or CR3, wherein at least one of X, Y and Z is N and at least one of X, Y and Z is CR³;
-A-B-Q- taken together form -CHR⁴C(O)NH-, -C(O)CHR⁴NH-, -C(O)NH-, -CH₂C(O)O-, -C(O)CH₂O-, -C(O)O- or C(O)NR³;
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl;
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R³ is H, substituted or unsubstituted C₁₋₈alkyl, substituted or unsubstituted aryl, substituted or unsubstituted heteroaryl, substituted or unsubstituted cycloalkyl, substituted or unsubstituted heterocyclylalkyl, -NHR⁴ or -N(R⁴)₂; and
R⁴ is at each occurrence independently substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

Representative TOR kinase inhibitors of formula (I) as disclosed herein include:
(S)-1-(1-hydroxy-3-methylbutan-2-yl)-6-phenyl-1H-imidazo[4,5-b]pyrazin-2(3H)-one;
1-((tetrahydro-2H-pyran-4-yl)methyl)-6-(3,4,5-trimethoxyphenyl)-1H-imidazo[4,5-b]pyrazin-2(3H)-one;
(R)-6-(naphthalen-1-yl)-1-(1-phenylethyl)-1H-imidazo[4,5-b]pyrazin-2(3H)-one;
1-(3-methoxybenzyl)-6-(4-(methylsulfonyl)phenyl)-1H-imidazo[4,5-b]pyrazin-2(3H)-one;
(S)-1-(1-phenylethyl)-6-(quinolin-5-yl)-1H-imidazo[4,5-b]pyrazin-2(3H)-one;
6-(4-hydroxyphenyl)-1-((tetrahydro-2H-pyran-4-yl)methyl)-1H-imidazo[4,5-b]pyrazin-2(3H)-one;
(S)-6-(naphthalen-1-yl)-1-(1-phenylethyl)-1H-imidazo[4,5-b]pyrazin-2(3H)-one;
(S)-1-(1-hydroxy-3-methylbutan-2-yl)-6-(5-isopropyl-2-methoxyphenyl)-1H-imidazo[4,5-b]pyrazin-2(3H)-one;
(R)-1-(1-hydroxy-3-methylbutan-2-yl)-6-phenyl-1H-imidazo[4,5-b]pyrazin-2(3H)-one;
(R)-1-(1-phenylethyl)-6-(quinolin-5-yl)-1H-imidazo[4,5-b]pyrazin-2(3H)-one;
(S)-1-(1-hydroxy-3-methylbutan-2-yl)-6-(quinolin-5-yl)-1H-imidazo[4,5-b]pyrazin-2(3H)-one;
(R)-1-(1-hydroxy-3-methylbutan-2-yl)-6-(quinolin-5-yl)-1H-imidazo[4,5-b]pyrazin-2(3H)-one;
(R)-1-(1-hydroxy-3-methylbutan-2-yl)-6-(5-isopropyl-2-methoxyphenyl)-1H-imidazo[4,5-b]pyrazin-2(3H)-one;
1-benzyl-6-(quinolin-5-yl)-1H-imidazo[4,5-b]pyrazin-2(3H)-one;
1-(4-methoxybenzyl)-6-(quinolin-5-yl)-1H-imidazo[4,5-b]pyrazin-2(3H)-one;
(R)-1-(1-phenylethyl)-1H-imidazo[4,5-b]pyrazin-2(3H)-one;
(S)-1-(1-phenylethyl)-1H-imidazo[4,5-b]pyrazin-2(3H)-one;
1-isopropyl-6-(5-isopropyl-2-methoxyphenyl)-1H-imidazo[4,5-b]pyrazin-2(3H)-one;
1-cyclohexyl-6-(5-isopropyl-2-methoxyphenyl)-1H-imidazo[4,5-b]pyrazin-2(3H)-one;
5-(quinolin-5-yl)-1H-imidazo[4,5-b]pyrazin-2(3H)-one;
1-isobutyl-6-(5-isopropyl-2-methoxyphenyl)-1H-imidazo[4,5-b]pyrazin-2(3H)-one;
1-(2-hydroxyethyl)-6-(5-isopropyl-2-methoxyphenyl)-1H-imidazo[4,5-b]pyrazin-2(3H)-one;
6-(5-isopropyl-2-methoxyphenyl)-1-(tetrahydro-2H-pyran-4-yl)-1H-imidazo[4,5-b]pyrazin-2(3H)-one;
(R)-1-(1-phenylethyl)-6-(quinolin-5-yl)-1H-imidazo[4,5-c]pyridin-2(3H)-one;
(S)-1-(1-phenylethyl)-6-(quinolin-5-yl)-1H-imidazo[4,5-c]pyridin-2(3H)-one;
3-(1-phenylethyl)-5-(quinolin-5-yl)-1H-imidazo[4,5-b]pyridin-2(3H)-one;
(R)-3-(1-phenylethyl)-5-(quinolin-5-yl)-1H-imidazo[4,5-b]pyridin-2(3H)-one;
(R)-6-(5-isopropyl-2-methoxyphenyl)-1-(3-methylbutan-2-yl)-1H-imidazo[4,5-b]pyrazin-2(3H)-one;
(S)-6-(5-isopropyl-2-methoxyphenyl)-1-(tetrahydrofuran-3-yl)-1H-imidazo[4,5-b]pyrazin-2(3H)-one;
(S)-6-(5-isopropyl-2-methoxyphenyl)-1-(3-methylbutan-2-yl)-1H-imidazo[4,5-b]pyrazin-2(3H)-one;
1-cyclopentyl-6-(5-isopropyl-2-methoxyphenyl)-1H-imidazo[4,5-b]pyrazin-2(3H)-one;
(R)-6-(5 -isopropyl-2-methoxyphenyl)-1-(tetrahydrofuran-3-yl)-1H-imidazo[4,5-b]pyrazin-2(3H)-one;
1-(cyclopropylmethyl)-6-(5-isopropyl-2-methoxyphenyl)-1H-imidazo[4,5-b]pyrazin-2(3H)-one;
1-(cyclopentylmethyl)-6-(5-isopropyl-2-methoxyphenyl)-1H-imidazo[4,5-b]pyrazin-2(3H)-one;
1-(cyclohexylmethyl)-6-(5-isopropyl-2-methoxyphenyl)-1H-imidazo[4,5-b]pyrazin-2(3H)-one;
6-(5-isopropyl-2-methoxyphenyl)-1-neopentyl-1H-imidazo[4,5-b]pyrazin-2(3H)-one;
1-isopropyl-6-(3-isopropylphenyl)-1H-imidazo[4,5-b]pyrazin-2(3H)-one;
1-isopropyl-6-(2-methoxyphenyl)-1H-imidazo[4,5-b]pyrazin-2(3H)-one;
(S)-3-(1-hydroxy-3-methylbutan-2-yl)-5-(5-isopropyl-2-methoxyphenyl)-1H-imidazo[4,5-b]pyridin-2(3H)-one;
(R)-1-(2-hydroxy-1-phenylethyl)-6-(quinolin-5-yl)-1H-imidazo[4,5-b]pyrazin-2(3H)-one;
(S)-1-(2-hydroxy-1-phenylethyl)-6-(quinolin-5-yl)-1H-imidazo[4,5-b]pyrazin-2(3H)-one;
1-(1-phenylethyl)-6-(quinolin-5-yl)-1H-imidazo[4,5-b]pyrazin-2(3H)-one;
1-benzhydryl-6-(quinolin-5-yl)-1H-imidazo[4,5-b]pyrazin-2(3H)-one;
(S)-1-(1-phenylpropyl)-6-(quinolin-5-yl)-1H-imidazo [4,5-b]pyrazin-2(3H)-one;
(R)-1-(1-phenylpropyl)-6-(quinolin-5-yl)-1H-imidazo[4,5-b]pyrazin-2(3H)-one;
6-(5 -isopropyl-2-methoxyphenyl)-1-(tetrahydro-2H-pyran-3-yl)-1H-imidazo[4,5-b]pyrazin-2(3H)-one;
1-(3-methoxybenzyl)-6-(quinolin-5-yl)-1H-imidazo[4,5-b]pyrazin-2(3H)-one;
(R)-1-methyl-3-(1-phenylethyl)-5-(quinolin-5-yl)-1H-imidazo[4,5-b]pyrazin-2(3H)-one;
(S)-1-methyl-3-(1-phenylethyl)-5-(quinolin-5-yl)-1H-imidazo[4,5-b]pyrazin-2(3H)-one;
1-(cyclopentylmethyl)-6-(quinolin-5-yl)-1H-imidazo[4,5-b]pyrazin-2(3H)-one;
1-(1-(2-fluorophenyl)ethyl)-6-(quinolin-5-yl)-1H-imidazo[4,5-b]pyrazin-2(3H)-one;
1-(1-(4-fluorophenyl)ethyl)-6-(quinolin-5-yl)-1H-imidazo[4,5-b]pyrazin-2(3H)-one;
1-cyclopentyl-6-(quinolin-5-yl)-1H-imidazo[4,5-b]pyrazin-2(3H)-one;
1-(1-(3-fluorophenyl)ethyl)-6-(quinolin-5-yl)-1H-imidazo[4,5-b]pyrazin-2(3H)-one;
1-(1-(3-methoxyphenyl)ethyl)-6-(quinolin-5-yl)-1H-imidazo[4,5-b]pyrazin-2(3H)-one;
1-(1-(4-methoxyphenyl)ethyl)-6-(quinolin-5-yl)-1H-imidazo[4,5-b]pyrazin-2(3H)-one;
6-(quinolin-5-yl)-1-(tetrahydro-2H-pyran-4-yl)-1H-imidazo[4,5-b]pyrazin-2(3H)-one;
6-(quinolin-5-yl)-1-(tetrahydro-2H-pyran-3-yl)-1H-imidazo[4,5-b]pyrazin-2(3H)-one;
1-((1s,4s)-4-hydroxycyclohexyl)-6-(quinolin-5-yl)-1H-imidazo[4,5-b]pyrazin-2(3H)-one;
1-((1r,4r)-4-hydroxycyclohexyl)-6-(quinolin-5-yl)-1H-imidazo[4,5-b]pyrazin-2(3H)-one;
6-(isoquinolin-5-yl)-1-(1-phenylethyl)-1H-imidazo[4,5-b]pyrazin-2(3H)-one;
(R)-1-(1-phenylethyl)-6-(quinolin-5-yl)-1H-imidazo[4,5-b]pyridin-2(3H)-one;
1-(1-phenylethyl)-6-(quinolin-5-yl)-1H-imidazo[4,5-b]pyridin-2(3H)-one;
1-isopropyl-6-(quinolin-5-yl)-1H-imidazo[4,5-b]pyrazin-2(3H)-one;
1-(1-(4-chlorophenyl)ethyl)-6-(quinolin-5-yl)-1H-imidazo[4,5-b]pyrazin-2(3H)-one;
1-(1-(4-(methylsulfonyl)phenyl)ethyl)-6-(quinolin-5-yl)-1H-imidazo[4,5-b]pyrazin-2(3H)-one;
1-(1-(pyridin-4-yl)ethyl)-6-(quinolin-5-yl)-1H-imidazo[4,5-b]pyrazin-2(3H)-one;
5-methyl-1-((S)-1-phenylethyl)-6-(quinolin-5-yl)-1H-imidazo[4,5-b]pyrazin-2(3H)-one;
5-methyl-1-((R)-1-phenylethyl)-6-(quinolin-5-yl)-1H-imidazo[4,5-b]pyrazin-2(3H)-one;
1-(1-phenylethyl)-6-(quinolin-4-yl)-1H-imidazo[4,5-b]pyrazin-2(3H)-one;
6-(3-fluorophenyl)-1-(1-phenylethyl)-1H-imidazo[4,5-b]pyrazin-2(3H)-one;
6-(2-fluorophenyl)-1-(1-phenylethyl)-1H-imidazo[4,5-b]pyrazin-2(3H)-one;
1-(1-phenylethyl)-6-(quinolin-6-yl)-1H-imidazo[4,5-b]pyrazin-2(3H)-one;
1-(piperidin-4-ylmethyl)-6-(quinolin-5-yl)-1H-imidazo[4,5-b]pyrazin-2(3H)-one;
1-(1-(pyridin-2-yl)ethyl)-6-(quinolin-5-yl)-1H-imidazo[4,5-b]pyrazin-2(3H)-one;
1-(1-(pyridin-3-yl)ethyl)-6-(quinolin-5-yl)-1H-imidazo[4,5-b]pyrazin-2(3H)-one;
1-((1s,4s)-4-(hydroxymethyl)cyclohexyl)-6-(quinolin-5-yl)-1H-imidazo[4,5-b]pyrazin-2(3H)-one;
N-(4-(2-oxo-3-(1-phenylethyl)-2,3-dihydro-1H-imidazo[4,5-b]pyrazin-5-yl)phenyl)methanesulfonamide;
6-(3-(methylsulfonyl)phenyl)-1-(1-phenylethyl)-1H-imidazo[4,5-b]pyrazin-2(3H)-one;
6-(3-aminophenyl)-1-(1-phenylethyl)-1H-imidazo[4,5-b]pyrazin-2(3H)-one;
6-(3-(dimethylamino)phenyl)-1-(1-phenylethyl)-1H-imidazo[4,5-b]pyrazin-2(3H)-one;
1-phenyl-6-(quinolin-5-yl)-1H-imidazo[4,5-b]pyrazin-2(3H)-one;
1-(1-phenylethyl)-6-(4-(trifluoromethyl)phenyl)-1H-imidazo[4,5-b]pyrazin-2(3H)-one;
N-(3-(2-oxo-3-(1-phenylethyl)-2,3-dihydro-1H-imidazo[4,5-b]pyrazin-5-yl)phenyl)methanesulfonamide;
6-(4-(methylsulfonyl)phenyl)-1-(1-phenylethyl)-1H-imidazo[4,5-b]pyrazin-2(3H)-one;
3-(1-phenylethyl)-5-(quinolin-5-yl)oxazolo[5,4-b]pyrazin-2(3H)-one;
1-(cyclopentylmethyl)-6-(4-hydroxyphenyl)-1H-imidazo[4,5-b]pyrazin-2(3H)-one
6-(4-hydroxyphenyl)-1-isopropyl-1H-imidazo[4,5-b]pyrazin-2(3H)-one;
6-(4-hydroxyphenyl)-1-isobutyl-1H-imidazo[4,5-b]pyrazin-2(3H)-one;
6-(4-hydroxyphenyl)-1-((tetrahydro-2H-pyran-3-yl)methyl)-1H-imidazo[4,5-b]pyrazin-2(3H)-one;
1-(cyclohexylmethyl)-6-(4-hydroxyphenyl)-1H-imidazo[4,5-b]pyrazin-2(3H)-one;
5-(3-Hydroxyphenyl)-3-(2-methoxyphenyl)-1H-imidazo[4,5-b]pyridin-2(3H)-one;
4-(3-(3-Methoxybenzyl)-2-oxo-2,3-dihydrooxazolo[5,4-b]pyrazin-5-yl)-N-methyl benzamide;
1-Cyclopentyl-6-(4-hydroxyphenyl)-1H-imidazo[4,5-b]pyrazin-2(3H)-one;
1-Cyclohexyl-6-(4-hydroxyphenyl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)benzamide;
Methyl 4-(3-(cyclohexylmethyl)-2-oxo-2,3-dihydro-1H-imidazo[4,5-b]pyrazin-5-yl)benzoate;
1-(Cyclohexylmethyl)-6-(pyridin-4-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-N-methylbenzamide;
1-(Cyclohexylmethyl)-6-(4-(hydroxymethyl)phenyl)-1H-imidazo[4,5-b]pyrazin-2(3H)-one;
1-(Cyclohexylmethyl)-6-(pyridin-3-yl)-1H-imidazo[4,5-b]pyrazin-2(3H)-one;
3-(Cyclohexylmethyl)-2-oxo-2,3-dihydro-1H-imidazo[4,5-b]pyrazin-5-yl)benzonitrile;
1-(Cyclohexylmethyl)-6-(1H-indol-5-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-N-isopropylbenzamide;
1-(2-Hydroxyethyl)-6-(4-hydroxyphenyl)-1H-imidazo[4,5-b]pyrazin-2(3H)-one;
1-(Cyclohexylmethyl)-6-(1H-indol-6-yl)-1H-imidazo[4,5-b]pyrazin-2(3H)-one;
3-(3-(Cyclohexylmethyl)-2-oxo-2,3-dihydro-1H-imidazo[4,5-b]pyrazin-5-yl)benzamide;
6-(4-(Aminomethyl)phenyl)-1-(cyclohexylmethyl)-1H-imidazo[4,5-b]pyrazin-2(3H)-one;
6-(4-Hydroxyphenyl)-1-((1-methylpiperidin-4-yl)methyl)-1H-imidazo[4,5-b]pyrazin-2(3H)-one;;
4-(3-(Cyclohexylmethyl)-2-oxo-2,3-dihydro-1H-imidazo[4,5-b]pyrazin-5-yl)benzonitrile;
1-((1s,4s)-4-Hydroxycyclohexyl)-6-(4-hydroxyphenyl)-1H-imidazo[4,5-b]pyrazin-2(3H)-one;
1-(Cyclohexylmethyl)-6-(pyridin-2-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-N-ethylbenzamide;
1-(Cyclohexylmethyl)-6-(4-(2-hydroxypropan-2-yl)phenyl)-1H-imidazo[4,5-b]pyrazin-2(3H)-one;
1-(Cyclohexylmethyl)-6-(4-hydroxy-2-methylphenyl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)benzoic acid;
6-(4-Hydroxyphenyl)-1-(2-methoxyethyl)-1H-imidazo[4,5-b]pyrazin-2(3H)-one;
6-(4-Hydroxyphenyl)-1-(3-methoxypropyl)-1H-imidazo[4,5-b]pyrazin-2(3H)-one;
6-(4-Hydroxyphenyl)-4-(3-methoxybenzyl)-3,4-dihydropyrazino[2,3-b]pyrazin-2(1H)-one;
6-(4-Hydroxyphenyl)-1-(2-(tetrahydro-2H-pyran-4-yl)ethyl)-1H-imidazo[4,5-b]pyrazin-2(3H)-one;
6-(4-Hydroxyphenyl)-1-phenethyl-1H-imidazo[4,5-b]pyrazin-2(3H)-one;
1-((1r,4r)-4-Hydroxycyclohexyl)-6-(4-hydroxyphenyl)-1H-imidazo[4,5-b]pyrazin-2(3H)-one;
6-(4-(1H-1,2,4-Triazol-3-yl)phenyl)-1-(cyclohexylmethyl)-1H-imidazo[4,5-b]pyrazin**-**2(3H)-one;
1-(Cyclohexylmethyl)-6-phenyl-1H-imidazo[4,5-b]pyrazin-2(3H)-one;
1-(Cyclohexylmethyl)-6-(1H-pyrazol-5-yl)-1H-imidazo[4,5-b]pyrazin-2(3H)-one;
1-(Cyclohexylmethyl)-6-(1H-pyrazol-4-yl)-1H-imidazo[4,5-b]pyrazin-2(3H)-one;
1-(Cyclohexylmethyl)-6-(1-oxoisoindolin-5-yl)-1H-imidazo[4,5-b]pyrazin-2(3H)-one;
6-(3-(1H-Tetrazol-5-yl)phenyl)-1-(cyclohexylmethyl)-1H-imidazo[4,5-b]pyrazin-2(3H)-one;
1-(Cyclohexylmethyl)-6-(2-oxoindolin-5-yl)-1H-imidazo[4,5-b]pyrazin-2(3H)-one;
1-(Cyclohexylmethyl)-6-(1H-indazol-5-yl)-1H-imidazo[4,5-b]pyrazin-2(3H)-one;
1-(Cyclohexylmethyl)-6-(6-methoxypyridin-3-yl)-1H-imidazo[4,5-b]pyrazin-2(3H)-one;
6-(4-Hydroxyphenyl)-1-(tetrahydro-2H-pyran-4-yl)-1H-imidazo[4,5-b]pyrazin-2(3H)-one;
6-(4-Hydroxyphenyl)-1-(piperidin-4-ylmethyl)-1H-imidazo[4,5-b]pyrazin-2(3H)-one;
1-(((1r,4r)-4-Aminocyclohexyl)methyl)-6-(4-hydroxyphenyl)-1H-imidazo[4,5-b]pyrazin-2(3H)-one;
1-(Cyclohexylmethyl)-6-(6-hydroxypyridin-3-yl)-1H-imidazo[4,5-b]pyrazin-2(3H)-one;
1-(Cyclohexylmethyl)-6-(2-methoxypyridin-4-yl)-1H-imidazo[4,5-b]pyrazin-2(3H)-one;
4-(3-((1r,4r)-4-Hydroxycyclohexyl)-2-oxo-2,3-dihydro-1H-imidazo[4,5-b]pyrazin-5-yl)benzamide;
2-(4-(3-(Cyclohexylmethyl)-2-oxo-2,3-dihydro-1H-imidazo[4,5-b]pyrazin-5-yl)phenyl) acetic acid;
2-(4-(3-(Cyclohexylmethyl)-2-oxo-2,3-dihydro-1H-imidazo[4,5-b]pyrazin-5-yl)phenyl) acetamide;
1-(Cyclohexylmethyl)-6-(2-oxoindolin-6-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-3-methyl benzoic acid;
N-Methyl-4-(2-oxo-3-((tetrahydro-2H-pyran-4-yl)methyl)-2,3-dihydro-1H-imidazo[4,5-b]pyrazin-5-yl)benzamide;
4-(2-oxo-3-((Tetrahydro-2H-pyran-4-yl)methyl)-2,3-dihydro-1H-imidazo[4,5-b]pyrazin-5-yl)benzamide;
7-(4-Hydroxyphenyl)-1-(3-methoxybenzyl)-3,4-dihydropyrazino[2,3-b]pyrazin-2(1H)-one;
6-(4-(2-Hydroxypropan-2-yl)phenyl)-1-((tetrahydro-2H-pyran-4-yl)methyl)-1H-imidazo[4,5-b]pyrazin-2(3H)-one;
6-(1H-Indol-5-yl)-1-((tetrahydro-2H-pyran-4-yl)methyl)-1H-imidazo[4,5-b]pyrazin-2(3H)-one;
6-(4-(4H-1,2,4-Triazol-3-yl)phenyl)-1-((tetrahydro-2H-pyran-4-yl)methyl)-1H-imidazo[4,5-b]pyrazin-2(3H)-one;
6-(1H-Benzo[d]imidazol-5-yl)-1-(cyclohexylmethyl)-1H-imidazo[4,5-b]pyrazin-2(3H)-one;
4-(2-oxo-3-(2-(Tetrahydro-2H-pyran-4-yl)ethyl)-2,3-dihydro-1H-imidazo[4,5-b]pyrazin-5-yl)benzamide;
6-(3-(2H-1,2,3-Triazol-4-yl)phenyl)-1-(cyclohexylmethyl)-1H-imidazo[4,5-b]pyrazin-2(3H)-one;
6-(4-(1H-Imidazol-1-yl)phenyl)-1-(cyclohexylmethyl)-1H-imidazo[4,5-b]pyrazin-2(3H)-one;
6-(4-(1H-1,2,4-Triazol-3-yl)phenyl)-1-((1r,4r)-4-hydroxycyclohexyl)-1H-imidazo[4,5-b]pyrazin-2(3H)-one;
6-(4-(2H-tetrazol-5-yl)phenyl)-1-(cyclohexylmethyl)-1H-imidazo[4,5-b]pyrazin-2(3H)-one;
1-(Cyclohexylmethyl)-6-(2-hydroxypyridin-4-yl)-1H-imidazo[4,5-b]pyrazin-2(3H)-one;
6-(4-(1H-1 ,2,4-Triazol-3-yl)phenyl)-1-(2-(tetrahydro-2H-pyran-4-yl)ethyl)-1H-imidazo[4,5-b]pyrazin-2(3H)-one;
6-(4-(1H-Imidazol-2-yl)phenyl)-1-(cyclohexylmethyl)-1H-imidazo[4,5-b]pyrazin-2(3H)-one;
6-(4-(1H-1,2,3-Triazol-1-yl)phenyl)-1-(cyclohexylmethyl)-1H-imidazo[4,5-b]pyrazin-2(3H)-one;
6-(4-(2-Hydroxypropan-2-yl)phenyl)-1-(2-(tetrahydro-2H-pyran-4-yl)ethyl)-1H-imidazo[4,5-b]pyrazin-2(3H)-one;
1-(Cyclohexylmethyl)-6-(4-(5-methyl-1H-1,2,4-triazol-3-yl)phenyl)-1H-imidazo[4,5-b]pyrazin-2(3H)-one;
6-(4-(1H-Pyrazol-3-yl)phenyl)-1-(cyclohexylmethyl)-1H-imidazo[4,5-b]pyrazin-2(3H)-one;
6-(4-(1H-Pyrazol-4-yl)phenyl)-1-(cyclohexylmethyl)-1H-imidazo[4,5-b]pyrazin-2(3H)-one;
6-(4-(5-(Aminomethyl)-1H-1,2,4-triazol-3-yl)phenyl)-1-(cyclohexylmethyl)-1H-imidazo[4,5-b]pyrazin-2(3H)-one hydrochloride;
1-(Cyclohexylmethyl)-6-(4-(5-(trifluoromethyl)-1H-1,2,4-triazol-3-yl)phenyl)-1H-imidazo[4,5-b]pyrazin-2(3H)-one;
6-(4-Hydroxyphenyl)-1-((1r,4r)-4-methoxycyclohexyl)-1H-imidazo[4,5-b]pyrazin-2(3H)-one;
6-(4-Hydroxyphenyl)-1-((tetrahydrofuran-2-yl)methyl)-1H-imidazo[4,5-b]pyrazin-2(3H)-one;
6-(3-(1H-1,2,4-Triazol-3-yl)phenyl)-1-(cyclohexylmethyl)-1H-imidazo[4,5-b]pyrazin-2(3H)-one;
1-((1r,4r)-4-(Hydroxymethyl)cyclohexyl)-6-(4-hydroxyphenyl)-1H-imidazo[4,5-b]pyrazin-2(3H)-one;
6-(4-Hydroxyphenyl)-1-((1s,4s)-4-methoxycyclohexyl)-1H-imidazo[4,5-b]pyrazin-2(3H)-one;
6-(4-Hydroxyphenyl)-1-((1r,4r)-4-(methoxymethyl)cyclohexyl)-1H-imidazo[4,5-b]pyrazin-2(3H)-one;
6-(1-Methyl-1H-pyrazol-4-yl)-1-((tetrahydro-2H-pyran-4-yl)methyl)-1H-imidazo[4,5-b]pyrazin-2(3H)-one;
1-(((1r,4r)-4-Hydroxycyclohexyl)methyl)-6-(4-hydroxyphenyl)-1H-imidazo[4,5-b]pyrazin-2(3H)-one;
6-(4-Hydroxyphenyl)-1-((tetrahydrofuran-3-yl)methyl)-1H-imidazo[4,5-b]pyrazin-2(3H)-one;
1-(((1s,4s)-4-Hydroxycyclohexyl)methyl)-6-(4-hydroxyphenyl)-1H-imidazo[4,5-b]pyrazin-2(3H)-one;
6-(1H-Benzo[d]imidazol-5-yl)-1-((tetrahydro-2H-pyran-4-yl)methyl)-1H-imidazo[4,5-b]pyrazin-2(3H)-one hydrochloride;
6-(4-(5-(Morpholinomethyl)-1H-1,2,4-triazol-3-yl)phenyl)-1-((tetrahydro-2H-pyran-4-yl)methyl)-1H-imidazo[4,5-b]pyrazin-2(3H)-one;
6-(4-Hydroxyphenyl)-1-(3-(2-oxopyrrolidin-1-yl)propyl)-1H-imidazo[4,5-b]pyrazin-2(3H)-one;
6-(4-Hydroxyphenyl)-1-(2-morpholinoethyl)-1H-imidazo[4,5-b]pyrazin-2(3H)-one hydrochloride;
1-(Cyclohexylmethyl)-6-(4-(oxazol-5-yl)phenyl)-1H-imidazo[4,5-b]pyrazin-2(3H)-one;
6-(2-Methyl-1H-benzo[d]imidazol-5-yl)-1-((tetrahydro-2H-pyran-4-yl)methyl)-1H-imidazo[4,5-b]pyrazin-2(3H)-one hydrocholoride;
6-(4-(5-(Methoxymethyl)-1H-1,2,4-triazol-3-yl)phenyl)-1-((tetrahydro-2H-pyran-4-yl)methyl)-1H-imidazo[4,5-b]pyrazin-2(3H)-one;
1-((1s,4s)-4-(Hydroxymethyl)cyclohexyl)-6-(4-hydroxyphenyl)-1H-imidazo[4,5-b]pyrazin-2(3H)-one;
6-(3-Methyl-1H-pyrazol-4-yl)-1-((tetrahydro-2H-pyran-4-yl)methyl)-1H-imidazo[4,5-b]pyrazin-2(3H)-one;
6-(1H-Pyrazol-4-yl)-1-((tetrahydro-2H-pyran-4-yl)methyl)-1H-imidazo[4,5-b]pyrazin-2(3H)-one;
6-(2-Amino-1H-benzo[d]imidazol-5-yl)-1-((tetrahydro-2H-pyran-4-yl)methyl)-1H-imidazo[4,5-b]pyrazin-2(3H)-one di hydrochloride;
6-(4-(5-(2-Hydroxypropan-2-yl)-1H-1,2,4-triazol-3-yl)phenyl)-1-((tetrahydro-2H-pyran-4-yl)methyl)-1H-imidazo[4,5-b]pyrazin-2(3H)-one;
6-(4-(5-Isopropyl-1H-1,2,4-triazol-3-yl)phenyl)-1-((tetrahydro-2H-pyran-4-yl)methyl)-1H-imidazo[4,5-b]pyrazin-2(3H)-one;
4-(2-Methoxy-1-(2-morpholinoethyl)-1H-imidazo[4,5-b]pyrazin-6-yl)benzamide hydrochloride;
4-(1-((1s,4s)-4-Hydroxycyclohexyl)-2-methoxy-1H-imidazo[4,5-b]pyrazin-6-yl) benzamide;
6-(4-Hydroxyphenyl)-1-((1s,4s)-4-(methoxymethyl)cyclohexyl)-1H-imidazo[4,5-b]pyrazin-2(3H)-one;
6-(3H-imidazo[4,5-b]pyridin-6-yl)-1-((tetrahydro-2H-pyran-4-yl)methyl)-1H-imidazo[4,5-b]pyrazin-2(3H)-one;
1-(2-(2,2-Dimethyltetrahydro-2H-pyran-4-yl)ethyl)-6-(4-hydroxyphenyl)-1H-imidazo[4,5-b]pyrazin-2(3H)-one;
6-(4-(1H-Pyrazol-1-yl)phenyl)-1-((tetrahydro-2H-pyran-4-yl)methyl)-1H-imidazo[4,5-b]pyrazin-2(3H)-one;
6-(4-(4H-1,2,4-Triazol-3-yl)phenyl)-1-(2-morpholinoethyl)-1H-imidazo[4,5-b]pyrazin-2(3H)-one;
6-(4-(1H-Benzo[d]imidazol-2-yl)phenyl)-1-((tetrahydro-2H-pyran-4-yl)methyl)-1H-imidazo[4,5-b]pyrazin-2(3H)-one;
6-(4-(1H-Imidazol-2-yl)phenyl)-1-((tetrahydro-2H-pyran-4-yl)methyl)-1H-imidazo[4,5-b]pyrazin-2(3H)-one hydrochloride;
6-(4-(5-(Hydroxymethyl)-1H-1,2,4-triazol-3-yl)phenyl)-1-((tetrahydro-2H-pyran-4-yl)methyl)-1H-imidazo[4,5-b]pyrazin-2(3H)-one;
6-(4-(1H-Imidazol-5-yl)phenyl)-1-((tetrahydro-2H-pyran-4-yl)methyl)-1H-imidazo[4,5-b]pyrazin-2(3H)-one hydrochloride;
6-(4-Hydroxyphenyl)-1-((5-oxopyrrolidin-2-yl)methyl)-1H-imidazo[4,5-b]pyrazin-2(3H)-one;
6-(4-(4,5-Dimethyl-1H-imidazol-2-yl)phenyl)-1 -((tetrahydro-2H-pyran-4-yl)methyl)-1H-imidazo[4,5-b]pyrazin-2(3H)-one;
6-(4-(1H-1,2,4-Triazol-5-yl)phenyl)-1-(((1s,4s)-4-methoxycyclohexyl)methyl)-1H-imidazo[4,5-b]pyrazin-2(3H)-one;
6-(4-(1H-1,2,4-Triazol-5-yl)phenyl)-1-(((1r,4r)-4-methoxycyclohexyl)methyl)-1H-imidazo[4,5-b]pyrazin-2(3H)-one;
6-(6-(1H-1,2,4-Triazol-3-yl)pyridin-3-yl)-1-((tetrahydro-2H-pyran-4-yl)methyl)-1H-imidazo[4,5-b]pyrazin-2(3H)-one;
6-(4-(1H-1,2,4-Triazol-3-yl)phenyl)-1-(2-(2-oxopyrrolidin-1-yl)ethyl)-1H-imidazo[4,5-b]pyrazin-2(3H)-one;
6-(4-(5-((dimethylamino)methyl)-1H-1,2,4-triazol-3-yl)phenyl)-1-((tetrahydro-2H-pyran-4-yl)methyl)-1H-imidazo[4,5-b]pyrazin-2(3H)-one;
6-(4-Hydroxyphenyl)-1-(pyrrolidin-2-ylmethyl)-1H-imidazo[4,5-b]pyrazin-2(3H)-one hydrochloride;
6-(2-Aminobenzimidazol-5-yl)-1-(cyclohexylmethyl)-4-imidazolino[4,5-b]pyrazin-2-one di hydrochloride;
6-(2-(Dimethylamino)-1H-benzo[d]imidazol-5-yl)-1-((tetrahydro-2H-pyran-4-yl)methyl)-1H-imidazo[4,5-b]pyrazin-2(3H)-one;
6-(4-Hydroxyphenyl)-1-(piperidin-3-ylmethyl)-1H-imidazo[4,5-b]pyrazin-2(3H)-one;
6-(4-(4H-1,2,4-triazol-3-yl)phenyl)-1-(2-(piperidin-1-yl)ethyl)-1H-imidazo[4,5-b]pyrazin-2(3H)-one hydrochloride;
1-(Cyclohexylmethyl)-6-(2-(methylamino)pyrimidin-5-yl)-1H-imidazo[4,5-b]pyrazin-2(3H)-one;
6-(3-methyl-4-(1H-1,2,4-triazol-3-yl)phenyl)-1-((tetrahydro-2H-pyran-4-yl)methyl)-1H-imidazo[4,5-b]pyrazin-2(3H)-one;
1-(Cyclohexylmethyl)-6-(2-(2-methoxyethylamino)pyrimidin-5-yl)-1H-imidazo[4,5-b]pyrazin-2(3H)-one;
6-(4-(5-((methylamino)methyl)-1H-1,2,4-triazol-3-yl)phenyl)-1-((tetrahydro-2H-pyran-4-yl)methyl)-1H-imidazo[4,5-b]pyrazin-2(3H)-one;
6-(4-(5-Oxopyrrolidin-2-yl)phenyl)-1-(2-(tetrahydro-2H-pyran-4-yl)ethyl)-1H-imidazo[4,5-b]pyrazin-2(3H)-one;
6-(4-(5-methyl-1H-1,2,4-triazol-3-yl)phenyl)-1-(2-(tetrahydro-2H-pyran-4-yl)ethyl)-1H-imidazo[4,5-b]pyrazin-2(3H)-one;
6-(4-(1H-imidazol-2-yl)phenyl)-1-(2-(tetrahydro-2H-pyran-4-yl)ethyl)-1H-imidazo[4,5-b]pyrazin-2(3H)-one;
6-(4-(4H-1,2,4-triazol-3-yl)phenyl)-1-(2-methyl-2-morpholinopropyl)-1H-imidazo[4,5-b]pyrazin-2(3H)-one;
6-(4-(4H-1,2,4-Triazol-3-yl)phenyl)-1-(1-morpholinopropan-2-yl)-1H-imidazo[4,5-b]pyrazin-2(3H)-one;
6-(4-(Pyrrolidin-2-yl)phenyl)-1-(2-(tetrahydro-2H-pyran-4-yl)ethyl)-1H-imidazo[4,5-b]pyrazin-2(3H)-one;
6-(4-(5-(aminomethyl)-1H-1,2,4-triazol-3-yl)phenyl)-1-(2-(tetrahydro-2H-pyran-4-yl)ethyl)-1H-imidazo[4,5-b]pyrazin-2(3H)-one;
6-(5-(Hydroxymethyl)thiophen-2-yl)-1-((tetrahydro-2H-pyran-4-yl)methyl)-1H-imidazo[4,5-b]pyrazin-2(3H)-one;
(1r,4r)-4-(6-(4-Hydroxyphenyl)-2-oxo-2,3-dihydro-1H-imidazo[4,5-b]pyrazin-1-yl)cyclohexanecarboxamide;
(1s,4s)-4-(6-(4-Hydroxyphenyl)-2-oxo-2,3-dihydro-1H-imidazo[4,5-b]pyrazin-1-yl)cyclohexanecarboxamide;
6-(4-(5-methyl-1H-1,2,4-triazol-3-yl)phenyl)-1-(2-morpholinoethyl)-1H-imidazo[4,5-b]pyrazin-2(3H)-one;
6-(4-(5-Oxopyrrolidin-3-yl)phenyl)-1-(2-(tetrahydro-2H-pyran-4-yl)ethyl)-1H-imidazo[4,5-b]pyrazin-2(3H)-one;
6-(4-(Pyrrolidin-3-yl)phenyl)-1-(2-(tetrahydro-2H-pyran-4-yl)ethyl)-1H-imidazo[4,5-b]pyrazin-2(3H)-one;
6-(1H-benzo[d]imidazol-5-yl)-1-(2-(tetrahydro-2H-pyran-4-yl)ethyl)-1H-imidazo[4,5-b]pyrazin-2(3H)-one;
6-(3-(Hydroxymethyl)thiophen-2-yl)-1-((tetrahydro-2H-pyran-4-yl)methyl)-1H-imidazo[4,5-b]pyrazin-2(3H)-one;
6-(5-(2-Hydroxyethyl)thiophen-2-yl)-1-((tetrahydro-2H-pyran-4-yl)methyl)-1H-imidazo[4,5-b]pyrazin-2(3H)-one;
1-(Cyclohexylmethyl)-6-(pyrimidin-5-yl)-1H-imidazo[4,5-b]pyrazin-2(3H)-one;
6-(6-Fluoropyridin-3-yl)-1-((tetrahydro-2H-pyran-4-yl)methyl)-1H-imidazo[4,5-b]pyrazin-2(3H)-one;
6-(6-pyridin-3-yl)-1-((tetrahydro-2H-pyran-4-yl)methyl)-1H-imidazo[4,5-b]pyrazin-2(3H)-one;
6-(4-(5-methyl-1H-imidazol-2-yl)phenyl)-1-((tetrahydro-2H-pyran-4-yl)methyl)-1H-imidazo[4,5-b]pyrazin-2(3H)-one;
6-(4-(5-Methyl-1H-1,2,4-triazol-3-yl)phenyl)-1-(2-(2-oxopyrrolidin-1-yl)ethyl)-1H-imidazo[4,5-b]pyrazin-2(3H)-one;
6-(6-(Methylamino)pyridin-3-yl)-1-((tetrahydro-2H-pyran-4-yl)methyl)-1H-imidazo[4,5-b]pyrazin-2(3H)-one;
6-(2-aminopyrimidin-5-yl)-1-(cyclohexylmethyl)-1H-imidazo[4,5-b]pyrazin-2(3H)-one;
6-(4-(2-hydroxypropan-2-yl)phenyl)-1-(((1r,4r)-4-methoxycyclohexyl)methyl)-1H-imidazo[4,5-b]pyrazin-2(3H)-one;
6-(4-hydroxyphenyl)-1-((1-methylpiperidin-3-yl)methyl)-1H-imidazo[4,5-b]pyrazin-2(3H)-one;
6-(2-methyl-4-(1H-1,2,4-triazol-3-yl)phenyl)-1-(2-(tetrahydro-2H-pyran-4-yl)ethyl)-1H-imidazo[4,5-b]pyrazin-2(3H)-one;
1-(cyclohexylmethyl)-6-(6-(2-hydroxypropan-2-yl)pyridin-3-yl)-1H-imidazo[4,5-b]pyrazin-2(3H)-one;
6-(4-(hydroxymethyl)thiophen-2-yl)-1-((tetrahydro-2H-pyran-4-yl)methyl)-1H-imidazo [4,5-b]pyrazin-2(3H)-one;
6-(1H-benzo[d]imidazol-6-yl)-1-(((1r,4r)-4-methoxycyclohexyl)methyl)-1H-imidazo [4,5- b]pyrazin-2(3H)-one;
6-(4-(4,5-dimethyl-1H-imidazol-2-yl)phenyl)-1-(2-morpholinoethyl)-1H-imidazo[4,5-b]pyrazin-2(3H)-one;
6-(6-(2-hydroxypropan-2-yl)pyridin-3-yl)-1-((tetrahydro-2H-pyran-4-yl)methyl)-1H-imidazo[4,5-b]pyrazin-2(3H)-one;
6-(6-(2-hydroxypropan-2-yl)pyridin-3-yl)-1-(2-(tetrahydro-2H-pyran-4-yl)ethyl)-1H-imidazo[4,5-b]pyrazin-2(3H)-one;
6-(4-(4H-1,2,4-triazol-3-yl)phenyl)-1-(2-morpholino-2-oxoethyl)-1H-imidazo[4,5-b]pyrazin-2(3H)-one;
6-(4-(4H-1,2,4-triazol-3-yl)phenyl)-3-(cyclohexylmethyl)-3,4-dihydropyrazino[2,3-b]pyrazin-2(1H)-one;
6-(4-(1H-1,2,4-triazol-3-yl)phenyl)-1-(2-(tetrahydro-2H-pyran-4-yl)ethyl)-1H-imidazo[4,5-b]pyridin-2(3H)-one;
(R)-6-(4-(1H-1,2,4-triazol-3-yl)phenyl)-1-(1-phenylethyl)-1H-imidazo[4,5-b]pyrazin-2(3H)-one;
(S)-6-(4-(1H-1,2,4-triazol-3-yl)phenyl)-1-(1-phenylethyl)-1H-imidazo[4,5-b]pyrazin-2(3H)-one;
(1r,4r)-4-(6-(4-(2-hydroxypropan-2-yl)phenyl)-2-oxo-2,3-dihydro-1H-imidazo[4,5-b]pyrazin-1-yl)cyclohexanecarboxamide;
6-(3-Methyl-4-(1H-1,2,4-Triazol-3-yl)phenyl)-1-((tetrahydro-2H-pyran-4-yl)methyl)-1H-imidazo[4,5-B]pyrazin-2(3H)-one;
6-(4-(1H-imidazol-2-yl)phenyl)-1-(2-(tetrahydro-2H-pyran-4-yl)ethyl)-1H-imidazo[4,5-b]pyrazin-2(3H)-one;
6-(4-(5-(Aminomethyl)-1H-1,2,4-triazol-3-yl)phenyl)-1-(2-(tetrahydro-2H-pyran-4-yl)ethyl)-1H-imidazo[4,5-b]pyrazin-2(3H)-one;
6-(1H-benzo[d]imidazol-5-yl)-1-(2-(tetrahydro-2H-pyran-4-yl)ethyl)-1H-imidazo[4,5-b]pyrazin-2(3H)-one;
6-(2-Aminopyrimidin-5-yl)-1-(cyclohexylmethyl)-1H-imidazo[4,5-b]pyrazin-2(3H)-one;
6-(4-Hydroxyphenyl)-1-((1-methylpiperidin-2-yl)methyl)-1H-imidazo[4,5-b]pyrazin-2(3H)-one hydrochloride;
6-(3-Methyl-4-(1H-1,2,4-Triazol-3-yl)phenyl)-1-((tetrahydro-2H-pyran-4-yl)methyl)-1H-imidazo[4,5-B]pyrazin-2(3H)-one;
1-(Cyclohexylmethyl)-6-(6-(2-hydroxypropan-2-yl)pyridin-3-yl)-1H-imidazo[4,5-b]pyrazin-2(3H)-one;
6-(6-(2-Hydroxypropan-2-yl)pyridin-3-yl)-1-((tetrahydro-2H-pyran-4-yl)methyl)-1H-imidazo[4,5-b]pyrazin-2(3H)-one;
6-(6-(2-Hydroxypropan-2-yl)pyridin-3-yl)-1-(2-(tetrahydro-2H-pyran-4-yl)ethyl)-1H-imidazo[4,5-b]pyrazin-2(3H)-one;
6-(4-(4H-1,2,4-Triazol-3-yl)phenyl)-1-(2-morpholino-2-oxoethyl)-1H-imidazo[4,5-b]pyrazin-2(3H)-one;
(R)-6-(4-(4H-1,2,4-Triazol-3-yl)phenyl)-3-(cyclohexylmethyl)-3,4-dihydropyrazino[2,3-b]pyrazin-2(1H)-one;
(R)-6-(4-(1H-1,2,4-triazol-3-yl)phenyl)-1-(1-phenylethyl)-1H-imidazo[4,5-b]pyrazin-2(3H)-one;
(S)-6-(4-(4H-1,2,4-Triazol-3-yl)phenyl)-1-(1-phenylethyl)-1H-imidazo[4,5-b]pyrazin-2(3H)-one;
(1r,4r)-4-(6-(4-(2-Hydroxypropan-2-yl)phenyl)-2-oxo-2,3-dihydro-1H-imidazo[4,5-b]pyrazin-1-yl)cyclohexanecarboxamide; and
6-(4-(5-Methyl-1H-1,2,4-triazol-3-yl)phenyl)-1-(2-(tetrahydro-2H-pyran-4-yl)ethyl)-1H-imidazo[4,5-b]pyrazin-2(3H)-one,
and pharmaceutically acceptable salts, clathrates, solvates, stereoisomers, tautomers, and prodrugs thereof.

As also disclosed herein, the TOR kinase inhibitors include compounds having the following formula (II): 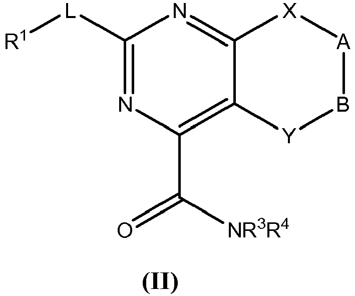 and pharmaceutically acceptable salts, clathrates, solvates, stereoisomers, tautomers, and prodrugs thereof, wherein:
R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
-X-A-B-Y- taken together form -N(R²)CH₂C(O)NH-, -N(R²)C(O)CH₂NH-, -N(R²)C(O)NH-, -N(R²)C=N-, or -C(R²)=CHNH-;
L is a direct bond, NH or O;
R² is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl; and
R³ and R⁴ are independently H or C₁₋₈alkyl.

Representative TOR kinase inhibitors of formula (II) as disclosed herein include:
9-benzyl-8-oxo-2-(pyridin-3-yl)-8,9-dihydro-7H-purine-6-carboxamide;
N-methyl-8-oxo-9-phenyl-2-(pyridin-3-yl)-8,9-dihydro-7H-purine-6-carboxamide;
8-oxo-9-phenyl-2-(pyridin-2-yl)-8,9-dihydro-7H-purine-6-carboxamide;
2-(2-chloropyridin-3-yl)-8-oxo-9-phenyl-8,9-dihydro-7H-purine-6-carboxamide;
2-(2-methoxypyridin-3-yl)-8-oxo-9-phenyl-8,9-dihydro-7H-purine-6-carboxamide;
N,N-dimethyl-8-oxo-9-phenyl-2-(pyridin-3-yl)-8,9-dihydro-7H-purine-6-carboxamide;
9-methyl-8-oxo-2-(pyridin-3-yl)-8,9-dihydro-7H-purine-6-carboxamide;
2-(4-hydroxyphenyl)-9-(2-methoxyphenyl)-8-oxo-8,9-dihydro-7H-purine-6-carboxamide;
2-(3-hydroxyphenyl)-8-oxo-9-o-tolyl-8,9-dihydro-7H-purine-6-carboxamide;
2-(1H-indol-4-yl)-9-(2-methoxyphenyl)-8-oxo-8,9-dihydro-7H-purine-6-carboxamide;
2-(1H-indol-6-yl)-9-(2-methoxyphenyl)-8-oxo-8,9-dihydro-7H-purine-6-carboxamide;
2-(3-hydroxyphenyl)-9-(4-methoxyphenyl)-8-oxo-8,9-dihydro-7H-purine-6-carboxamide;
2-(2-hydroxypyridin-4-yl)-9-(2-methoxyphenyl)-8-oxo-8,9-dihydro-7H-purine-6-carboxamide;
9-(2-chlorophenyl)-2-(3-hydroxyphenyl)-8-oxo-8,9-dihydro-7H-purine-6-carboxamide;
9-(2-fluorophenyl)-2-(3-hydroxyphenyl)-8-oxo-8,9-dihydro-7H-purine-6-carboxamide;
9-(2,6-difluorophenyl)-2-(3-hydroxyphenyl)-8-oxo-8,9-dihydro-7H-purine-6-carboxamide;
9-cycloheptyl-8-oxo-2-(pyridin-3-yl)-8,9-dihydro-7H-purine-6-carboxamide;
9-(2-methoxyphenyl)-8-oxo-2-(quinolin-5-yl)-8,9-dihydro-7H-purine-6-carboxamide;
2-cyclopentyl-9-(2-methoxyphenyl)-8-oxo-8,9-dihydro-7H-purine-6-carboxamide;
9-(2-methoxyphenyl)-8-oxo-2-(3-(trifluoromethyl)phenyl)-8,9-dihydro-7H-purine-6-carboxamide;
9-(2-methoxyphenyl)-2-(6-methoxypyridin-3-yl)-8-oxo-8,9-dihydro-7H-purine-6-carboxamide;
2-(3-hydroxyphenyl)-8-oxo-9-(4-(trifluoromethyl)phenyl)-8,9-dihydro-7H-purine-6-carboxamide;
9-benzyl-2-(3-hydroxyphenyl)-8-oxo-8,9-dihydro-7H-purine-6-carboxamide;
2-(3-hydroxyphenyl)-8-oxo-9-(2-(trifluoromethoxy)phenyl)-8,9-dihydro-7H-purine-6-carboxamide;
9-(2,4-dichlorophenyl)-2-(3-hydroxyphenyl)-8-oxo-8,9-dihydro-7H-purine-6-carboxamide;
9-(2-methoxyphenyl)-2-(3-nitrophenyl)-8-oxo-8,9-dihydro-7H-purine-6-carboxamide;
2-(3-cyanophenyl)-8-oxo-9-phenyl-8,9-dihydro-7H-purine-6-carboxamide;
9-(3-fluorophenyl)-2-(3-hydroxyphenyl)-8-oxo-8,9-dihydro-7H-purine-6-carboxamide;
9-(2-methoxyphenyl)-8-oxo-2-(2-(trifluoromethyl)phenyl)-8,9-dihydro-7H-purine-6-carboxamide;
2-(5-fluoropyridin-3-yl)-9-(2-methoxyphenyl)-8-oxo-8,9-dihydro-7H-purine-6-carboxamide;
2-(1-benzylpiperidin-4-yl)-9-(2-methoxyphenyl)-8-oxo-8,9-dihydro-7H-purine-6-carboxamide;
benzyl 4-(6-carbamoyl-8-oxo-2-(pyridin-3-yl)-7H-purin-9(8H)-yl)piperidine-1-carboxylate;
9-cyclohexyl-2-(3-hydroxyphenyl)-8-oxo-8,9-dihydro-7H-purine-6-carboxamide;
9-(2-methoxyphenyl)-8-oxo-2-(3-(trifluoromethoxy)phenyl)-8,9-dihydro-7H-purine-6-carboxamide;
9-phenyl-2-(pyridin-3-yl)-9H-purine-6-carboxamide;
6-oxo-8-phenyl-2-(pyridin-3-yl)-5,6,7,8-tetrahydropteridine-4-carboxamide;
6-oxo-8-phenyl-2-(pyridin-4-yl)-5,6,7,8-tetrahydropteridine-4-carboxamide;
2-(3-aminophenyl)-9-(2-methoxyphenyl)-8-oxo-8,9-dihydro-7H-purine-6-carboxamide;
2-(3-hydroxyphenyl)-9-(2-methoxyphenyl)-9H-purine-6-carboxamide;
9-Cyclopentyl-2-(3-hydroxyphenyl)-8-oxo-8,9-dihydro-7H-purine-6-carboxamide;
9-tert-Butyl-2-(3-hydroxy-phenyl)-8-oxo-8,9-dihydo-7H-purine-6-carboxamide;
[2-(3-Hydroxyphenyl)-9-(2-methoxyphenyl)-8-oxo(7-hydropurin-6-yl)]-N-methylcarbox-amide;
2-phenyl-5H-pyrrolo[3,2-d]pyrimidine-4-carboxamide;
[2-(3-Hydroxyphenyl)-9-(2-methoxyphenyl)-8-oxo(7-hydropurin-6-yl)]-N,N-dimethyl carboxamide;
2-(3-Hydroxyphenylamino)-9-(2-methoxyphenyl)-8-oxo-8,9-dihydro-7H-purine-6-carboxamide;
2-(4-Hydroxyphenylamino)-9-(2-methoxyphenyl)-8-oxo-8,9-dihydro-7H-purine-6-carboxamide;
9-(trans-4-Hydroxycyclohexyl)-2-(3-hydroxyphenyl)-8-oxo-8,9-dihydro-7H-purine-6-carboxamide;
9-(trans-4-Hydroxycyclohexyl)-8-oxo-2-(pyridin-3-yl)-8,9-dihydro-7H-purine-6-carboxamide;
9-(trans-4-Hydroxycyclohexyl)-2-(3-hydroxyphenyl)-8-oxo-8,9-dihydro-7H-purine-6-carboxamide;
9-(trans-4-Hydroxycyclohexyl)-8-oxo-2-(pyridin-3-yl)-8,9-dihydro-7H-purine-6-carboxamide;
2-(3-Hydroxyphenylamino)-9-(2-methoxyphenyl)-9H-purine-6-carboxamide;
9-Isopropyl-2-(3-hydroxy-phenyl)-8-oxo-8,9-dihydo-7H-purine-6-carboxamide;
Methyl 4-(6-carbamoyl-9-(2-methoxyphenyl)-8-oxo-8,9-dihydro-7H-purin-2-yl) benzoate;
2-(2-Chloro-3-hydroxyphenyl)-9-(2-methoxyphenyl)-8-oxo-7-hydropurine-6-carbox amide;
2-(3-Cyanophenyl)-9-(2-methoxyphenyl)-8-oxo-8,9-dihydro-7H-purine-6-carboxamide;
2-(2-Hydroxyphenylamino)-9-(2-methoxyphenyl)-8-oxo-8,9-dihydro-7H-purine-6-carboxamide;
2-(3-Hydroxyphenyl)-9-(4-methoxy-2-methylphenyl)-8-oxo-8,9-dihydro-7H-purine-6-carboxamide;
2-(3-Hydroxyphenyl)-8-oxo-9-(2-(trifluoromethyl)phenyl)-8,9-dihydro-7H-purine-6-carboxamide;
2-(4-Cyano-phenyl)-9-(2-methoxy-phenyl)-8-oxo-8,9-dihydro-7H-purine-6-carboxamide;
4-[6-Carbamoyl-9-(2-methoxy-phenyl)-8-oxo-8,9-dihydro-7H-purin-2-yl]-benzoic acid;
Methyl 3-(6-carbamoyl-9-(2-methoxyphenyl)-8-oxo-8,9-dihydro-7H-purin-2-yl)benzoate;
3-(6-Carbamoyl-9-(2-methoxyphenyl)-8-oxo-8,9-dihydro-7H-purin-2-yl)benzoic acid;
2-(3-Hydroxyphenyl)-9-(2-isopropylphenyl)-8-oxo-8,9-dihydro-7H-purine-6-carboxamide;
2-(1H-Indazol-6-yl)-9-(2-methoxyphenyl)-8-oxo-7-hydropurine-6-carboxamide;
2-(4-Carbamoylphenyl)-9-(2-methoxyphenyl)-8-oxo-8,9-dihydro-7H-purine-6-carboxamide;
9-(2-Ethylphenyl)-2-(3-hydroxyphenyl)-8-oxo-8,9-dihydro-7H-purine-6-carboxamide;
9-(2,5-Dichlorophenyl)-2-(3-hydroxyphenyl)-8-oxo-7-hydropurine-6-carboxamide;
2-(3-Carbamoylphenyl)-9-(2-methoxyphenyl)-8-oxo-8,9-dihydro-7H-purine-6-carbox amide;
9-(2,6-Dichlorophenyl)-2-(3-hydroxyphenyl)-8-oxo-7-hydropurine-6-carboxamide;
2-(2-Hydroxyphenyl)-9-(2-methoxyphenyl)purine-6-carboxamide;
2-(1H-Indazol-5-yl)-9-(2-methoxyphenyl)-8-oxo-7-hydropurine-6-carboxamide;
9-(2,3-Dichlorophenyl)-2-(3-hydroxyphenyl)-8-oxo-7-hydropurine-6-carboxamide;
2-[4-(Hydroxymethyl)phenyl]-9-(2-methoxyphenyl)-8-oxo-7-hydropurine-6-carbox-amide;
2-[3-(Hydroxymethyl)phenyl]-9-(2-methoxyphenyl)-8-oxo-7-hydropurine-6-carbox-amide;
9-(2-Methoxyphenyl)-8-oxo-2-(pyridin-4-yl)-8,9-dihydro-7H-purine-6-carboxamide;
2-(4-Fluoro-3-hydroxyphenyl)-9-(2-methoxyphenyl)-8-oxo-7-hydropurine-6-carbox-amide;
2-(2-Fluoro-3-hydroxyphenyl)-9-(2-methoxyphenyl)-8-oxo-7-hydropurine-6-carbox-amide;
2-[4-(1-Hydroxy-isopropyl)phenyl]-9-(2-methoxyphenyl)-8-oxo-7-hydropurine-6-carboxamide;
2-[3-(1-Hydroxy-isopropyl)phenyl]-9-(2-methoxyphenyl)-8-oxo-7-hydropurine-6-carboxamide;
9-(2-Methoxyphenyl)-2-(2-nitrophenyl)-8-oxo-7-hydropurine-6-carboxamide;
9-(2-Methoxyphenyl)-2-(4-nitrophenyl)-8-oxo-7-hydropurine-6-carboxamide;
9-(2-Methoxyphenyl)-2-(2-nitrophenyl)-8-oxo-7-hydropurine-6-carboxamide;
9-(2,4-Difluorophenyl)-2-(3-hydroxyphenyl)-8-oxo-7-hydropurine-6-carboxamide;
9-(2-Methoxyphenyl)-2-{3-[(methylsulfonyl)amino]phenyl}-8-oxo-7-hydropurine-6-carboxamide;
9-(4-Chloro-2-fluorophenyl)-2-(3-hydroxyphenyl)-8-oxo-7-hydropurine-6-carboxamide;
9-(2-Chlorophenyl)-8-oxo-2-(3-pyridyl)-7-hydropurine-6-carboxamide;
8-Oxo-2-(3-pyridyl)-9-[2-(trifluoromethyl)phenyl]-7-hydropurine-6-carboxamide;
9-(3-Chloro-2-fluorophenyl)-2-(3-hydroxyphenyl)-8-oxo-7-hydropurine-6-carboxamide;
9-(2-Fluoro-3-trifluoromethylphenyl)-2-(3-hydroxyphenyl)-8-oxo-7-hydropurine-6-carboxamide;
9-(2, 3, 4-Trifluorophenyl)-2-(3-hydroxyphenyl)-8-oxo-7-hydropurine-6-carboxamide;
2-(1H-Benzo[d]imidazol-6-yl)-9-(2-methoxyphenyl)-8-oxo-8,9-dihydro-7H-purine-6-carboxamide;
2-[3-(Acetylamino)phenyl]-9-(2-methoxyphenyl)-8-oxo-7-hydropurine-6-carboxamide;
2-(3-hydroxyphenyl)-8-(2-methoxyphenyl)-6-oxo-5,6,7,8-tetrahydropteridine-4-carbox-amide;
9-(2-Methoxyphenyl)-8-oxo-2-pyrazol-4-yl-7-hydropurine-6-carboxamide;
9-(2-Methoxyphenyl)-8-oxo-2-pyrazol-3-yl-7-hydropurine-6-carboxamide;
9-(4-Aminocyclohexyl)-2-(3-hydroxyphenyl)-8-oxo-7-hydropurine-6-carboxamide;
2-[3-(Difluoromethyl)phenyl]-9-(2-methoxyphenyl)-8-oxo-7-hydropurine-6-carbox-amide;
2-[5-(Difluoromethyl)-2-fluorophenyl]-9-(2-methoxyphenyl)-8-oxo-7-hydropurine-6-carboxamide;
2-(1H-benzo[d]imidazol-4-yl)-9-(2-methoxyphenyl)-8-oxo-8,9-dihydro-7H-purine-6-carboxamide;
2-(6-Hydroxypyridin-3-yl)-8-oxo-9-(2-(trifluoromethyl)phenyl)-8,9-dihydro-7H-purine-6-carboxamide;
2-(1H-benzo[d]imidazol-6-yl)-9-(2-fluorophenyl)-8-oxo-8,9-dihydro-7H-purine-6-carboxamide;
2-Benzimidazol-6-yl-8-oxo-9-[2-(trifluoromethyl)phenyl]-7-hydropurine-6-carboxamide;
2-(5-Chloropyridin-3-yl)-8-oxo-9-(2-(trifluoromethyl)phenyl)-8,9-dihydro-7H-purine-6-carboxamide;
trans-4-(6-Carbamoyl-9-(2-methoxyphenyl)-8-oxo-8,9-dihydro-7H-purin-2-ylamino) cyclohexyl carbamate;
(R)-9-(2-Methoxyphenyl)-8-oxo-2-(pyrrolidin-3-ylamino)-8,9-dihydro-7H-purine-6-carboxamide;
(S)-9-(2-Methoxyphenyl)-8-oxo-2-(pyrrolidin-3-ylamino)-8,9-dihydro-7H-purine-6-carboxamide;
(cis)-4-(6-Carbamoyl-9-(2-methoxyphenyl)-8-oxo-8,9-dihydro-7H-purin-2-ylamino) cyclohexyl carbamate;
2-(trans-4-Hydroxycyclohexylamino)-9-(2-methoxyphenyl)-8-oxo-8,9-dihydro-7H-purine-6-carboxamide;
2-(4-Chloropyridin-3-yl)-8-oxo-9-(2-(trifluoromethyl)phenyl)-8,9-dihydro-7H-purine-6-carboxamide;
2-(cis-4-Hydroxycyclohexylamino)-9-(2-methoxyphenyl)-8-oxo-8,9-dihydro-7H-purine-6-carboxamide;
2-(4-((1H-Imidazol-1-yl)methyl)phenylamino)-9-(2-methoxyphenyl)-8-oxo-8,9-dihydro-7H-purine-6-carboxamide;
2-(4-Hydroxypyridin-3-yl)-8-oxo-9-(2-(trifluoromethyl)phenyl)-8,9-dihydro-7H-purine-6-carboxamide;
(R)-9-(2-Methoxyphenyl)-8-oxo-2-(pyrrolidin-2-ylmethylamino)-8,9-dihydro-7H-purine-6-carboxamide;
(S)-9-(2-Methoxyphenyl)-8-oxo-2-(pyrrolidin-2-ylmethylamino)-8,9-dihydro-7H-purine-6-carboxamide;
2-(4-(1H-1,2,4-Triazol-3-yl)phenyl)-9-(2-methoxyphenyl)-8-oxo-7-hydropurine-6-carboxamide;
2-(2-Hydroxyethylamino)-9-(2-methoxyphenyl)-8-oxo-8,9-dihydro-7H-purine-6-carboxamide ;
9-(2-Methoxyphenyl)-8-oxo-2-(2-(trifluoromethyl)-1H-benzo[d]imidazol-6-yl)-8,9-dihydro-7H-purine-6-carboxamide;
2-(3-(1H-1,2,4-Triazol-3-yl)phenyl)-9-(2-methoxyphenyl)-8-oxo-7-hydropurine-6-carboxamide;
9-(Biphenyl-2-yl)-2-(3-hydroxyphenyl)-8-oxo-8,9-dihydro-7H-purine-6-carboxamide;
2-(4-(1H-1,2,4-Triazol-3-yl)phenyl)-9-(2-fluorophenyl)-8-oxo-7-hydropurine-6-carboxamide;
2-(4-(1H-1,2,4-Triazol-3-yl)phenyl)-9-(2-isopropylphenyl)-8-oxo-8,9-dihydro-7H-purine-6-carboxamide;
9-(2-Methoxyphenyl)-2-(2-methyl-1H-benzo[d]imidazol-6-yl)-8-oxo-8,9-dihydro-7H-purine-6-carboxamide;
2-(3-(Hydroxymethyl)phenylamino)-9-(2-methoxyphenyl)-8-oxo-8,9-dihydro-7H-purine-6-carboxamide;
2-(2-(Hydroxymethyl)phenylamino)-9-(2-methoxyphenyl)-8-oxo-8,9-dihydro-7H-purine-6-carboxamide;
9-(2-tert-Butylphenyl)-2-(3-hydroxyphenyl)-8-oxo-8,9-dihydro-7H-purine-6-carboxamide;
2-(3-Hydroxyphenyl)-8-oxo-9-(2-phenoxyphenyl)-8,9-dihydro-7H-purine-6-carboxamide;
2-(1H-Benzo[d]imidazol-6-yl)-9-(2-isopropylphenyl)-8-oxo-8,9-dihydro-7H-purine-6-carboxamide;
2-(1H-Indazol-4-yl)-9-(2-methoxyphenyl)-8-oxo-8,9-dihydro-7H-purine-6-carboxamide;
2-(2-Hydroxypyridin-3-yl)-8-oxo-9-(2-(trifluoromethyl)phenyl)-8,9-dihydro-7H-purine-6-carboxamide;
2-(1H-Imidazo[4,5-b]pyridin-6-yl)-9-(2-methoxyphenyl)-8-oxo-8,9-dihydro-7H-purine-6-carboxamide;
2-(4-(1H-Imidazol-1-yl)phenyl)-9-(2-isopropylphenyl)-8-oxo-8,9-dihydro-7H-purine-6-carboxamide;
9-(2-Cyclohexylphenyl)-2-(3-hydroxyphenyl)-8-oxo-8,9-dihydro-7H-purine-6-carboxamide;
2-(4-(1H-Imidazol-2-yl)phenyl)-9-(2-isopropylphenyl)-8-oxo-8,9-dihydro-7H-purine-6-carboxamide;
2-(1H-Benzo[d]imidazol-1-yl)-9-(2-methoxyphenyl)-8-oxo-8,9-dihydro-7H-purine-6-carboxamide;
2-(1H-Imidazo[4,5-b]pyridin-6-yl)-9-(2-isopropylphenyl)-8-oxo-8,9-dihydro-7H-purine-6-carboxamide;
9-(2-Isopropylphenyl)-8-oxo-2-(1H-pyrrolo[2,3-b]pyridin-5-yl)-8,9-dihydro-7H-purine-6-carboxamide;
2-(1H-Imidazo[4,5-b]pyridin-6-yl)-8-oxo-9-(2-(trifluoromethyl)phenyl)-8,9-dihydro-7H-purine-6-carboxamide;
9-(2-Methoxyphenyl)-2-(2-(methylthio)-1H-benzo[d]imidazol-5-yl)-8-oxo-8,9-dihydro-7H-purine-6-carboxamide;
2-(1H-Indol-5-yl)-9-(2-isopropylphenyl)-8-oxo-8,9-dihydro-7H-purine-6-carboxamide;
9-(Cyclohexylmethyl)-2-(3-hydroxyphenyl)-8-oxo-8,9-dihydro-7H-purine-6-carboxamide;
9-(2,3-Dihydro-1H-inden-1-yl)-2-(3-hydroxyphenyl)-8-oxo-8,9-dihydro-7H-purine-6-carboxamide;
2-(3-Hydroxyphenyl)-9-isobutyl-8-oxo-8,9-dihydro-7H-purine-6-carboxamide;
9-(trans-4-Methoxycyclohexyl)-2-(3-hydroxyphenyl)-8-oxo-8,9-dihydro-7H-purine-6-carboxamide;
9-(cis-4-Methoxycyclohexyl)-2-(3-hydroxyphenyl)-8-oxo-8,9-dihydro-7H-purine-6-carboxamide;
2-(3-Hydroxyphenyl)-8-oxo-9-(5,6,7,8-tetrahydronaphthalen-1-yl)-8,9-dihydro-7H-purine-6-carboxamide;
2-(4-(1H-1,2,4-Triazol-3-yl)phenyl)-9-cyclohexyl-8-oxo-8,9-dihydro-7H-purine-6-carboxamide;
2-(3-Hydroxyphenyl)-9-(1H-indol-4-yl)-8-oxo-8,9-dihydro-7H-purine-6-carboxamide;
9-(2-Fluoro-3-methoxyphenyl)-2-(3-hydroxyphenyl)-8-oxo-8,9-dihydro-7H-purine-6-carboxamide;
9-(2-Fluoro-5-methoxyphenyl)-2-(3-hydroxyphenyl)-8-oxo-8,9-dihydro-7H-purine-6-carboxamide;
9-Cyclohexyl-2-(1H-imidazo[4,5-b]pyridin-6-yl)-8-oxo-8,9-dihydro-7H-purine-6-carboxamide;
2-(3-Hydroxyphenyl)-8-oxo-9-(tetrahydro-2H-pyran-4-yl)-8,9-dihydro-7H-purine-6-carboxamide;
2-(3-Hydroxyphenyl)-8-oxo-9-((tetrahydro-2H-pyran-4-yl)methyl)-8,9-dihydro-7H-purine-6-carboxamide;
9-(2-Cyclopentylphenyl)-2-(3-hydroxyphenyl)-8-oxo-8,9-dihydro-7H-purine-6-carboxamide;
2-(3-Hydroxyphenyl)-8-oxo-9-(piperidin-4-yl)-8,9-dihydro-7H-purine-6-carboxamide;
9-(2-Fluoro-4-methoxyphenyl)-2-(3-hydroxyphenyl)-8-oxo-8,9-dihydro-7H-purine-6-carboxamide;
2-(1H-benzo[d]imidazol-6-yl)-9-cyclohexyl-8-oxo-8,9-dihydro-7H-purine-6-carboxamide;
2-Benzimidazol-6-yl-9-(trans-4-methoxycyclohexyl)-8-oxo-7-hydropurine-6-carboxamide;
2-(4-(Aminomethyl)phenyl)-9-(2-methoxyphenyl)-8-oxo-8,9-dihydro-7H-purine-6-carboxamide;
2-(3-Hydroxyphenyl)-9-(cis-4-(methoxymethyl)cyclohexyl)-8-oxo-8,9-dihydro-7H-purine-6-carboxamide;
9-(trans-4-Aminocyclohexyl)-2-(3-hydroxyphenyl)-8-oxo-8,9-dihydro-7H-purine-6-carboxamide;
2-(3-Hydroxyphenyl)-9-(2-isobutylphenyl)-8-oxo-8,9-dihydro-7H-purine-6-carboxamide;
(R)-2-(3-Hydroxyphenyl)-8-oxo-9-(tetrahydrofuran-3-yl)-8,9-dihydro-7H-purine-6-carboxamide;
(S)-2-(3-Hydroxyphenyl)-8-oxo-9-(tetrahydrofuran-3-yl)-8,9-dihydro-7H-purine-6-carboxamide;
2-(3-(Aminomethyl)phenyl)-9-(2-methoxyphenyl)-8-oxo-8,9-dihydro-7H-purine-6-carboxamide;
2-(4-(1H-1,2,3-Triazol-5-yl)phenyl)-9-(2-isopropylphenyl)-8-oxo-8,9-dihydro-7H-purine-6-carboxamide;
2-(4-(1H-1,2,4-Triazol-3-yl)phenyl)-9-(cis-4-methoxycyclohexyl)-8-oxo-8,9-dihydro-7H-purine-6-carboxamide;
2-(1H-Benzo[d]imidazol-6-yl)-9-(cis-4-methoxycyclohexyl)-8-oxo-8,9-dihydro-7H-purine-6-carboxamide;
2-(1H-Imidazo[4,5-b]pyridin-6-yl)-9-(cis-4-methoxycyclohexyl)-8-oxo-8,9-dihydro-7H-purine-6-carboxamide;
2-(3-Hydroxyphenyl)-9-((1r,4r)-4-(methoxymethyl)cyclohexyl)-8-oxo-8,9-dihydro-7H-purine-6-carboxamide; and
9-(2-Isopropylphenyl)-2-(4-(5-methyl-4H-1,2,4-triazol-3-yl)phenyl)-8-oxo-8,9-dihydro-7H-purine-6-carboxamide,
and pharmaceutically acceptable salts, clathrates, solvates, stereoisomers, tautomers, and prodrugs thereof.

As also disclosure herein, the TOR kinase inhibitors include compounds having the following formula (III): 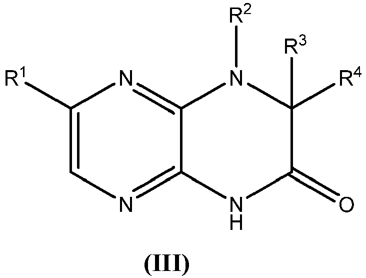 and pharmaceutically acceptable salts, clathrates, solvates, stereoisomers, tautomers, and prodrugs thereof, wherein:
R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl;
R² is H, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl;
R³ and R⁴ are each independently H, substituted or unsubstituted C₁₋₈ alkyl, substituted or unsubstituted aryl, substituted or unsubstituted cycloalkyl, substituted or unsubstituted heterocyclyl, substituted or unsubstituted heterocyclylalkyl, substituted or unsubstituted aralkyl, substituted or unsubstituted cycloalkylalkyl, or R³ and R⁴, together with the atoms to which they are attached, form a substituted or unsubstituted cycloalkyl or substituted or unsubstituted heterocyclyl;
or R² and one of R³ and R⁴, together with the atoms to which they are attached, form a substituted or unsubstituted heterocyclyl,
wherein in certain examples, the TOR kinase inhibitors do not include the compounds depicted below, namely: 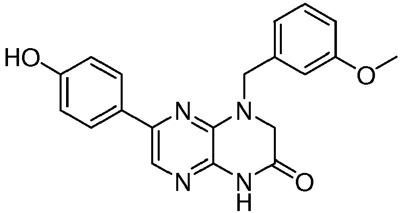 6-(4-hydroxyphenyl)-4-(3-methoxybenzyl)-3,4-dihydropyrazino[2,3-b]pyrazin-2(1H)-one; 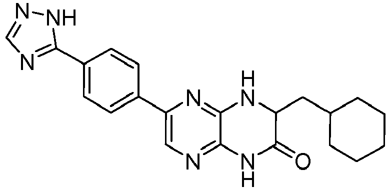 6-(4-(1H-1,2,4-triazol-5-yl)phenyl)-3-(cyclohexylmethyl)-3,4-dihydropyrazino[2,3-b]pyrazin-2(1H)-one;
   or, 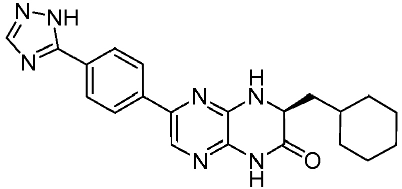 (R)-6-(4-(1H-1,2,4-triazol-5-yl)phenyl)-3-(cyclohexylmethyl)-3,4-dihydropyrazino[2,3-b]pyrazin-2(1H)-one.

In some examples of compounds of formula (III), the compound at a concentration of 10 µM inhibits mTOR, DNA-PK, or PI3K or a combination thereof, by at least about 50%. Compounds of formula (III) may be shown to be inhibitors of the kinases above in any suitable assay system.

Representative TOR kinase inhibitors of formula (III) as disclosed herein include:
6-(1H-pyrrolo[2,3-b]pyridin-3-yl)-4-(2-(tetrahydro-2H-pyran-4-yl)ethyl)-3,4-dihydropyrazino[2,3-b]pyrazin-2(1H)-one;
6-(4-methyl-6-(1H-1,2,4-triazol-3-yl)pyridin-3-yl)-4-((tetrahydro-2H-pyran-4-yl)methyl)-3,4-dihydropyrazino[2,3-b]pyrazin-2(1H)-one;
6-(5-fluoro-2-methyl-4-(1H-1,2,4-triazol-3-yl)phenyl)-4-((*trans*-4-methoxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-((*cis*-4-methoxycyclohexyl)methyl)-3,4-dihydropyrazino[2,3-b]pyrazin-2(1H)-one;
6-(6-(1H-1,2,4-triazol-3-yl)pyridin-3-yl)-4-((*trans*-4-methoxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-((*trans*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-((*cis*-4-methoxycyclohexyl)methyl)-3,4-dihydropyrazino[2,3-b]pyrazin-2(1H)-one;
6-(6-(1H-1,2,4-triazol-3-yl)pyridin-3-yl)-4-((*trans*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-(*cis-*4-hydroxycyclohexyl)-3,4-dihydropyrazino[2,3-b]pyrazin-2(1H)-one;
6-(6-(1H-1,2,4-triazol-3-yl)pyridin-3-yl)-4-((*cis*-4-hydroxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-(*trans*-4-methoxycyclohexyl)-3,4-dihydropyrazino[2,3-b]pyrazin-2(1H)-one;
6-(6-(1H-1,2,4-triazol-3-yl)pyridin-3-yl)-4-(*trans*-4-methoxycyclohexyl)-3,4-dihydropyrazino[2,3-b]pyrazin-2(1H)-one;
6-(6-(1H-1,2,4-triazol-3-yl)pyridin-3-yl)-4-(*trans*-4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-((*cis*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-(*cis*-4-methoxycyclohexyl)-3,4-dihydropyrazino[2,3-b]pyrazin-2(1 H)-one;
6-(6-(1H-1,2,4-triazol-3-yl)pyridin-3-yl)-4-(2-methoxyethyl)-3,4-dihydropyrazino[2,3-b]pyrazin-2(1H)-one;
6-(6-(1H-1,2,4-triazol-3-yl)pyridin-3-yl)-4-isopropyl-3,4-dihydropyrazino[2,3-b]pyrazin-2(1H)-one;
6-(5-fluoro-2-methyl-4-(1H-1,2,4-triazol-3-yl)phenyl)-4-(*cis*-4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(*cis*-4-methoxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(2-methoxyethyl)-3,4-dihydropyrazino[2,3-b]pyrazin-2(1H)-one;
6-(6-(1H-1,2,4-triazol-3-yl)pyridin-3-yl)-4-(tetrahydro-2H-pyran-4-yl)-3,4-dihydropyrazino[2,3-b]pyrazin-2(1H)-one;
6-(6-(1H-1,2,4-triazol-3-yl)pyridin-3-yl)-4-ethyl-3,4-dihydropyrazino[2,3-b]pyrazin-2(1H)-one;
6-(5-fluoro-2-methyl-4-(1H-1,2,4-triazol-3-yl)phenyl)-4-(*trans*-4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(tetrahydro-2H-pyran-4-yl)-3,4-dihydropyrazino[2,3-b]pyrazin-2(1H)-one;
6-(5-fluoro-2-methyl-4-(1H-1,2,4-triazol-3-yl)phenyl)-4-isopropyl-3,4-dihydropyrazino[2,3-b]pyrazin-2(1H)-one;
4-ethyl-6-(5-fluoro-2-methyl-4-(1H-1,2,4-triazol-3-yl)phenyl)-3,4-dihydropyrazino[2,3-b]pyrazin-2(1H)-one;
6-(3-fluoro-2-methyl-4-(1H-1,2,4-triazol-3-yl)phenyl)-4-(tetrahydro-2H-pyran-4-yl)-3,4-dihydropyrazino[2,3-b]pyrazin-2(1H)-one;
6-(3-fluoro-2-methyl-4-(1H-1,2,4-triazol-3-yl)phenyl)-4-(*cis*-4-methoxycyclohexyl)-3,4-dihydropyrazino[2,3-b]pyrazin-2(1H)-one;
6-(3-fluoro-2-methyl-4-(1H-1,2,4-triazol-3-yl)phenyl)-4-(*trans*-4-methoxycyclohexyl)-3,4-dihydropyrazino[2,3-b]pyrazin-2(1H)-one;
4-(2-methoxyethyl)-6-(4-methyl-6-(1H-1,2,4-triazol-3-yl)pyridin-3-yl)-3,4-dihydropyrazino[2,3-b]pyrazin-2(1H)-one;
6-(3-(1H-1,2,4-triazol-5-yl)phenyl)-4-(2-(tetrahydro-2H-pyran-4-yl)ethyl)-3,4-dihydropyrazino[2,3-b]pyrazin-2(1H)-one;
5-(8-(2-methoxyethyl)-6-oxo-5,6,7,8-tetrahydropyrazino[2,3-b]pyrazin-2-yl)-4-methylpicolinamide;
3-(6-oxo-8-(2-(tetrahydro-2H-pyran-4-yl)ethyl)-5,6,7,8-tetrahydropyrazino[2,3-b]pyrazin-2-yl)benzamide;
3-(6-oxo-8-(2-(tetrahydro-2H-pyran-4-yl)ethyl)-5,6,7,8-tetrahydropyrazino[2,3-b]pyrazin-2-yl)benzonitrile;
5-(8-(*trans*-4-methoxycyclohexyl)-6-oxo-5,6,7,8-tetrahydropyrazino[2,3-b]pyrazin-2-yl)-4-methylpicolinamide;
6-(1H-imidazo[4,5-b]pyridin-6-yl)-4-(2-(tetrahydro-2H-pyran-4-yl)ethyl)-3,4-dihydropyrazino[2,3-b]pyrazin-2(1H)-one;
6-(1H-indazol-6-yl)-4-(2-(tetrahydro-2H-pyran-4-yl)ethyl)-3,4-dihydropyrazino[2,3-b]pyrazin-2(1H)-one;
4-((1R,3S)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1S,3R)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1R,3R)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1S,3S)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-ethyl-6-(2-methyl-6-(4H-1,2,4-triazol-3-yl)pyridin-3-yl)-3,4-dihydropyrazino[2,3-b]pyrazin-2(1H)-one;
6-(1H-pyrrolo[2,3-b]pyridin-5-yl)-4-(2-(tetrahydro-2H-pyran-4-yl)ethyl)-3,4-dihydropyrazino[2,3-b]pyrazin-2(1H)-one;
6-(1H-indol-6-yl)-4-(2-(tetrahydro-2H-pyran-4-yl)ethyl)-3,4-dihydropyrazino[2,3-b]pyrazin-2(1H)-one;
6-(1H-indol-5-yl)-4-(2-(tetrahydro-2H-pyran-4-yl)ethyl)-3,4-dihydropyrazino[2,3-b]pyrazin-2(1H)-one;
4-(((1R,3S)-3-methoxycyclopentyl)methyl)-6-(2-methyl-6-(4H-1,2,4-triazol-3-yl)pyridin-3-yl)-3,4-dihydropyrazino[2,3-b]pyrazin-2(1H)-one;
4-(((1S,3R)-3-methoxycyclopentyl)methyl)-6-(2-methyl-6-(4H-1,2,4-triazol-3-yl)pyridin-3-yl)-3,4-dihydropyrazino[2,3-b]pyrazin-2(1H)-one;
6-(3-fluoro-2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(3-fluoro-2-methyl-4-(4H-1,2,4-triazol-3-yl)phenyl)-4-(2-methoxyethyl)-3,4-dihydropyrazino[2,3-b]pyrazin-2(1H)-one;
3,3-dimethyl-6-(4-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-((1R,3S)-3-methoxycyclopentyl)-3,4-dihydropyrazino[2,3-b]pyrazin-2(1H)-one;
6-(6-(2-hydroxypropan-2-yl)pyridin-3-yl)-4-((1S,3R)-3-methoxycyclopentyl)-3,4-dihydropyrazino[2,3-b]pyrazin-2(1H)-one;
6-(6-(2-hydroxypropan-2-yl)pyridin-3-yl)-4-(((1S,3S)-3-methoxycyclopentyl)methyl)-3,4-dihydropyrazino[2,3-b]pyrazin-2(1H)-one;
6-(6-(2-hydroxypropan-2-yl)pyridin-3-yl)-4-(((1R,3R)-3-methoxycyclopentyl)methyl)-3,4-dihydropyrazino[2,3-b]pyrazin-2(1H)-one;
6-(6-(2-hydroxypropan-2-yl)pyridin-3-yl)-4-((1S,3S)-3-methoxycyclopentyl)-3,4-dihydropyrazino[2,3-b]pyrazin-2(1H)-one;
6-(6-(2-hydroxypropan-2-yl)pyridin-3-yl)-4-((1R,3R)-3-methoxycyclopentyl)-3,4-dihydropyrazino[2,3-b]pyrazin-2(1H)-one;
6-(6-(2-hydroxypropan-2-yl)pyridin-3-yl)-4-(((1R,3S)-3-methoxycyclopentyl)methyl)-3,4-dihydropyrazino[2,3-b]pyrazin-2(1H)-one;
6-(6-(2-hydroxypropan-2-yl)pyridin-3-yl)-4-(((1S,3R)-3-methoxycyclopentyl)methyl)-3,4-dihydropyrazino[2,3-b]pyrazin-2(1H)-one;
6-(3-fluoro-4-(4H-1,2,4-triazol-3-yl)phenyl)-4-(2-methoxyethyl)-3,4-dihydropyrazino[2,3-b]pyrazin-2(1H)-one;
6-(3-fluoro-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
7'-(2-methyl-4-(4H-1,2,4-triazol-3-yl)phenyl)-1'-((tetrahydro-2H-pyran-4-yl)methyl)-1'H-spiro[cyclopentane-1,2'-pyrazino[2,3-b]pyrazin]-3'(4'H)-one;
7'-(2-methyl-4-(4H-1,2,4-triazol-3-yl)phenyl)-1'-((tetrahydro-2H-pyran-4-yl)methyl)-1'H-spiro[cyclobutane-1,2'-pyrazino[2,3-b]pyrazin]-3'(4'H)-one;
4-(cyclopropylmethyl)-6-(6-(2-hydroxypropan-2-yl)pyridin-3-yl)-3,4-dihydropyrazino[2,3-b]pyrazin-2(1H)-one;
7'-(2-methyl-4-(4H-1,2,4-triazol-3-yl)phenyl)-1'H-spiro[cyclopentane-1,2'-pyrazino[2,3-b]pyrazin]-3'(4'H)-one;
7'-(2-methyl-4-(4H-1,2,4-triazol-3-yl)phenyl)-1'H-spiro[cyclobutane-1,2'-pyrazino[2,3-b]pyrazin]-3'(4'H)-one;
7'-(2-methyl-4-(4H-1,2,4-triazol-3-yl)phenyl)-1'H-spiro[cyclopropane-1,2'-pyrazino[2,3-b]pyrazin]-3'(4'H)-one;
(R)-6-(4-(4H-1,2,4-triazol-3-yl)phenyl)-4-((tetrahydrofuran-2-yl)methyl)-3,4-dihydropyrazino[2,3-b]pyrazin-2(1H)-one;
(S)-6-(4-(4H-1,2,4-triazol-3-yl)phenyl)-4-((tetrahydrofuran-2-yl)methyl)-3,4-dihydropyrazino[2,3-b]pyrazin-2(1H)-one;
6-(1H-indazol-5-yl)-4-(2-(tetrahydro-2H-pyran-4-yl)ethyl)-3,4-dihydropyrazino[2,3-b]pyrazin-2(1H)-one;
4-(6-oxo-8-(2-(tetrahydro-2H-pyran-4-yl)ethyl)-5,6,7,8-tetrahydropyrazino[2,3-b]pyrazin-2-yl)benzamide;
4-(2-methoxyethyl)-3,3-dimethyl-6-(2-methyl-4-(4H-1,2,4-triazol-3-yl)phenyl)-3,4-dihydropyrazino[2,3-b]pyrazin-2(1H)-one;
4-ethyl-3,3-dimethyl-6-(2-methyl-4-(4H-1,2,4-triazol-3-yl)phenyl)-3,4-dihydropyrazino[2,3-b]pyrazin-2(1 H)-one;
6-(2-methyl-4-(4H-1,2,4-triazol-3-yl)phenyl)-3,4-dihydropyrazino[2,3-b]pyrazin-2(1H)-one;
3,3-dimethyl-6-(2-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
(R)-6-(6-(1-hydroxyethyl)pyridin-3-yl)-4-(2-(tetrahydro-2H-pyran-4-yl)ethyl)-3,4-dihydropyrazino[2,3-b]pyrazin-2(1H)-one;
3,3-dimethyl-6-(2-methyl-4-(4H-1,2,4-triazol-3-yl)phenyl)-4-((tetrahydro-2H-pyran-4-yl)methyl)-3,4-dihydropyrazino[2,3-b]pyrazin-2(1H)-one;
6-(6-(2-hydroxypropan-2-yl)-4-methylpyridin-3-yl)-4-(*trans*-4-methoxycyclohexyl)-3,4-dihydropyrazino[2,3-b]pyrazin-2(1H)-one;
6-(6-(2-hydroxypropan-2-yl)-4-methylpyridin-3-yl)-4-((tetrahydro-2H-pyran-4-yl)methyl)-3,4-dihydropyrazino[2,3-b]pyrazin-2(1H)-one;
3,3-dimethyl-6-(2-methyl-4-(4H-1,2,4-triazol-3-yl)phenyl)-3,4-dihydropyrazino[2,3-b]pyrazin-2(1H)-one;
3,3-dimethyl-6-(2-methyl-6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
6-(6-(2-hydroxypropan-2-yl)-2-methylpyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)-2-methylpyridin-3-yl)-4-(*trans*-4-methoxycyclohexyl)-3,4-dihydropyrazino[2,3-b]pyrazin-2(1H)-one;
(S)-6-(6-(1-hydroxyethyl)pyridin-3-yl)-4-(2-(tetrahydro-2H-pyran-4-yl)ethyl)-3,4-dihydropyrazino[2,3-b]pyrazin-2(1H)-one;
3,3-dimethyl-6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(6-(2-hydroxypropan-2-yl)pyridin-3-yl)-3,3-dimethyl-4-(2-(tetrahydro-2H-pyran-4-yl)ethyl)-3,4-dihydropyrazino[2,3-b]pyrazin-2(1H)-one;
6-(4-(2-hydroxypropan-2-yl)phenyl)-4-(*trans*-4-methoxycyclohexyl)-3,4-dihydropyrazino[2,3-b]pyrazin-2(1 H)-one;
6-(4-(2-hydroxypropan-2-yl)phenyl)-4-((*trans*-4-methoxycyclohexyl)methyl)-3,4-dihydropyrazino[2,3-b]pyrazin-2(1H)-one;
4-(*cis-*4-methoxycyclohexyl)-6-(2-methyl-6-(4H-1,2,4-triazol-3-yl)pyridin-3-yl)-3,4-dihydropyrazino[2,3-b]pyrazin-2(1H)-one;
4-(*trans*-4-methoxycyclohexyl)-6-(2-methyl-6-(4H-1,2,4-triazol-3-yl)pyridin-3-yl)-3,4-dihydropyrazino[2,3-b]pyrazin-2(1H)-one;
6-(4-(2-hydroxypropan-2-yl)phenyl)-4-((tetrahydro-2H-pyran-4-yl)methyl)-3,4-dihydropyrazino[2,3-b]pyrazin-2(1H)-one;
4-(2-methoxyethyl)-6-(2-methyl-6-(4H-1,2,4-triazol-3-yl)pyridin-3-yl)-3,4-dihydropyrazino[2,3-b]pyrazin-2(1H)-one;
9-(6-(4H-1,2,4-triazol-3-yl)-3-pyridyl)-6,11,4a-trihydromorpholino[4,3-e]pyrazino[2,3-b]pyrazin-5-one;
6-(2-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
5-(8-(*cis*-4-methoxycyclohexyl)-6-oxo-5,6,7,8-tetrahydropyrazino[2,3-b]pyrazin-2-yl)-6-methylpicolinonitrile;
6-(6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
9-(4-(4H-1,2,4-triazol-3-yl)-2-methylphenyl)-3-(2-methoxyacetyl)-6,11,4a-trihydropiperazino[1,2-e]pyrazino[2,3-b]pyrazin-5-one;
9-(4-(4H-1,2,4-triazol-3-yl)-2-methylphenyl)-6,11,4a-trihydropiperazino[1,2-e]pyrazino[2,3-b]pyrazin-5-one;
9-(4-(4H-1,2,4-triazol-3-yl)-2-methylphenyl)-3-(2-methoxyethyl)-6,11,4a-trihydropiperazino[1 ,2-e]pyrazino[2,3-b]pyrazin-5-one;
4-(cyclopentylmethyl)-6-(2-methyl-6-(4H-1,2,4-triazol-3-yl)pyridin-3-yl)-3,4-dihydropyrazino[2,3-b]pyrazin-2(1H)-one;
9-(6-(4H-1,2,4-triazol-3-yl)-2-methyl-3-pyridyl)-6,11,4a-trihydromorpholino[4,3-e]pyrazino[2,3-b]pyrazin-5-one;
4-(*trans*-4-hydroxycyclohexyl)-6-(6-(2-hydroxypropan-2-yl)pyridin-3-yl)-3,4-dihydropyrazino[2,3-b]pyrazin-2(1H)-one;
4-(*cis*-4-hydroxycyclohexyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-((tetrahydrofuran-3-yl)methyl)-3,4-dihydropyrazino[2,3-b]pyrazin-2(1H)-one;
4-(cyclopentylmethyl)-6-(6-(2-hydroxypropan-2-yl)pyridin-3-yl)-3,4-dihydropyrazino[2,3-b]pyrazin-2(1 H)-one;
6-(6-(2-hydroxypropan-2-yl)pyridin-3-yl)-4-neopentyl-3,4-dihydropyrazino[2,3-b]pyrazin-2(1H)-one;
6-(6-(2-hydroxypropan-2-yl)pyridin-3-yl)-4-isobutyl-3,4-dihydropyrazino[2,3-b]pyrazin-2(1H)-one;
3-methyl-6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(6-(2-hydroxypropan-2-yl)pyridin-3-yl)-4-(piperidin-4-yl)-3,4-dihydropyrazino[2,3-b]pyrazin-2(1H)-one;
6-(6-(2-hydroxypropan-2-yl)pyridin-3-yl)-4-(2-(tetrahydro-2H-pyran-3-yl)ethyl)-3,4-dihydropyrazino[2,3-b]pyrazin-2(1H)-one;
8-(4-(4H-1,2,4-triazol-3-yl)-2-methylphenyl)(3aS,2R)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2R,3aR)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2S,3aR)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2S,3aS)-2-methoxy-5,10,3a-trihydropyrazino[2,3-b]pyrrolidino[1,2-e]pyrazin-4-one;
6-(6-(2-hydroxypropan-2-yl)pyridin-3-yl)-4-(3-methoxypropyl)-3,4-dihydropyrazino[2,3-b]pyrazin-2(1H)-one;
(S)-6-(6-(2-hydroxypropan-2-yl)pyridin-3-yl)-4-((tetrahydrofuran-2-yl)methyl)-3,4-dihydropyrazino[2,3-b]pyrazin-2(1H)-one;
(R)-6-(6-(2-hydroxypropan-2-yl)pyridin-3-yl)-4-((tetrahydrofuran-2-yl)methyl)-3,4-dihydropyrazino[2,3-b]pyrazin-2(1H)-one;
6-(2-methyl-6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
9-(4-(4H-1,2,4-triazol-3-yl)-2-methylphenyl)-3-methyl-6,11,4a-trihydropiperazino[1,2-e]pyrazino[2,3-b]pyrazin-5-one;
9-(4-(4H-1,2,4-triazol-3-yl)phenyl)-6,11,4a-trihydromorpholino[4,3-e]pyrazino[2,3-b]pyrazin-5-one;
9-(4-(4H-1,2,4-triazol-3-yl)-2-methylphenyl)-6,11,4a-trihydropiperidino[1,2-e]pyrazino[2,3-b]pyrazin-5-one;
6-(6-(2-hydroxypropan-2-yl)pyridin-3-yl)-4-(*trans*-4-methoxycyclohexyl)-3,4-dihydropyrazino[2,3-b]pyrazin-2(1H)-one;
6-(6-(2-hydroxypropan-2-yl)pyridin-3-yl)-4-(*cis*-4-methoxycyclohexyl)-3,4-dihydropyrazino[2,3-b]pyrazin-2(1H)-one;
6-(6-(2-hydroxypropan-2-yl)pyridin-3-yl)-4-(2-morpholinoethyl)-3,4-dihydropyrazino[2,3-b]pyrazin-2(1H)-one;
6-(6-(2-hydroxypropan-2-yl)pyridin-3-yl)-4-phenethyl-3,4-dihydropyrazino[2,3-b]pyrazin-2(1H)-one;
6-(6-(2-hydroxypropan-2-yl)pyridin-3-yl)-4-(tetrahydro-2H-pyran-4-yl)-3,4-dihydropyrazino[2,3-b]pyrazin-2(1H)-one;
4-(cyclohexylmethyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-((*trans*-4-methoxycyclohexyl)methyl)-3,4-dihydropyrazino[2,3-b]pyrazin-2(1H)-one;
6-(6-(2-hydroxypropan-2-yl)pyridin-3-yl)-4-((*cis*-4-methoxycyclohexyl)methyl)-3,4-dihydropyrazino[2,3-b]pyrazin-2(1H)-one;
(R)-6-(6-(2-hydroxypropan-2-yl)pyridin-3-yl)-4-(tetrahydrofuran-3-yl)-3,4-dihydropyrazino[2,3-b]pyrazin-2(1H)-one;
(S)-6-(6-(2-hydroxypropan-2-yl)pyridin-3-yl)-4-(tetrahydrofuran-3-yl)-3,4-dihydropyrazino[2,3-b]pyrazin-2(1H)-one;
6-(6-(2-hydroxypropan-2-yl)pyridin-3-yl)-4-phenyl-3,4-dihydropyrazino[2,3-b]pyrazin-2(1H)-one;
(S)-6-(6-(2-hydroxypropan-2-yl)pyridin-3-yl)-3-methyl-4-(2-(tetrahydro-2H-pyran-4-yl)ethyl)-3,4-dihydropyrazino[2,3-b]pyrazin-2(1H)-one;
9-[6-(1-hydroxy-isopropyl)-3-pyridyl]-6,11,4a-trihydromorpholino[4,3-e]pyrazino[2,3-b]pyrazin-5-one;
6-(6-(2-hydroxypropan-2-yl)pyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-(2-methoxyethyl)-3,4-dihydropyrazino[2,3-b]pyrazin-2(1H)-one;
6-(2-amino-7-methyl-1H-benzo[d]imidazol-5-yl)-4-(3-(trifluoromethyl)benzyl)-3,4-dihydropyrazino[2,3-b]pyrazin-2(1H)-one;
6-(6-(2-hydroxypropan-2-yl)pyridin-3-yl)-4-(3-(trifluoromethyl)benzyl)-3,4-dihydropyrazino[2,3-b]pyrazin-2(1H)-one;
9-(4-(4H-1,2,4-triazol-3-yl)-2-methylphenyl)-6,11,4a-trihydromorpholino[4,3-e]pyrazino[2,3-b]pyrazin-5-one;
6-(4-methyl-2-(methylamino)-1H-benzo[d]imidazol-6-yl)-4-(2-(tetrahydro-2H-pyran-4-yl)ethyl)-3,4-dihydropyrazino[2,3-b]pyrazin-2(1H)-one;
8-(4-(4H-1,2,4-triazol-3-yl)-2-methylphenyl)-5,10,3a-trihydropyrazino[2,3-b]pyrrolidino[1,2-e]pyrazin-4-one;
6-(4-(4H-1,2,4-triazol-3-yl)phenyl)-4-ethyl-3,4-dihydropyrazino[2,3-b]pyrazin-2(1H)-one;
6-(4-(4H-1,2,4-triazol-3-yl)phenyl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-(2-(tetrahydro-2H-pyran-4-yl)ethyl)-3,4-dihydropyrazino[2,3-b]pyrazin-2(1H)-one;
6-(4-(4H-1,2,4-triazol-3-yl)phenyl)-4-(2-methoxyethyl)-3,4-dihydropyrazino[2,3-b]pyrazin-2(1H)-one;
6-(4-(4H-1,2,4-triazol-3-yl)phenyl)-4-(3-(trifluoromethyl)benzyl)-3,4-dihydropyrazino[2,3-b]pyrazin-2(1 H)-one;
6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(4-methyl-1H-benzo[d]imidazol-6-yl)-4-(2-(tetrahydro-2H-pyran-4-yl)ethyl)-3,4-dihydropyrazino[2,3-b]pyrazin-2(1H)-one;
6-(4-(2-hydroxypropan-2-yl)phenyl)-4-(2-(tetrahydro-2H-pyran-4-yl)ethyl)-3,4-dihydropyrazino[2,3-b]pyrazin-2(1H)-one; and
6-(4-(1H-1,2,4-triazol-5-yl)phenyl)-4-(2-(tetrahydro-2H-pyran-4-yl)ethyl)-3,4-dihydropyrazino[2,3-b]pyrazin-2(1H)-one,
and pharmaceutically acceptable salts, clathrates, solvates, stereoisomers, tautomers, and prodrugs thereof.

As also disclosed herein, the TOR kinase inhibitors include compounds having the following formula (IV): 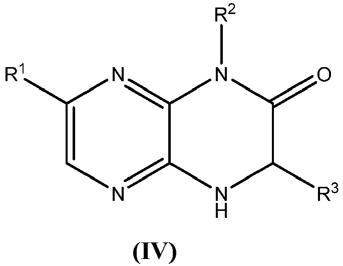 and pharmaceutically acceptable salts, clathrates, solvates, stereoisomers, tautomers, and prodrugs thereof, wherein:
R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl;
R² is H, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl;
R³ is H, or a substituted or unsubstituted C₁₋₈ alkyl,
wherein in certain examples, the TOR kinase inhibitors do not include 7-(4-hydroxyphenyl)-1-(3-methoxybenzyl)-3,4-dihydropyrazino[2,3-b]pyrazin-2(1H)-one, depicted below:

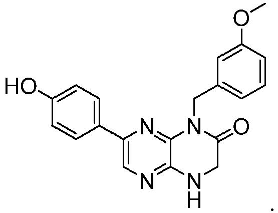

In some examples of compounds of formula (IV), the compound at a concentration of 10 µM inhibits mTOR, DNA-PK, PI3K, or a combination thereof by at least about 50%. Compounds of formula (IV) may be shown to be inhibitors of the kinases above in any suitable assay system. TOR kinase inhibitor of formula (IV) for uses of the present invention is 1-ethyl-7-(2-methyl-6-(1H-1,2,4-triazol-3-yl)pyridin-3-yl)-3,4-dihydropyrazino[2,3-b]pyrazin-2(1H)-one.

Representative TOR kinase inhibitors of formula (IV) as disclosed herein include:
7-(5-fluoro-2-methyl-4-(1H-1,2,4-triazol-3-yl)phenyl)-1-((*trans*-4-methoxycyclohexyl)methyl)-3 ,4-dihydropyrazino[2,3-b]pyrazin-2(1H)-one;
7-(6-(1H-1,2,4-triazol-3-yl)pyridin-3-yl)-1-(*cis*-4-methoxycyclohexyl)-3,4-dihydropyrazino[2,3-b]pyrazin-2(1H)-one;
7-(1H-pyrrolo[2,3-b]pyridin-3-yl)-1-(2-(tetrahydro-2H-pyran-4-yl)ethyl)-3,4-dihydropyrazino[2,3-b]pyrazin-2(1H)-one;
7-(5-fluoro-2-methyl-4-(1H-1,2,4-triazol-3-yl)phenyl)-1-((*cis*-4-methoxycyclohexyl)methyl)-3,4-dihydropyrazino[2,3-b]pyrazin-2(1H)-one;
1-ethyl-7-(1H-pyrrolo[3,2-b]pyridin-5-yl)-3,4-dihydropyrazino[2,3-b]pyrazin-2(1H)-one;
7-(6-(1H-1,2,4-triazol-3-yl)pyridin-3-yl)-1-((*cis*-4-methoxycyclohexyl)methyl)-3,4-dihydropyrazino[2,3-b]pyrazin-2(1H)-one;
7-(1H-benzo[d]imidazol-4-yl)-1-(2-(tetrahydro-2H-pyran-4-yl)ethyl)-3,4-dihydropyrazino[2,3-b]pyrazin-2(1 H)-one;
7-(1H-pyrrolo[2,3-b]pyridin-4-yl)-1-(2-(tetrahydro-2H-pyran-4-yl)ethyl)-3,4-dihydropyrazino[2,3-b]pyrazin-2(1H)-one;
7-(6-(1H-1,2,4-triazol-3-yl)pyridin-3-yl)-1-((*trans*-4-methoxycyclohexyl)methyl)-3,4-dihydropyrazino[2,3-b]pyrazin-2(1H)-one;
7-(6-(1H-1,2,4-triazol-3-yl)pyridin-3-yl)-1-((*trans*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-(*cis*-4-hydroxycyclohexyl)-3,4-dihydropyrazino[2,3-b]pyrazin-2(1 H)-one;
7-(5-fluoro-2-methyl-4-(1H-1,2,4-triazol-3-yl)phenyl)-1-(*cis*-4-hydroxycyclohexyl)-3,4-dihydropyrazino[2,3-b]pyrazin-2(1H)-one;
7-(6-(1H-1,2,4-triazol-3-yl)pyridin-3-yl)-1-(tetrahydro-2H-pyran-4-yl)-3,4-dihydropyrazino[2,3-b]pyrazin-2(1 H)-one;
7-(6-(1H-1,2,4-triazol-3-yl)pyridin-3-yl)-1-(2-methoxyethyl)-3,4-dihydropyrazino[2,3-b]pyrazin-2(1H)-one;
7-(6-(1H-1,2,4-triazol-3-yl)pyridin-3-yl)-1-ethyl-3,4-dihydropyrazino[2,3-b]pyrazin-2(1H)-one;
7-(5-fluoro-2-methyl-4-(1H-1,2,4-triazol-3-yl)phenyl)-1-((*cis*-4-hydroxycyclohexyl)methyl)-3,4-dihydropyrazino[2,3-b]pyrazin-2(1H)-one;
7-(5-fluoro-2-methyl-4-(1H-1,2,4-triazol-3-yl)phenyl)-1-(tetrahydro-2H-pyran-4-yl)-3,4-dihydropyrazino[2,3-b]pyrazin-2(1H)-one;
7-(1H-indol-4-yl)-1-(2-(tetrahydro-2H-pyran-4-yl)ethyl)-3,4-dihydropyrazino[2,3-b]pyrazin-2(1H)-one;
7-(5-fluoro-2-methyl-4-(1H-1,2,4-triazol-3-yl)phenyl)-1-((*trans*-4-hydroxycyclohexyl)methyl)-3 ,4-dihydropyrazino[2,3-b]pyrazin-2(1H)-one;
7-(6-(1H-1,2,4-triazol-3-yl)pyridin-3-yl)-1-((*cis*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-(*trans*-4-hydroxycyclohexyl)-3,4-dihydropyrazino[2,3-b]pyrazin-2(1H)-one;
7-(6-(1H-1,2,4-triazol-3-yl)pyridin-3-yl)-1-(*trans*-4-methoxycyclohexyl)-3,4-dihydropyrazino[2,3-b]pyrazin-2(1H)-one;
7-(6-(1H-1,2,4-triazol-3-yl)pyridin-3-yl)-1-isopropyl-3,4-dihydropyrazino[2,3-b]pyrazin-2(1H)-one;
7-(5-fluoro-2-methyl-4-(1H-1,2,4-triazol-3-yl)phenyl)-1-(*trans*-4-methoxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(*trans*-4-hydroxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(2-methoxyethyl)-3,4-dihydropyrazino[2,3-b]pyrazin-2(1H)-one;
7-(5-fluoro-2-methyl-4-(1H-1,2,4-triazol-3-yl)phenyl)-1-isopropyl-3,4-dihydropyrazino[2,3-b]pyrazin-2(1H)-one;
1-ethyl-7-(5-fluoro-2-methyl-4-(1H-1,2,4-triazol-3-yl)phenyl)-3,4-dihydropyrazino[2,3-b]pyrazin-2(1H)-one;
7-(2-hydroxypyridin-4-yl)-1-(2-(tetrahydro-2H-pyran-4-yl)ethyl)-3,4-dihydropyrazino[2,3-b]pyrazin-2(1H)-one;
1-isopropyl-7-(4-methyl-6-(1H-1,2,4-triazol-3-yl)pyridin-3-yl)-3,4-dihydropyrazino[2,3-b]pyrazin-2(1H)-one;
5-(8-isopropyl-7-oxo-5,6,7,8-tetrahydropyrazino[2,3-b]pyrazin-2-yl)-4-methylpicolinamide;
7-(1H-indazol-4-yl)-1-(2-(tetrahydro-2H-pyran-4-yl)ethyl)-3,4-dihydropyrazino[2,3-b]pyrazin-2(1H)-one;
7-(2-aminopyrimidin-5-yl)-1-(2-(tetrahydro-2H-pyran-4-yl)ethyl)-3,4-dihydropyrazino[2,3-b]pyrazin-2(1H)-one;
7-(2-aminopyridin-4-yl)-1-(2-(tetrahydro-2H-pyran-4-yl)ethyl)-3,4-dihydropyrazino[2,3-b]pyrazin-2(1H)-one;
7-(6-(methylamino)pyridin-3-yl)-1-(2-(tetrahydro-2H-pyran-4-yl)ethyl)-3,4-dihydropyrazino[2,3-b]pyrazin-2(1H)-one;
7-(6-hydroxypyridin-3-yl)-1-(2-(tetrahydro-2H-pyran-4-yl)ethyl)-3,4-dihydropyrazino[2,3-b]pyrazin-2(1H)-one;
7-(4-(1H-pyrazol-3-yl)phenyl)-1-(2-methoxyethyl)-3,4-dihydropyrazino[2,3-b]pyrazin-2(1H)-one;
7-(pyridin-3-yl)-1-(2-(tetrahydro-2H-pyran-4-yl)ethyl)-3,4-dihydropyrazino[2,3-b]pyrazin-2(1H)-one;
7-(1H-indazol-4-yl)-1-(2-methoxyethyl)-3,4-dihydropyrazino[2,3-b]pyrazin-2(1H)-one;
7-(1H-indazol-6-yl)-1-(2-methoxyethyl)-3,4-dihydropyrazino[2,3-b]pyrazin-2(1H)-one;
7-(pyrimidin-5-yl)-1-(2-(tetrahydro-2H-pyran-4-yl)ethyl)-3,4-dihydropyrazino[2,3-b]pyrazin-2(1H)-one;
7-(6-methoxypyridin-3-yl)-1-(2-(tetrahydro-2H-pyran-4-yl)ethyl)-3,4-dihydropyrazino[2,3-b]pyrazin-2(1H)-one;
1-(2-methoxyethyl)-7-(1H-pyrrolo[2,3-b]pyridin-5-yl)-3,4-dihydropyrazino[2,3-b]pyrazin-2(1H)-one;
1-ethyl-7-(1H-pyrrolo[2,3-b]pyridin-5-yl)-3,4-dihydropyrazino[2,3-b]pyrazin-2(1H)-one;
1-ethyl-7-(1H-indazol-4-yl)-3,4-dihydropyrazino[2,3-b]pyrazin-2(1H)-one;
7-(pyridin-4-yl)-1-(2-(tetrahydro-2H-pyran-4-yl)ethyl)-3,4-dihydropyrazino[2,3-b]pyrazin-2(1H)-one;
7-(6-aminopyridin-3-yl)-1-(2-(tetrahydro-2H-pyran-4-yl)ethyl)-3,4-dihydropyrazino[2,3-b]pyrazin-2(1H)-one;
1-methyl-7-(2-methyl-6-(4H-1,2,4-triazol-3-yl)pyridin-3-yl)-3,4-dihydropyrazino[2,3-b]pyrazin-2(1H)-one;
2-(2-hydroxypropan-2-yl)-5-(8-(*trans*-4-methoxycyclohexyl)-7-oxo-5,6,7,8-tetrahydropyrazino[2,3-b]pyrazin-2-yl)pyridine 1-oxide;
4-methyl-5-(7-oxo-8-((tetrahydro-2H-pyran-4-yl)methyl)-5,6,7,8-tetrahydropyrazino[2,3-b]pyrazin-2-yl)picolinamide;
5-(8-((*cis*-4-methoxycyclohexyl)methyl)-7-oxo-5,6,7,8-tetrahydropyrazino[2,3-b]pyrazin-2-yl)-4-methylpicolinamide;
7-(1H-pyrazol-4-yl)-1-(2-(tetrahydro-2H-pyran-4-yl)ethyl)-3,4-dihydropyrazino[2,3-b]pyrazin-2(1H)-one;
1-(*trans*-4-methoxycyclohexyl)-7-(4-methyl-6-(1H-1,2,4-triazol-3-yl)pyridin-3-yl)-3,4-dihydropyrazino[2,3-b]pyrazin-2(1H)-one;
3-((7-(2-methyl-6-(4H-1,2,4-triazol-3-yl)pyridin-3-yl)-2-oxo-3,4-dihydropyrazino[2,3-b]pyrazin-1(2H)-yl)methyl)benzonitrile;
1-((*trans*-4-methoxycyclohexyl)methyl)-7-(4-methyl-6-(1H-1,2,4-triazol-3-yl)pyridin-3-yl)-3,4-dihydropyrazino[2,3-b]pyrazin-2(1H)-one;
3-(7-oxo-8-(2-(tetrahydro-2H-pyran-4-yl)ethyl)-5,6,7,8-tetrahydropyrazino[2,3-b]pyrazin-2-yl)benzamide;
5-(8-((*trans*-4-methoxycyclohexyl)methyl)-7-oxo-5,6,7,8-tetrahydropyrazino[2,3-b]pyrazin-2-yl)-4-methylpicolinamide;
3-((7-(6-(2-hydroxypropan-2-yl)pyridin-3-yl)-2-oxo-3,4-dihydropyrazino[2,3-b]pyrazin-1(2H)-yl)methyl)benzonitrile;
7-(6-(2-hydroxypropan-2-yl)pyridin-3-yl)-1-((1R,3R)-3-methoxycyclopentyl)-3,4-dihydropyrazino[2,3-b]pyrazin-2(1H)-one;
7-(6-(2-hydroxypropan-2-yl)pyridin-3-yl)-1-((1S,3R)-3-methoxycyclopentyl)-3,4-dihydropyrazino[2,3-b]pyrazin-2(1H)-one;
7-(6-(2-hydroxypropan-2-yl)pyridin-3-yl)-1-((1S,3S)-3-methoxycyclopentyl)-3,4-dihydropyrazino[2,3-b]pyrazin-2(1H)-one;
7-(6-(2-hydroxypropan-2-yl)pyridin-3-yl)-1-((1R,3S)-3-methoxycyclopentyl)-3,4-dihydropyrazino[2,3-b]pyrazin-2(1H)-one;
7-(1H-mdazol-6-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-(2-morpholmoethyl)-3,4-dihydropyrazino[2,3-b]pyrazin-2(1H)-one;
1-(*trans*-4-hydroxycyclohexyl)-7-(2-methyl-6-(4H-1,2,4-triazol-3-yl)pyridin-3-yl)-3,4-dihydropyrazino[2,3-b]pyrazin-2(1H)-one;
1-(*cis*-4-hydroxycyclohexyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-(2-morpholinoethyl)-3,4-dihydropyrazino[2,3-b]pyrazin-2(1H)-one;
1-isopropyl-7-(2-methyl-6-(4H-1,2,4-triazol-3-yl)pyridm-3-yl)-3,4-dihydropyrazmo[2,3-b]pyrazin-2(1H)-one;
7-(1H-imidazo[4,5-b]pyridin-6-yl)-1-(2-(tetrahydro-2H-pyran-4-yl)ethyl)-3,4-dihydropyrazino[2,3-b]pyrazin-2(1H)-one;
1-((*cis*-4-methoxycyclohexyl)methyl)-7-(2-methyl-6-(1H-1,2,4-triazol-3-yl)pyridin-3-yl)-3,4-dihydropyrazino[2,3-b]pyrazin-2(1H)-one;
1-(*trans*-4-hydroxycyclohexyl)-7-(6-(2-hydroxypropan-2-yl)pyridin-3-yl)-3,4-dihydropyrazino[2,3-b]pyrazin-2(1H)-one;
1-(*cis*-4-hydroxycyclohexyl)-7-(6-(2-hydroxypropan-2-yl)pyridin-3-yl)-3,4-dihydropyrazino[2,3-b]pyrazin-2(1H)-one;
4-(7-oxo-8-(2-(tetrahydro-2H-pyran-4-yl)ethyl)-5,6,7,8-tetrahydropyrazino[2,3-b]pyrazin-2-yl)benzamide;
7-(1H-indazol-5-yl)-1-(2-(tetrahydro-2H-pyran-4-yl)ethyl)-3,4-dihydropyrazino[2,3-b]pyrazin-2(1H)-one;
7-(1H-pyrrolo[2,3-b]pyridin-5-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-(tetrahydro-2H-pyran-4-yl)-3,4-dihydropyrazino[2,3-b]pyrazin-2(1H)-one;
1-((1S,3R)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
1-((1R,3R)-3-methoxycyclopentyl)-7-(2-methyl-6-(4H-1,2,4-triazol-3-yl)pyridm-3-yl)-3,4-dihydropyrazino[2,3-b]pyrazin-2(1H)-one;
1-((1R,3S)-3-methoxycyclopentyl)-7-(2-methyl-6-(4H-1,2,4-tnazol-3-yl)pyridin-3-yl)-3,4-dihydropyrazino[2,3-b]pyrazin-2(1H)-one;
1-((1S,3S)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
7-(1H-indol-5-yl)-1-(2-(tetrahydro-2H-pyran-4-yl)ethyl)-3,4-dihydropyrazino[2,3-b]pyrazin-2(1H)-one;
7-(1H-indol-6-yl)-1-(2-(tetrahydro-2H-pyran-4-yl)ethyl)-3,4-dihydropyrazino[2,3-b]pyrazin-2(1H)-one;
7-(4-(2-hydroxypropan-2-yl)phenyl)-1-(*trans*-4-methoxycyclohexyl)-3,4-dihydropyrazino[2,3-b]pyrazin-2(1H)-one;
7-(6-(2-hydroxypropan-2-yl)pyridin-3-yl)-1-(tetrahydro-2H-pyran-4-yl)-3,4-dihydropyrazino[2,3-b]pyrazin-2(1H)-one;
1-((*trans*-4-methoxycyclohexyl)methyl)-7-(2-methyl-6-(1H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-((*cis*-4-methoxycyclohexyl)methyl)-3,4-dihydropyrazino[2,3-b]pyrazin-2(1H)-one;
1-(2-methoxyethyl)-7-(4-methyl-2-(methylamino)-1H-benzo[d]imidazol-6-yl)-3,4-dihydropyrazino[2,3-b]pyrazin-2(1H)-one;
7-(7-methyl-2-oxo-2,3-dihydro-1H-benzo[d]imidazol-5-yl)-1-((tetrahydro-2H-pyran-4-yl)methyl)-3,4-dihydropyrazino[2,3-b]pyrazin-2(1H)-one;
7-(2-methyl-4-(4H-1,2,4-triazol-3-yl)phenyl)-3,4-dihydropyrazino[2,3-b]pyrazin-2(1H)-one;
1-(2-methoxyethyl)-7-(4-methyl-6-(1H-1,2,4-triazol-3-yl)pyridin-3-yl)-3,4-dihydropyrazino[2,3-b]pyrazin-2(1H)-one;
1-benzyl-7-(2-methyl-4-(4H-1,2,4-triazol-3-yl)phenyl)-3,4-dihydropyrazmo[2,3-b]pyrazm-2(1H)-one;
7-(3-fluoro-4-(4H-1,2,4-triazol-3-yl)phenyl)-1-(2-methoxyethyl)-3,4-dihydropyrazino[2,3-b]pyrazin-2(1H)-one;
7-(3-fluoro-4-(4H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(3-fluoro-2-methyl-4-(1H-1,2,4-triazol-3-yl)phenyl)-1-(2-methoxyethyl)-3,4-dihydropyrazino[2,3-b]pyrazin-2(1H)-one;
1-(*trans*-4-methoxycyclohexyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-(*trans*-4-methoxycyclohexyl)-3,4-dihydropyrazino[2,3-b]pyrazin-2(1H)-one;
7-(5-fluoro-2-methyl-4-(4H-1,2,4-triazol-3-yl)phenyl)-1-(2-(tetrahydro-2H-pyran-4-yl)ethyl)-3 ,4-dihydropyrazino[2,3-b]pyrazin-2(1H)-one;
7-(3-fluoro-2-methyl-4-(1H-1,2,4-triazol-3-yl)phenyl)-1-(2-(tetrahydro-2H-pyran-4-yl)ethyl)-3,4-dihydropyrazino[2,3-b]pyrazin-2(1H)-one;
1-(2-methoxyethyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-((*trans*-4-methoxycyclohexyl)methyl)-3,4-dihydropyrazino[2,3-b]pyrazin-2(1H)-one;
1-(cyclopentylmethyl)-7-(6-(2-hydroxypropan-2-yl)pyridin-3-yl)-3,4-dihydropyrazino[2,3-b]pyrazin-2(1H)-one;
7-(4-(2-hydroxypropan-2-yl)phenyl)-1-(2-methoxyethyl)-3,4-dihydropyrazino[2,3-b]pyrazin-2(1H)-one;
(S)-7-(6-(1-hydroxyethyl)pyridin-3-yl)-1-(2-(tetrahydro-2H-pyran-4-yl)ethyl)-3,4-dihydropyrazino[2,3-b]pyrazin-2(1H)-one;
(R)-7-(6-(1-hydroxyethyl)pyridin-3-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-((tetrahydro-2H-pyran-4-yl)methyl)-3,4-dihydropyrazino[2,3-b]pyrazin-2(1H)-one;
7-(4-(2-hydroxypropan-2-yl)phenyl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(4-(trifluoromethyl)benzyl)-3,4-dihydropyrazino[2,3-b]pyrazin-2(1H)-one;
7-(6-(2-hydroxypropan-2-yl)pyridin-3-yl)-1-(3-(trifluoromethyl)benzyl)-3,4-dihydropyrazino[2,3-b]pyrazin-2(1H)-one;
7-(6-(2-hydroxypropan-2-yl)pyridin-3-yl)-1-(3-methoxypropyl)-3,4-dihydropyrazino[2,3-b]pyrazin-2(1H)-one;
7-(4-methyl-6-(1H-1,2,4-triazol-3-yl)pyridin-3-yl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(2-methoxyethyl)-3,4-dihydropyrazino[2,3-b]pyrazin-2(1H)-one;
7-(6-(2-hydroxypropan-2-yl)pyridin-3-yl)-1-((tetrahydro-2H-pyran-4-yl)methyl)-3,4-dihydropyrazino[2,3-b]pyrazin-2(1H)-one;
7-(4-methyl-2-(methylamino)-1H-benzo[d]imidazol-6-yl)-1-((tetrahydro-2H-pyran-4-yl)methyl)-3,4-dihydropyrazino[2,3-b]pyrazin-2(1H)-one;
7-(2-amino-4-methyl-1H-benzo[d]imidazol-6-yl)-1-((tetrahydro-2H-pyran-4-yl)methyl)-3,4-dihydropyrazino[2,3-b]pyrazin-2(1H)-one;
7-(2-methyl-6-(4H-1,2,4-triazol-3-yl)pyridin-3-yl)-1-(2-(tetrahydro-2H-pyran-4-yl)ethyl)-3,4-dihydropyrazino[2,3-b]pyrazin-2(1H)-one;
(R)-7-(6-(2-hydroxypropan-2-yl)pyridin-3-yl)-3-methyl-1-(2-(tetrahydro-2H-pyran-4-yl)ethyl)-3,4-dihydropyrazino[2,3-b]pyrazin-2(1H)-one;
(S)-7-(6-(2-hydroxypropan-2-yl)pyridin-3-yl)-3-methyl-1-(2-(tetrahydro-2H-pyran-4-yl)ethyl)-3 ,4-dihydropyrazino[2,3-b]pyrazin-2(1H)-one;
7-(6-(2-hydroxypropan-2-yl)pyridin-3-yl)-3,3 -dimethyl-1 -(2-(tetrahydro-2H-pyran-4-yl)ethyl)-3 ,4-dihydropyrazino[2,3-b]pyrazin-2(1H)-one;
7-(2-amino-4-methyl-1H-benzo[d]imidazol-6-yl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(2-(tetrahydro-2H-pyran-4-yl)ethyl)-3,4-dihydropyrazino[2,3-b]pyrazin-2(1H)-one;
7-(2-methyl-4-(1H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(4-(1H-1,2,4-triazol-5-yl)phenyl)-1-(2-(tetrahydro-2H-pyran-4-yl)ethyl)-3,4-dihydropyrazino[2,3-b]pyrazin-2(1H)-one;
1-(1-hydroxypropan-2-yl)-7-(2-methyl-6-(1H-1,2,4-triazol-3-yl)pyridin-3-yl)-3,4-dihydropyrazino[2,3-b]pyrazin-2(1H)-one; and
1-(2-hydroxyethyl)-7-(2-methyl-6-(1H-1,2,4-triazol-3-yl)pyridin-3-yl)-3,4-dihydropyrazino[2,3-b]pyrazin-2(1 H)-one,
and pharmaceutically acceptable salts, clathrates, solvates, stereoisomers, tautomers, and prodrugs thereof.

### METHODS FOR MAKING TOR KINASE INHIBITORS

The TOR kinase inhibitors can be obtained via standard, well-known synthetic methodology, see *e.g.,* March, J. Advanced Organic Chemistry; Reactions Mechanisms, and Structure, 4th ed., 1992. Starting materials useful for preparing compounds of formula (III) and intermediates therefore, are commercially available or can be prepared from commercially available materials using known synthetic methods and reagents.

Particular methods for preparing compounds disclosed herein may be found, for example in U.S. Patent Nos. 7,981,893, filed October 18, 2007, 7,968,556, filed October 18, 2007, and 8,110,578, filed October 26, 2009.

### METHODS OF USE

Provided herein is a TOR kinase inhibitor for use in methods for treating or preventing prostate cancer as defined in the claims. In certain embodiments, the TOR kinase inhibitor is administered to a patient who has locally advanced, recurrent or metastatic prostate cancer not amenable to curative surgical resection. In another embodiment, the TOR kinase inhibitor is administered to a patient who has received at least one prior line of platinum based chemotherapy. In some embodiments, the TOR kinase inhibitor is administered to a patient who has a tumor showing DNA-PK overexpression.

For the purpose of the invention, the prostate cancer is not ETS overexpressing castration resistant prostate cancer.

In certain embodiments the prostate cancer is rapamycin sensitive prostate cancer. In certain embodiments, the prostate cancer is rapamycin insensitive prostate cancer.

In certain embodiments, provided herein is a TOR kinase inhibitor for use in methods for treating prostate cancer as defined in the claims, wherein the TOR kinase inhibitor inhibits tumor cell proliferation.

In certain embodiments, the TOR kinase inhibitor induces apoptosis in tumor cells.

In certain embodiments, the TOR kinase inhibitor inhibits angiogenesis in tumor cells.

In certain embodiments, the TOR kinase inhibitor inhibits tumor cell proliferation, induces apoptosis of tumor cells, and inhibits angiogenesis.

In certain examples, there are methods for improving the Prostate-Specific Antigen Working Group 2 (PSAWG2) Criteria for prostate cancer (*see* Scher, H., Halab, S., Tannock, S., Morris, M., Sternberg, C. N., et al. Design and end points of clinical trials for patients with progressive prostate cancer and castrate levels of testosterone: Recommendations of the Prostate Cancer Clinical Trials Working Group. J Clin Oncol. 2008; (26) 1148-1159) of a patient, comprising administering an effective amount of a TOR kinase inhibitor to a patient having prostate cancer.

In one example, disclosed herein are methods for inhibiting phosphorylation of S6RP, 4E-BP1 and/or AKT in a patient having prostate cancer, comprising administering an effective amount of a TOR kinase inhibitor to said patient. For the uses of the invention, the inhibition of phosphorylation is assessed in a biological sample of the patient, such as in circulating blood and/or tumor cells, skin biopsies and/or tumor biopsies or aspirate. In such embodiments, the amount of inhibition of phosphorylation is assessed by comparison of the amount of phospho- S6RP, 4E-BP1 and/or AKT before and after administration of the TOR kinase inhibitor. In certain embodiments, provided herein is the TOR kinase inhibitor for use of the invention, wherein the method further comprises measuring inhibition of phosphorylation of S6RP, 4E-BP1 or AKT in a biological sample of a patient having prostate cancer, comprising measuring the amount of phosphorylated S6RP, 4E BP1 and/or AKT in said patient after the administration of the TOR kinase inhibitor as defined in the claims, and comparing said amount of phosphorylated S6RP, 4E BP1 and/or AKT to that of said patient prior to said administration.

In certain embodiments, provided herein is the TOR kinase inhibitor for use of the invention, wherein the method further comprises assessing inhibition of phosphorylation of S6RP, 4E-BP1 and/or AKT in a biological sample of a patient having prostate cancer, comprising comparing the amount of phosphorylated S6RP, 4E-BP1 and/or AKT in a biological sample of a patient obtained prior to and after administration of said TOR kinase inhibitor as defined in the claims, wherein less phosphorylated S6RP, 4E-BP1 and/or AKT in said biological sample obtained after administration of said TOR kinase inhibitor relative to the amount of phosphorylated S6RP, 4E-BP1 and/or AKT in said biological sample obtained prior to administration of said TOR kinase inhibitor indicates inhibition.

In one embodiment, provided herein is the TOR kinase inhibitor for use of the invention, wherein the method further comprises assessing inhibition of DNA-dependent protein kinase (DNA-PK) activity in a skin sample of a patient having prostate cancer as defined in the claims. In some embodiments, DNA-PK inhibition is assessed in a UV light-irradiated skin sample of said patient. In another example, DNA-PK inhibition is assessed in a tumor biopsy or aspirate of a patient having prostate cancer. In one embodiment, inhibition is assessed by measuring the amount of phosphorylated DNA-PK S2056 (also known as pDNA-PK S2056) before and after administration of the TOR kinase inhibitor. In certain embodiments, provided herein is the TOR kinase inhibitor for use of the invention, wherein the method further comprises measuring inhibition of phosphorylation of DNA-PK S2056 in a skin sample of a patient having prostate cancer, comprising measuring the amount of phosphorylated DNA-PK S2056 present in the skin sample after the administration of the TOR kinase inhibitor as defined in the claims, and comparing said amount of phosphorylated DNA-PK S2056 to that in a skin sample from said patient prior to said administration. In one embodiment, the skin sample is irradiated with UV light.

In certain examples, there are methods for inhibiting DNA-dependent protein kinase (DNA-PK) activity in a skin sample of a patient having prostate cancer, comprising administering an effective amount of a TOR kinase inhibitor to said patient and comparing the amount of phosphorylated DNA-PK in a biological sample of a patient obtained prior to and after administration of said TOR kinase inhibitor, wherein less phosphorylated DNA-PK in said biological sample obtained after administration of said TOR kinase inhibitor relative to the amount of phosphorylated DNA-PK in said biological sample obtained prior to administration of said TOR kinase inhibitor indicates inhibition.

In certain embodiments, G1 arrest in a patient is arrested. In certain examples, there are methods for inducing G1 arrest in a biological sample of a patient having prostate cancer, comprising administering an effective amount of a TOR kinase inhibitor to said patient.

In certain embodiments, the growth of rapamycin sensitive prostate cancer in a patient is inhibited. In certain examples, there are methods for inhibiting the growth of rapamycin sensitive prostate cancer in a biological sample of a patient having prostate cancer, comprising administering an effective amount of a TOR kinase inhibitor to said patient.

In certain embodiments, the growth of rapamycin insensitive prostate cancer in a patient is inhibited. In certain examples, there are methods for inhibiting the growth of rapamycin insensitive prostate cancer in a biological sample of a patient having prostate cancer, comprising administering an effective amount of a TOR kinase inhibitor to said patient.

In certain embodiments, pS6 in a patient is inhibited. In certain embodiments, pS6 is inhibited in a biological sample of said patient.

In certain embodiments, pAkt is inhibited by at least 40%, at least 50%, at least 60%, at least 70%, or at least 80% in a patient. In certain embodiments, pAkt is inhibited by at least 40%, at least 50%, at least 60%, at least 70%, or at least 80% in a biological sample of said patient.

In some examples, the TOR kinase inhibitor is a compound as described herein. In one example, the TOR kinase inhibitor is Compound 1 (a TOR kinase inhibitor set forth herein having molecular formula C₂₁H₂₇N₅O₃). In one example, Compound 1 is 7-(6-(2-hydroxypropan-2-yl)pyridin-3-yl)-1-((1r,4r)-4-methoxycyclohexyl)-3,4-dihydropyrazino-[2,3-b]pyrazin-2(1H)-one. The TOR kinase for uses of the present invention is Compound 2, namely 1-ethyl-7-(2-methyl-6-(1H-1,2,4-triazol-3-yl)pyridin-3-yl)-3,4-dihydropyrazino[2,3-b]pyrazin-2(1H)-one having a molecular formula C₁₆H₁₆N₈O.

A TOR kinase inhibitor can be combined with radiation therapy and/or surgery. In certain embodiments, the TOR kinase inhibitor is administered to patient who is undergoing radiation therapy, has previously undergone radiation therapy or will be undergoing radiation therapy. In certain embodiments, the TOR kinase inhibitor is administered to a patient who has undergone tumor removal surgery.

In further embodiments, the patients include patients who have been previously treated for prostate cancer, but are non-responsive to standard therapies, as well as those who have not previously been treated. In further embodiments, the patients include patients who have undergone surgery in an attempt to treat the condition at issue, as well as those who have not. Because patients with prostate cancer may have heterogenous clinical manifestations and varying clinical outcomes, the treatment given to a patient may vary, depending on his/her prognosis. The skilled clinician will be able to readily determine without undue experimentation specific secondary agents, types of surgery, and types of non-drug based standard therapy that can be effectively used to treat an individual patient with prostate cancer.

In one embodiment, the prostate cancer is one in which the PI3K/mTOR pathway is activated. In certain embodiments, the prostate cancer is one in which the PI3K/mTOR pathway is activated due to PTEN loss, a PIK3Ca mutation or EGFR overexpression, or a combination thereof.

### PHARMACEUTICAL COMPOSITIONS AND ROUTES OF ADMINISTRATION

Provided herein are compositions comprising an effective amount of the TOR kinase inhibitor for uses of the present invention and compositions comprising an effective amount of said TOR kinase inhibitor and a pharmaceutically acceptable carrier or vehicle. In some embodiments, the pharmaceutical composition described herein are suitable for oral, parenteral, mucosal, transdermal or topical administration.

The TOR kinase inhibitors can be administered to a patient orally or parenterally in the conventional form of preparations, such as capsules, microcapsules, tablets, granules, powder, troches, pills, suppositories, injections, suspensions and syrups. Suitable formulations can be prepared by methods commonly employed using conventional, organic or inorganic additives, such as an excipient *(e.g.,* sucrose, starch, mannitol, sorbitol, lactose, glucose, cellulose, talc, calcium phosphate or calcium carbonate), a binder *(e.g.,* cellulose, methylcellulose, hydroxymethylcellulose, polypropylpyrrolidone, polyvinylpyrrolidone, gelatin, gum arabic, polyethyleneglycol, sucrose or starch), a disintegrator *(e.g.,* starch, carboxymethylcellulose, hydroxypropylstarch, low substituted hydroxypropylcellulose, sodium bicarbonate, calcium phosphate or calcium citrate), a lubricant *(e.g.,* magnesium stearate, light anhydrous silicic acid, talc or sodium lauryl sulfate), a flavoring agent *(e.g.,* citric acid, menthol, glycine or orange powder), a preservative *(e.g,* sodium benzoate, sodium bisulfite, methylparaben or propylparaben), a stabilizer *(e.g*., citric acid, sodium citrate or acetic acid), a suspending agent *(e.g*., methylcellulose, polyvinyl pyrroliclone or aluminum stearate), a dispersing agent *(e.g.,* hydroxypropylmethylcellulose), a diluent *(e.g.,* water), and base wax *(e.g.,* cocoa butter, white petrolatum or polyethylene glycol). The effective amount of the TOR kinase inhibitor in the pharmaceutical composition may be at a level that will exercise the desired effect; for example, about 0.005 mg/kg of a patient's body weight to about 10 mg/kg of a patient's body weight in unit dosage for both oral and parenteral administration.

The dose of a TOR kinase inhibitor to be administered to a patient is rather widely variable and can be patient to the judgment of a health-care practitioner. The dose for the uses of the present invention is 0.5 mg/day to 128 mg/day. In general, the TOR kinase inhibitors can be administered one to four times a day in a dose of about 0.005 mg/kg of a patient's body weight to about 10 mg/kg of a patient's body weight in a patient, but the above dosage may be properly varied depending on the age, body weight and medical condition of the patient and the type of administration. In one example, the dose is about 0.01 mg/kg of a patient's body weight to about 5 mg/kg of a patient's body weight, about 0.05 mg/kg of a patient's body weight to about 1 mg/kg of a patient's body weight, about 0.1 mg/kg of a patient's body weight to about 0.75 mg/kg of a patient's body weight or about 0.25 mg/kg of a patient's body weight to about 0.5 mg/kg of a patient's body weight. In one example, one dose is given per day In another example, two doses are given per day. In any given case, the amount of the TOR kinase inhibitor administered will depend on such factors as the solubility of the active component, the formulation used and the route of administration.

In another example, there are are methods for the treatment or prevention of a disease or disorder comprising the administration of about 0.375 mg/day to about 750 mg/day, about 0.75 mg/day to about 375 mg/day, about 3.75 mg/day to about 75 mg/day, about 7.5 mg/day to about 55 mg/day or about 18 mg/day to about 37 mg/day of a TOR kinase inhibitor to a patient in need thereof. In a particular embodiment, the dose for administration is of 15 mg/day, 30 mg/day, 45 mg/day or 60 mg/day. In another embodiment, the dose for administration is of 0.5 mg/day, 1 mg/day, 2 mg/day, 4 mg/day, 8 mg/day, 16 mg/day, 20 mg/day, 25 mg/day, 30 mg/day or 40 mg/day.

In another example, there are are methods for the treatment or prevention of a disease or disorder comprising the administration of about 0.1 mg/day to about 1200 mg/day, about 1 mg/day to about 100 mg/day, about 10 mg/day to about 1200 mg/day, about 10 mg/day to about 100 mg/day, about 100 mg/day to about 1200 mg/day, about 400 mg/day to about 1200 mg/day, about 600 mg/day to about 1200 mg/day, about 400 mg/day to about 800 mg/day or about 600 mg/day to about 800 mg/day of a TOR kinase inhibitor to a patient in need thereof. In a particular embodiment, the doses for administration is of 0.5 mg/day, 1 mg/day, 10 mg/day, 15 mg/day, 20 mg/day, 30 mg/day, 40 mg/day, 45 mg/day, 50 mg/day, 60 mg/day, 75 mg/day, 100 mg/day, or 125 mg/day.

In another example, there are unit dosage formulations that comprise between about 0.1 mg and about 2000 mg, about 1 mg and 200 mg, about 35 mg and about 1400 mg, about 125 mg and about 1000 mg, about 250 mg and about 1000 mg, or about 500 mg and about 1000 mg of a TOR kinase inhibitor.

In a particular example, there, are unit dosage formulation comprising about 0.1 mg, 0.25 mg, 0.5 mg, 1 mg, 5 mg, 10 mg, 15 mg, 20 mg, 30 mg, 45 mg, 50 mg, 60 mg, 75 mg, 100 mg, 125 mg, 150 mg, 200 mg, 250 mg, 300 mg, 400 mg, 600 mg or 800 mg of a TOR kinase inhibitor.

In another example, there are unit dosage formulations that comprise 0.1 mg, 0.25 mg, 0.5 mg, 1 mg, 2.5 mg, 5 mg, 10 mg, 15 mg, 20 mg, 30 mg, 35 mg, 50 mg, 70 mg, 100 mg, 125 mg, 140 mg, 175 mg, 200 mg, 250 mg, 280 mg, 350 mg, 500 mg, 560 mg, 700 mg, 750 mg, 1000 mg or 1400 mg of a TOR kinase inhibitor. In a particular example, there are unit dosage formulations that comprise 10 mg, 15 mg, 20 mg, 30 mg, 45 mg or 60 mg of a TOR kinase inhibitor.

A TOR kinase inhibitor can be administered once, twice, three, four or more times daily.

A TOR kinase inhibitor can be administered orally for reasons of convenience. In one embodiment, when administered orally, the TOR kinase inhibitor is administered with a meal and water. In another embodiment, the TOR kinase inhibitor is dispersed in water or juice *(e.g.,* apple juice or orange juice) and administered orally as a suspension. In another embodiment, when administered orally, a TOR kinase inhibitor is administered in a fasted state.

The TOR kinase inhibitor can also be administered intradermally, intramuscularly, intraperitoneally, percutaneously, intravenously, subcutaneously, intranasally, epidurally, sublingually, intracerebrally, intravaginally, transdermally, rectally, mucosally, by inhalation, or topically to the ears, nose, eyes, or skin. The mode of administration is left to the discretion of the health-care practitioner, and can depend in-part upon the site of the medical condition.

In one embodiment, provided herein are capsules containing the TOR kinase inhibitor for uses the present invention without an additional carrier, excipient or vehicle.

In another embodiment, provided herein are compositions comprising an effective amount of the TOR kinase inhibitor for uses of the present invention and a pharmaceutically acceptable carrier or vehicle, wherein a pharmaceutically acceptable carrier or vehicle can comprise an excipient, diluent, or a mixture thereof. In one embodiment, the composition is a pharmaceutical composition.

The compositions can be in the form of tablets, chewable tablets, capsules, solutions, parenteral solutions, troches, suppositories and suspensions and the like. Compositions can be formulated to contain a daily dose, or a convenient fraction of a daily dose, in a dosage unit, which may be a single tablet or capsule or convenient volume of a liquid. In one embodiment, the solutions are prepared from water-soluble salts, such as the hydrochloride salt. In general, all of the compositions are prepared according to known methods in pharmaceutical chemistry. Capsules can be prepared by mixing a TOR kinase inhibitor with a suitable carrier or diluent and filling the proper amount of the mixture in capsules. The usual carriers and diluents include, but are not limited to, inert powdered substances such as starch of many different kinds, powdered cellulose, especially crystalline and microcrystalline cellulose, sugars such as fructose, mannitol and sucrose, grain flours and similar edible powders.

Tablets can be prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. In one embodiment, the pharmaceutical composition is lactose-free. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant might be necessary in a tablet formulation to prevent the tablet and punches from sticking in the die. The lubricant can be chosen from such slippery solids as talc, magnesium and calcium stearate, stearic acid and hydrogenated vegetable oils. Tablet disintegrators are substances that swell when wetted to break up the tablet and release the compound. They include starches, clays, celluloses, algins and gums. More particularly, corn and potato starches, methylcellulose, agar, bentonite, wood cellulose, powdered natural sponge, cation-exchange resins, alginic acid, guar gum, citrus pulp and carboxymethyl cellulose, for example, can be used as well as sodium lauryl sulfate. Tablets can be coated with sugar as a flavor and sealant, or with film-forming protecting agents to modify the dissolution properties of the tablet. The compositions can also be formulated as chewable tablets, for example, by using substances such as mannitol in the formulation.

When it is desired to administer a TOR kinase inhibitor as a suppository, typical bases can be used. Cocoa butter is a traditional suppository base, which can be modified by addition of waxes to raise its melting point slightly. Water-miscible suppository bases comprising, particularly, polyethylene glycols of various molecular weights are in wide use.

The effect of the TOR kinase inhibitor can be delayed or prolonged by proper formulation. For example, a slowly soluble pellet of the TOR kinase inhibitor can be prepared and incorporated in a tablet or capsule, or as a slow-release implantable device. The technique also includes making pellets of several different dissolution rates and filling capsules with a mixture of the pellets. Tablets or capsules can be coated with a film that resists dissolution for a predictable period of time. Even the parenteral preparations can be made long-acting, by dissolving or suspending the TOR kinase inhibitor in oily or emulsified vehicles that allow it to disperse slowly in the serum.

### KITS

In certain embodiments, provided herein are kits comprising the TOR kinase inhibitor for uses of the present invention.

In other embodiments, provide herein are kits comprising the TOR kinase inhibitor for uses of the present invention and means for monitoring patient response to administration of said TOR kinase inhibitor. In particular embodiments, the patient response measured is inhibition of disease progression, inhibition of tumor growth, reduction of primary and/or secondary tumor(s), relief of tumor-related symptoms, improvement in quality of life, delayed appearance of primary and/or secondary tumors, slowed development of primary and/or secondary tumors, decreased occurrence of primary and/or secondary tumors, slowed or decreased severity of secondary effects of disease, arrested tumor growth or regression of tumor.

In other embodiments, provided herein are kits comprising the TOR kinase inhibitor for uses of the present invention and means for measuring the amount of inhibition of phosphorylation of S6RP, 4F-BP1 and/or AKT. In certain embodiments, the kits comprise means for measuring inhibition of phosphorylation of S6RP, 4E-BP1 and/or AKT in circulating blood or tumor cells and/or skin biopsies or tumor biopsies/aspirates of a patient. In certain embodiments, provided herein are kits comprising the TOR kinase inhibitor for uses of the present invention and means for measuring the amount of inhibition of phosphorylation as assessed by comparison of the amount of phospho- S6RP, 4E-BP1 and/or AKT before, during and/or after administration of the TOR kinase inhibitor.

In other embodiments, provided herein are kits comprising the TOR kinase inhibitor for uses of the present invention and means for measuring the amount of inhibition of DNA-dependent protein kinase (DNA-PK) activity. In certain embodiments, the kits comprise means for measuring the amount of inhibition of DNA-dependent protein kinase (DNA-PK) activity in a skin sample and/or a tumor biopsy/aspirate of a patient. In one embodiment, the kits comprise a means for measuring the amount of pDNA-PK S2056 in a skin sample and/or a tumor biopsy/aspirate of a patient. In one embodiment, the skin sample is irradiated by UV light. In certain embodiments, provided herein are kits comprising the TOR kinase inhibitor for uses of the present invention and means for measuring the amount of inhibition of DNA-dependent protein kinase (DNA-PK) activity before, during and/or after administration of the TOR kinase inhibitor. In certain embodiments, provided herein are kits comprising a TOR kinase inhibitor and means for measuring the amount of phosphorylated DNA-PK S2056 before, during and/or after administration of the TOR kinase inhibitor.

In certain examples the kits disclosed herein comprise an amount of a TOR kinase inhibitor effective for treating or preventing prostate cancer. In certain examples, the kits disclosed herein comprise a TOR kinase inhibitor having molecular formula C₂₁H₂₇N₅O₃. In certain examples, the kits disclosed herein comprise Compound 1. The kits provided herein comprise Compound 2 having molecular formula C₁₆H₁₆N₈O.

In certain embodiments, the kits provided herein further comprise instructions for use, such as for administering the TOR kinase inhibitor for uses of the present invention and/or monitoring patient response to administration of said TOR kinase inhibitor.

### EXAMPLES

### IN VITRO STUDIES. (disclosed as reference)

The PC3 and HeLa cell lines described herein were purchased from American Tissue Culture Collection (ATCC). The cells were cultured in growth media as recommended by the vendor.

PC3 tumor cells were plated at 250,000 cells per well in a 6-well plate and allowed to equilibrate overnight in 5% CO₂ at 37 °C. For treatment, the media was aspirated to remove dead/floating cells before addition of 3 mL of media with or without compound. The cells were incubated in the presence of vehicle control (DMSO, 0.2%) or Compound 1 (0.5, 1 or 5 µM) for 24 hours. The cells were then processed for cell cycle analysis. The media was transferred into a 15 mL tube. The cells were trypsinized and transferred to the same tube. The cells were then spun at 12 000 rpm, re-suspended in 500 µL propidium iodide (PI) staining buffer, and incubated at room temperature in the dark for 30 minutes. The sample was then analyzed on a FACSCaliber instrument. The cell cycle experiments were performed on two independent occasions.

The raw data collected from BD FACS Calibur was analyzed by cell cycle software ModFit LT (Verity Software House, Inc). The histogram was generated using the automatic analysis setting. All numbers generated by ModFit LT were transferred to Microsoft Excel and graphed.

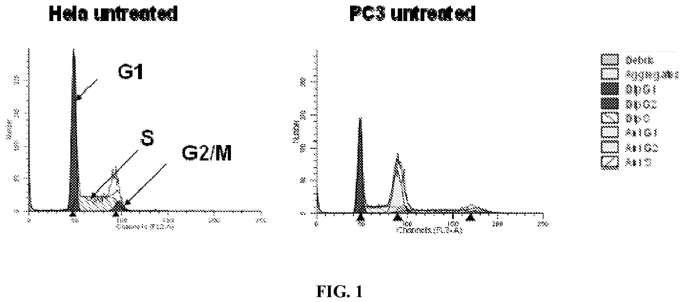

The effects of Compound 1 on cell cycle distribution were evaluated using flow cytometry-based PI staining. As shown in Figure 1, a comparison of the DNA histograms for untreated HeLa and PC3 cells is shown. PC3 cells display an aneuploid phenotype which is evident in the comparison with HeLa cells.

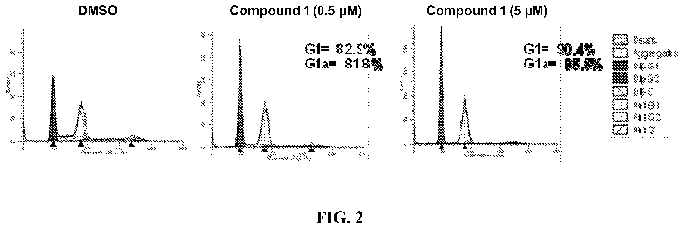

The redistribution of PC3 cells through the cell cycle in response to exposure to Compound 1 at 0.5 and 5 µM compared with DMSO control is shown in Figure 2. There does not appear in this experiment to be a sub-Gl peak indicative of apoptosis at either concentration of Compound 1. Both concentrations of Compound 1 had a marked effect on the cell cycle, indicating a G1 arrest. Quantitation of data from the experiments is shown below in Table 1.

**TABLE 1**

| | **Conc (µM)** | **%G1** | **N** | **%G1a** | **N** |
|---|---|---|---|---|---|
| DMSO | | 59.9 | 2 | 63 | 2 |
| Compound 1 | 0.5 | 81.1 | 2 | 79 | 2 |
| Compound 1 | 1 | 88.6 | 2 | 84.8 | 2 |
| Compound 1 | 5 | 89.2 | 2 | 83.9 | 2 |

Compound 1 was evaluated for its effect on cell cycle distribution in PC3 cells. At concentrations relevant to those required to produce growth inhibition on this cell line, Compound 1 was capable of inducing a G1 arrest. There did not appear to be induction of apoptosis.

### CELLULAR PROFILING OF COMPOUND 1 IN RAPAMYCIN SENSITIVE AND INSENSITIVE CELL LINES (disclosed as reference)

*Study Design.* Antiproliferative activity of Compound 1 was evaluated in prostate tumor cell line. Cells were plated into 96 well plates and after an overnight equilibration period were exposed for 3 days to 0.05, 0.15, 0.5, 1.5 and 5 µM concentrations of Compound 1. When necessary, Compound 1 concentrations were increased to 20 µM. Rapamycin growth inhibition was determined under the same conditions over a concentration range of 0.01 -1 µM or 0.1 - 10 µM, depending on the cell line. All test wells were in triplicate on the plate and in a final volume of 200 µL. The final concentration of DMSO in all wells was 0.2%. Growth inhibition was determined from comparison of Compound 1 with the DMSO control and the extent of cell proliferation measured with WST-1. All growth inhibition experiments were repeated on different occasions at least twice. Potency was determined from calculating the IC₅₀ value from the data describing the growth inhibition curve. A reference compoundwas included in each plate assay to monitor inter-assay variation and the IC₅₀ from the reference was used as part of the acceptance criteria for the assay.

The intracellular inhibition of the mTOR pathway by either Compound 1 or rapamycin over a range of concentrations was monitored from direct and downstream substrates of the TORC1 and TORC2 complexes that contain mTOR kinase activity. Cells were plated as for the proliferation assay and exposed to either Compound 1 or rapamycin for 1 hour prior to assay for the different phospho-proteins. The direct substrate for TORC1 was 4E-BP1(T46) and indirect downstream substrate was S6RP(S235/S236). The direct substrate for TORC2 was Akt(S473) and indirect downstream substrates measured were GSK3B(S9) and PRAS40(T246). Additionally, the phosphorylation status of Akt(T308), a phosphorylation site for PDK1 was monitored.

*Cell Proliferation Assay.* The growth inhibition effect of Compound 1 and rapamycin were determined as follows: cells were plated in 180 µL of growth media in a 96-well flat bottom plate (Costar Catalog Number 33595; the optimum seeding density had been determined previously) and allowed to equilibrate at 37 °C, 5% CO₂ overnight. The following day, Compound 1 or rapamycin compound dilutions were prepared from a 10 mM stock by first diluting to the appropriate concentration in 100% DMSO and then diluted 1:50 into growth media. Next, compound was added to the designated well at a dilution of 1:10 (*i.e*., 20 µL of the diluted compound was added to 180 µL of culture media in each well). The final dilution of compound was 1:500 which yielded a final DMSO concentration of 0.2% in each well. All concentrations were performed in triplicate. Additionally, each plate tested contained cells treated with a reference compound in order to evaluate inter-assay variation and the IC₅₀ generated was used as criteria in accepting the test data. The coefficient of variation for the cell proliferation assay with the reference compound on PC3 cells was 21.5% (n=100). Typical concentrations tested for Compound 1 were: 5, 1.5, 0.5, 0.15, 0.05, and 0.015 µM and for rapamycin were: 1.5, 0.5, 0.15, 0.05, 0.015, and 0.005 µM. Cells were exposed to test compounds for 3 days in 5% CO₂ at 37 °C. The WST-1 assay was used to measure cell proliferation based on measurement of metabolic activity via the reduction of tetrazolium salts to formazan salts. The WST-1 assay is a one-step assay which does not require wash steps. At the end of the 3-day incubation, 20 µL of WST-1 was added to each well and incubated for 1 hour at 5% CO₂ at 37 °C. Absorbance was measured at 450 nm on the VICTOR™ X2 multilabel plate reader (PerkinElmer).

Raw data was saved as a text file and then entered into Activity Base (Cell-based SAR; Prolif-MTT, MTT-6pt-5plates unfixed fit.Version2). The software (XLfit from IDBS) calculated the percent inhibition at each compound concentration by normalizing to the DMSO control values for each set of triplicate wells. Percent inhibition curves were plotted and IC₅₀ values calculated.

*Determination of phospho-Akt(S473), phospho-Akt(T308) and phospho-S6RP(S235*/*5236) from Cell Lysates and the Effect of Compound 1 or Rapamycin.* (disclosed as reference) MSD® biomarker detection assays provide a rapid and convenient method for measuring the total and phosphorylated levels of protein targets within a single small volume sample. These assays are available in both single-plex and multiplex formats. In a single-plex assay, an antibody for a specific protein target is coated on one electrode (or spot) per well. In a multiplex assay, an array of capture antibodies against different targets is patterned on distinct spots on the same well. The single-plex assay for either phospho-Akt or phospho-S6RP is a sandwich immunoassay. MSD provides a plate that has been pre-coated with the capture antibody. The samples (cell lysates) are added to the well with a solution containing the labeled detection antibody labeled with an electrochemiluminescent (ECL) compound, MSD SULFO-TAG™ label, over the course of one or more incubation periods. The total Akt or S6RP present in the sample binds to the capture antibodies immobilized on the working electrode surface; recruitment of the labeled detection antibody by bound phospho-Akt or phospho-S6RP completes the sandwich. MSD Read Buffer that provides the appropriate chemical environment for ECL is added and the plate is read using an MSD SECTOR™ Imager. Inside the SECTOR Imager, a voltage is applied to the plate electrodes which cause the labels bound to the electrode surface to emit light. The instrument measures intensity of the emitted light to afford a quantitative measure of the amount of phosphorylated Akt or S6RP present in the sample.

Cells were plated at the required density and were treated with either Compound 1 or rapamycin over a range of concentrations for 1 hour in 5% CO₂ at 37 °C. Compound 1 was used at the same concentrations as for the proliferation assay. Rapamycin was used at concentrations from 0.001 nM to 100 nM in the assay for p-S6RP(S235/S236) because of its potent activity in this particular assay. After the incubation period, the culture media was removed carefully with an aspirator. The plate was placed on ice and 50 µL of 1X Tris Lysis Buffer was added to each well. The plate was placed on a shaker at 4 °C for 1 hour to lyse the cells. At that point, the plate was either frozen at -80°C for later use or assayed for phosphoproteins. As for the cell proliferation assay, a reference standard was included in the assay for PC3 cells and the coefficient of variation for assay of p-S6RP(S235/S236) was 9.3% (n=50) and for assay of p-Akt(S473) was 28.6% (n=50).

The assay plate was incubated with 150 µL of MSD Blocker A Solution for 1 hour with shaking at room temperature. The plates were washed three times with Tris Wash Buffer. Then 35 µL of cell lysate was added to the wells and incubated for 1 hour with shaking at room temperature. The solution was removed from the wells and the plate was washed three times with wash buffer. Then 150 µL of IX MSD Read Buffer T was added to each well. The plate was read on the SECTOR Imager plate reader. The Excel data was transferred to Activity Base and IC₅₀ values were calculated.

*Determination of phospho-GSKβ(S9), phospho-PRAS40(T246) and phospho-4E-BP1 (T46) and the effect of Compound 1 or Rapamycin.* (disclosed as reference) The multiplex Luminex assay format differs from conventional enzyme-linked immunosorbent assay (ELISA) in that the multiplex capture antibody is attached to a polystyrene bead whereas the ELISA capture antibody is attached to the microplate well. The use of the suspension bead-based technology enables the multiplexing capabilities of the Luminex assays. The xMAP® technology uses 5.6 micron polystyrene microspheres, which are internally dyed with red and infrared fluorophores of differing intensities. Each bead is given a unique number, or bead region, allowing differentiation of one bead from another. Beads covalently bound to different specific antibodies can be mixed in the same assay, utilizing a 96-well microplate format. At the completion of the sandwich immunoassay, beads can be read, using the Luminex 100™ or Luminex 200 detection system, in single-file by dual lasers for classification and quantification of each analyte. The Akt-Pathway Phospho 5-plex kit used includes the ability to simultaneously measure several markers, including p-GSK3β(S9), p-PRAS40(T246), and p-4E-BP1(T46).

Cells were plated at the required density and were treated with either Compound 1 or rapamycin over a range of concentrations for 1 hour in 5% CO₂ at 37 °C. Compound 1 and rapamycin were used at the same concentrations as for the proliferation assay. As for the cell proliferation assay, a reference standard was included in the assay for PC3 cells and the coefficient of variation for assay of p-GSK3β(S9) was 37.8% (n=30), p-PRAS40(T246) was 27.8% (n=30) and for p4E-BP1(T46) was 13.5% (n=4). The media was aspirated from the wells and the plate was placed on ice. Then 35 µL of Lysis Buffer was added to each well and incubated on ice for at least 30 minutes. During the incubation, the standard curve for each phosphoprotein was prepared. The standards provided were reconstituted and serial dilutions were prepared according to the manufacturer's instructions. At the end of the cell lysis step, 36 µL of the lysate was transferred to a V-bottom plate and spun at 4 °C at 2000 rpm for 5 minutes. Both the IX Luminex bead and IX detector antibody solutions were prepared during this time. The IX bead solution was vortexed and 25 µL was added into each well of a black round bottom plate. Then 50 µL of the IX detector antibody was added to each well. Fifty microliters (50 µL) of the prepared standards were added to the designated wells. Then 25 µL of the assay diluent was added to each well designated for the compound-treated cell lysates. At the end of the spin, 25 µL of cell lysate was added to the corresponding wells of the black assay plate. The assay plate was covered with the white plate lid and incubated for 3 hours at room temperature on an orbital shaker.

Approximately 10-15 minutes prior to the end of the first incubation, the secondary antibody solution was prepared according to the vendor's protocol. Goat anti-rabbit R-phycoerythrin red fluorescent protein (RPE) supplied as a 10X concentrate was diluted to generate a IX goat anti-rabbit RPE stock. The 96-well filter plate was pre-wet with 200 µL of IX Working wash solution. At the end of the 3 hour incubation, a multichannel pipette was used to transfer the entire contents from each assay well into the wells of the filter plate. The liquid from the wells was removed by aspiration with the vacuum manifold. Then 200 µL of IX Working wash solution was added into each well. The wash solution was aspirated using the vacuum manifold after 15-30 seconds. This wash step was repeated one time. The bottom of the plate was blotted on clean paper towels to remove residual liquid. One hundred microliters (100 µL) of diluted anti-rabbit RPE was added to each well and incubated for 30 minutes at room temperature on an orbital shaker. The liquid was then aspirated with the vacuum manifold. Then 200 µL of IX Working wash solution was added into each well. The plates were washed three times with the wash solution. The plate was blotted on clean paper towels to remove residual liquid. Then 100 µL of IX Working wash solution was added to each well to re-suspend the beads. The plates were then read on the Luminex 200 detection system. The Excel data was transferred to Activity Base and the IC₅₀ values were calculated.

All data was analyzed using XLfit from IDBS. The formula used for determining IC₅₀ in XLfit was model number 205 which utilizes a 4-parameter logistic model or sigmoidal dose-response model to calculate IC₅₀ values. IC₅₀ values are reported as an average except for those assays that were only done once. The IC₅₀ values obtained from multiple experiments had to be within 2.5-fold of each other to be accepted (for n=2) or within 2.5-fold of the average for (n>2) in order to be accepted as a valid IC₅₀.

*Results.* The potency for growth inhibition was determined for the PC-3 cell line by either Compound 1 or rapamycin. Over the concentration range used for rapamycin, a relatively constant "plateau" effect was observed. As this data did not facilitate the determination of an IC₅₀ value from the growth inhibition curve, relative sensitivities based on rapamycin potency were defined as 0-45% remaining = sensitive, 46-69% remaining = partial and 70-100% remaining = resistant.

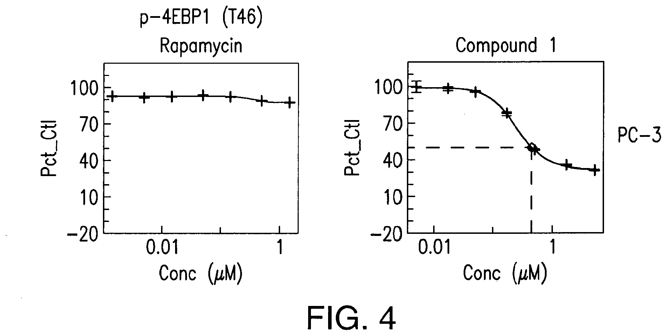

The activity of the TORC1 complex can be followed from the phosphorylation status of 4E-BP1(T46), a direct substrate of mTOR kinase and S6RP(S235/S236), a downstream substrate from mTOR. The activity of the TORC2 complex can be followed directly from the phosphorylation status of Akt(S473) and indirectly from the phosphorylation status of AKT substrates, GSK3β(S9) and PRAS40(T246). The potencies for Compound 1 inhibition of molecular phospho-biomarkers of the mTOR pathway analyzed at the molecular level in PC-3 prostate cancer cells are shown inTable 2. The potency for Compound 1 inhibition of the particular biomarkers 4E-BP1(T46), S6RP(S235/S236), and Akt(S473) for the mTOR pathway have been compared with rapamycin. The data shown in Figure 4 illustrate typical dose response curves for Compound 1 and rapamycin for 4EBP1. Rapamycin demonstrated a remarkable potency in the picomolar range for the inhibition of the indirect substrate, S6RP(S235/S236), a substrate of the p70S6 kinase which is directly activated by the TORC1 complex. The IC₅₀ values for rapamycin were 19 and 29 pM for inhibition of S6RP(S235/S236) phosphorylation in PC3 cells. However, one of the direct TORC1 substrates 4E-BP1(T46) was not inhibited by rapamycin up to concentrations of 10 µM. Compound 1 inhibited phosphorylation of both the direct substrate and downstream substrate at sub-micromolar concentrations. As expected, rapamycin did not inhibit the phosphorylation of Akt(S473) (data not shown), a direct substrate of the TORC2 complex, for which rapamycin is known not to affect. As shown in below in Table 2, Compound 1 was able to produce sub-micromolar IC₅₀ values for the inhibition of both TORC1 and TORC2 direct and indirect substrates and exhibited a sub-micromolar IC₅₀ value for growth inhibition in the PC-3 prostate cancer cell line.

**TABLE 2**

| | **PC3** |
|---|---|
| p-4E-BP1(T46) | 0.41 (n=2) |
| p-S6RP(S235/S236) | 0.02 (n=3) |
| p-Akt (S473) | 0.01 (n=2) |
| p-GSK3β (S9) | 0.23 (n=3) |
| p-PRAS40 (T246) | 0.14 (n=4) |
| p-Akt(T308) | 0.13 (n=2) |
| Compound 1 Growth Inhibition IC₅₀ | 0.11 |

The indirect readouts of TORC2 inhibition, GSK3β(S9) and PRAS40(T246) were less potently inhibited consequent to Compound 1 treatment compared with the effect on Akt(S473). Full activation of Akt requires an additional phosphorylation on T308 that is done by PDK1 and is speculated to be co-dependent with the TORC2 phosphorylation of the Akt(S473). An indication of this is suggested by the data, but further confirmation is required.

*Conclusions.* The growth inhibition potential was determined for Compound 1 in PC3 cells. Using specific molecular phospho-biomarkers of TORC1 activity (4E-BP1(T46) directly and indirectly S6RP(S235/S236) and TORC2 (Akt(S473) directly and indirectly GSK3β(S9) and PRAS40(T246)), Compound 1 was demonstrated to inhibit the mTOR kinase target within the cell in both these complexes. Interestingly, rapamycin was only capable of inhibiting the indirect marker of TORC1 activity and did not affect the phosphorylation of the direct substrate 4E-BP1(T46) and rapamycin did not inhibit the kinase activity of the TORC2 complex, consistent with previous reports. In comparison to the growth inhibitory activity of rapamycin, Compound 1 had the ability to produce, based on the shape of the dose-response curves, a different type of antiproliferative activity in vitro.

### IN-VITRO STUDY OF COMPOUNDS OF FORMULA (III). (disclosed as reference)

Compounds of Formula (III) provided similar potency relative to compounds of formula (I) (Table 3). These analogs maintained selectivity for mTOR over the related lipid kinase PI3K-α, maintaining 65 to >500 fold selectivity for mTOR. In PC3 prostate cancer cell lines, the compounds proved to be inhibitors of both mTOR complexes as measured by inhibition of pS6 (mTORC1) and pAktS473 (mTORC2). Inhibition of cellular proliferation as a functional effect of mTOR pathway inhibition in these cells was also observed.

**TABLE 3**

| Analog | Structure | mTOR IC₅₀ (µM)^{b} | PI₃Kα IC₅₀ (µM)^{b} | PC₃ Cellular Activity | | |
|---|---|---|---|---|---|---|
| | | | | pS6 IC₅₀ (µM)^{b} | pakt IC₅₀ (µM)^{b} | Prolif IC₅₀ (µM)^{b} |
| Compound 3 | 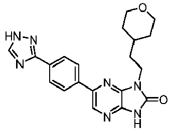 | 0.002 | 1.38 | 0.040 | 0.018 | 0.224 |
| Compound 4 | 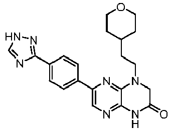 | 0.008 | 0.526 | 0.046 | 0.034 | 0.148 |
| Compound 5 | 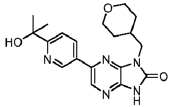 | 0.176 | >30^{c} | 2.41 | 2.55 | 4.18^{c} |
| Compound 6 | 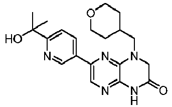 | 0.106 | >30^{c} | 0.386 | 0.315 | 1.55 |

| | | | | | | |
|---|---|---|---|---|---|---|
| ^{b}: average of 2 or more experiments | | | | | | |

While analogs such as Compound 3 and Compound 4 demonstrated low nanomolar on-target potency and corresponding cellular efficacy, these generally suffered from poor oral bioavailability. Comparison of matched-pair Compound 5 and Compound 6 shows the ringexpanded core provides improved cellular efficacy, as assessed by both biomarker and proliferation assays, relative to the parent imidazo[4,5-b]pyrazin-2-one. Compound 6, also showed excellent oral bioavailablity in rodent, allowing for the exploration of mTOR kinase in in vivo sytems.

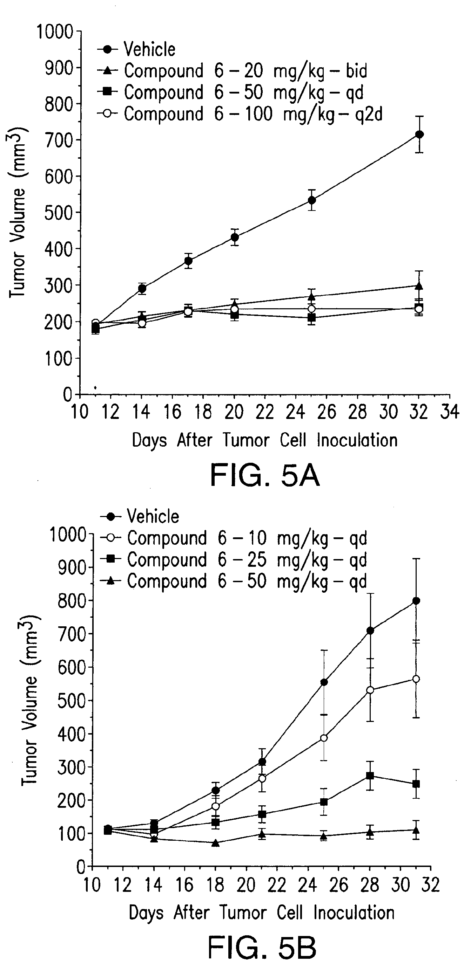

Compound 6, was further profiled for overall kinase selectivity. When tested in a single point assay against 249 kinases, only one kinase other than mTOR was inhibited >80% at 10 µM. Generation of concentration-response curves for the one kinase, FMS, yielded an IC₅₀ value of 2.70 µM. The antitumor activity of Compound 6 in PC3 xenograft model was initially determined using a number of oral dosing paradigms; once or twice daily or every second day. Compound 6 significantly inhibited PC3 tumor growth under these dosing regimens (Figure 5A). The activity was further explored using a number of dose levels with a once daily dosing regimen. In this study, Compound 6 significantly inhibited tumor growth in a dose dependent manner, demonstrating 18%, 63% and 84% tumor volume inhibition compared to vehicle contol at 10, 25 and 50 mg/kg, respectively (Figure 5B). The compound was well tolerated at all dose levels and schedules following 3 weeks of treatment.

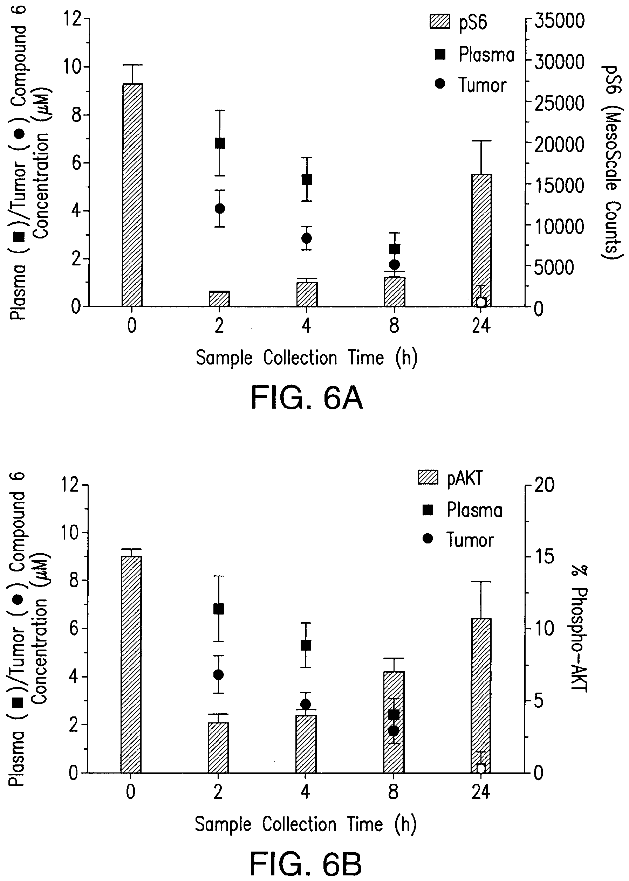

In order to determine the extent of mTOR pathway inhibition and PK/PD relationship, PC3 tumor-bearing mice were administered with a single dose of Compound 6, and plasma and tumor samples were collected at various time points for analysis. Significant inhibition of mTOR pathway markers pS6 and pAktS473 was observed, indicating that the antitumor activity was mediated through the inhibition of both mTORC1 (pS6) and mTORC2 (pAktS473). When dosed at 100 mg/kg, Compound 6 achieved full biomarker inhibition in the PC3 tumor model through 24 hours and compound levels in both tumor and plasma were more than 10 fold above the cellular biomarker IC₅₀ values at 24 hours. When Compound 6 was dosed at 30 mg/kg, pS6 inhibition was maintained at 87-93% from 2-8h, with 43% inhibition at 24 h (Figure 5A). Similarly, inhibition of pAktS473 from 2-8h was 53-77% and fell to 28% at 24h (Figure 5B). These levels of biomarker inhibition correlated well with the plasma and tumor level of compound, which was almost completely cleared by 24h (Figure 6).

### IN VITRO STUDY OF Compound 2

*Antiproliferative Activity.* Antiproliferative activity of Compound 2 was evaluated in a prostate tumor cell line. Cells were plated into 96-well plates and, after an overnight equilibration period, were exposed for 3 days to 0.05, 0.15, 0.5, 1.5, and 5 µM concentrations of Compound 2. The extent of cell proliferation was measured using the WST-1 assay and growth inhibition was determined from comparison of Compound 2-treated samples with the DMSO control. All growth inhibition experiments were repeated on different occasions at least twice. Potency was determined by calculating the IC₅₀ value using the data describing the growth inhibition curve. A reference compound was included in each plate assay to monitor inter-assay variation and the IC₅₀ value for the reference compound was used as part of the acceptance criteria for the assay.

*Intracellular Inhibition of the mTOR Pathway.* The intracellular inhibition of the mTOR pathway by Compound 2 over a range of concentrations was evaluated in PC3 cells by monitoring the direct and downstream substrates of the mTORC1 and mTORC2 complexes, which both contain mTOR kinase activity. Cells were plated as for the proliferation assay and exposed to Compound 2 for 1 hour prior to the assay for the different phosphoproteins. The direct substrate for mTORC1 was 4E-BP1(T46) and the indirect downstream substrate was S6RP (S235/S236). The direct substrate for mTORC2 was AKT (S473) and the indirect downstream substrates measured were GSK3β(S9) and PRAS40 (T246). Additionally, the phosphorylation status of AKT (T308), a phosphorylation site for PDK1, was monitored. Concentration-response curves were plotted and IC₅₀ values determined.

*Cell Proliferation Assay* - *WST-1.* The growth inhibition effect of Compound 2 was determined as follows: cells were plated in 180 µL of growth media in a 96-well flat bottom plate (Costar Catalog Number 33595) at a pre-determined densitiy (PC-3: 3000 cells/well) and allowed to equilibrate at 37 °C, 5% CO₂ overnight.

The following day, Compound 2 compound dilutions were prepared from a 10 mM stock by first diluting to the appropriate concentration in 100% DMSO and then diluted 1:50 into growth media. Next, compound was added to the designated well at a dilution of 1:10 (*i.e.,* 20 µL of the diluted compound was added to 180 µL of culture media in each well). The final dilution of compound was 1:500, which yielded a final DMSO concentration of 0.2% in each well. All concentrations were performed in triplicate. Additionally, each plate tested contained cells treated with a reference compound in order to evaluate interassay variation and the IC₅₀ value generated was used as criteria in accepting the test data. The coefficient of variation for the cell proliferation assay with the reference compound on PC-3 cells was 21.5% (n = 100). Typical concentrations tested for Compound 2 were: 5, 1.5, 0.5, 0.15, and 0.05 µM.

Cells were exposed to test compounds for 3 days in 5% CO₂ at 37 °C. Cell proliferation was assessed using the WST-1 assay, which is based on measurement of metabolic activity via the reduction of tetrazolium salts to formazan salts. The WST-1 assay is a one-step assay not requiring wash steps. At the end of the 3-day incubation, 20 µL of WST-1 (Roche, Catalog # 11 644 807 001) was added to each well and incubated for 1 hour at 5% CO₂ at 37 °C. Absorbance was measured at 450 nm on the VICTOR™ X2 multilabel plate reader (PerkinElmer). Raw data was saved as a text file and then entered into Activity Base (Cell-based SAR; Prolif-MTT, MTT-6pt-5plates unfixed fit Version2). The software (XLfit from IDBS) calculated the percentage inhibition at each concentration of compound by normalizing to the DMSO control values. The percentage inhibition was determined for each replicate and then the 3 values were averaged for each set of triplicate wells. Percentage inhibition curves were plotted and IC₅₀ values calculated.

*Determination of phospho-AKT (S473), phospho-AKT (T308), phospho-S6RP (S235*/*S236)*, *phospho-GSK3beta (S9), and phospho-PRAS40 (T246) in Cell Lysates.* Cells were plated in 96-well flat bottom plates at the required density (PC-3 cells/well) and the cells were allowed to equilibrate at 37 °C, 5% CO₂ overnight. The following day, the cells were treated with Compound 2 over a range of concentrations for 1 hour in 5% CO₂ at 37 °C. Compound 2 was tested at the following concentrations: 10, 3, 1, 0.3, 0.1, 0.03, and 0.01 µM. After the incubation period, the culture media was removed carefully with an aspirator. The plate was placed on ice and 50 µL of 1X Tris Lysis Buffer was added to each well. The plate was placed on a shaker at 4 °C for 1 hour to lyse the cells. At that point, the plate was either frozen at -80 °C for later analysis or immediately assayed for phosphoproteins as described below. To monitor interassay variability, a reference standard was included in the assay for PC-3 cells and the coefficient of variation for assay of p-S6RP (S235/S236) was 9.3% (n = 50) and for assay of p-AKT (S473) was 28.6% (n = 50).

The assay plate was incubated with 150 µL of MSD Blocking Buffer for 1 hour with shaking at room temperature. The plates were washed 3 times with Tris Wash Buffer. Then 35 µL of cell lysate was added to the wells and incubated for 1 hour with shaking at room temperature. The solution was removed from the wells and the plate was washed 3 times with wash buffer. Twenty-five µL of the appropriate antibody solution was then incubated for 1 hour with shaking at room temperature. The solution was removed from the wells and the plate was washed 3 times with wash buffer. Then 150 µL of IX MSD Read Buffer T was added to each well. The plate was read on the SECTOR Imager plate reader.

*Determination of p-4E-BP1 (T46) in Cell Lysates.* Cells were plated at the required density as described in the previous section detailing the Meso Scale assays. The cells were treated with Compound 2 over a range of concentrations for 1 hour in 5% CO₂ at 37 °C. Compound 2 was tested at the following concentrations: 10, 3, 1, 0.3, 0.1, 0.03, and 0.01 µM. After incubation with compound, the culture medium was removed via aspiration. Then the plate was placed on ice and 35 µL of the Invitrogen Cell Extraction Buffer (containing fresh PMSF and protease inhibitor cocktail) were added to each well. The treated cells were allowed to undergo lysis for a total of 30 minutes on ice and then shaken for 2 minutes at 4 °C. At this point, the plate could be either frozen at -80 °C for later analysis or analyzed immediately with the Invitrogen 4E-BP1[pT46] assay kit.

Standards were prepared according to the supplied protocol and 100 µL of each standard was added to appropriate wells. Samples containing Cell Extraction buffer only or diluent buffers only also were included for background controls. Then 85 µL of standard diluent buffer were added to each well for test lysates. After cell lysis was complete, 15 µL of each test lysate were added to appropriate wells of the ELISA plate for a final volume of 100 µL. The plate was gently tapped to distribute test lysates and then allowed to incubate in the dark at room temperature for 2 hours, after which the contents were decanted and washed 4 times with IX wash buffer. Then 100 µL of Detection Antibody (anti-4E-BP1 [pT46]) were added to each well and gently tapped to distribute. Incubation with detection antibody for 1 hour occurred at room temperature in the dark (no shaking). Next, the contents of the plate were decanted and the plate was washed 4 times with 1X wash buffer. Then, 100 µL of the Anti-Rabbit IgG-HRP were added to each well, gently tapped to distribute, and allowed to incubate for 45 minutes at room temperature in the dark (no shaking). The contents of the plate were then decanted and the plate washed 4 times with 1X wash buffer. Next, 100 µL of stabilized chromogen were added to each well, gently tapped, and allowed to react for 20 minutes in the dark at room temperature (no shaking). After 20 minutes, 100 µL of stop solution were added to discontinue the reaction, as confirmed by a color change from blue to yellow. Absorbance was measured at 450 nm on the VICTOR™ X2 multilabel plate reader (Perkin Elmer).

*Determination of phospho-DNA-PK (S2056) in Cell Lysates.* One million cells were plated in each well of a 6-well plate. The cells were treated with Compound 2 at the following concentrations: 10, 3, 1, and 0.3 µM for 4 hours in 5% CO₂ at 37 °C. After incubation with compound, the culture medium was removed via aspiration and the cells were rinsed with PBS. Then the plate was placed on ice and 100 µL of RIPA Buffer (containing PhoSTOP and protease inhibitor cocktail) was added to each well. The cells were scraped and the suspension was transferred to cold Eppendorf tubes and allowed to undergo lysis for a total of 30 minutes on ice. The lysates were then spun at 14,000 rpm for 20 minutes at 4°C to remove cell debris. Cleared lysate was then transferred to a new chilled Eppendorf tube.

The protein concentration of the lysates was determined by the Bio-Rad protein assay (Bio-Rad: Catalog Number 500-0001). Forty µg of protein from each lysate was then combined with LDS loading buffer and reducing agent (both to IX final concentration) and heated for 10 minutes at 70 °C. Samples were then loaded onto NuPAGE Novex 3-8% Tris-Acetate gels and run in 1X Tris-acetate SDS running buffer for 1 hour at 150V. Antioxidant (500 µL) was loaded into the upper buffer chamber of the XCell SureLock™ Mini-Cell as described in the protocol for the gel. Protein was then transferred to a nitrocellulose membrane using a 1X NuPAGE Transfer Buffer/20% methanol solution at 4 °C (100V for 1.5 hours). The membrane was then rinsed with PBS and blocked for 1 hour at room temperature in a 1:1 solution of Odyssey Blocking Buffer and PBS. Primary antibody to detect phosphorylated DNA-PK at Serine 2056 was then diluted 1:1000 into Blocking Buffer/0.1% Tween-20. β-actin was used as a loading control and was diluted 1:20,000. The membrane was incubated at 4 °C overnight with rocking. The following day, the membrane was rinsed 4 times with PBS/0.1% Tween-20 for 5 minutes per wash. The secondary antibodies (Goat anti-rabbit AlexaFluor 680 and Goat anti-mouse IRDye 800) were diluted 1:10,000 into Blocking Buffer/0.1% Tween-20/0.01% SDS and added to the membrane in a black incubation box. This incubation took place for 1 hour at room temperature with rocking. The secondary antibody solution was then removed and the membrane was rinsed 4 times with PBS/0.1% Tween-20 for 5 minutes per wash. A final wash with PBS only was done to reduce background signal. The membranes were then scanned using the Odyssey Infrared Imaging System (LI-COR Biosciences).

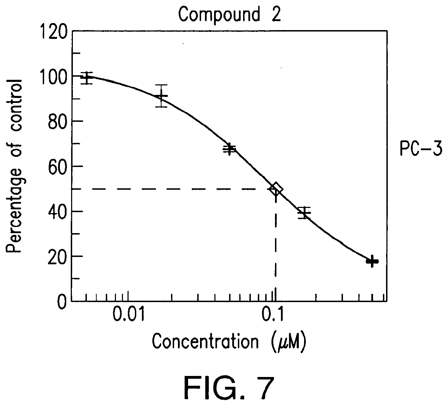

*Results.* The potency of Compound 2 for growth inhibition was determined in the PC3 cancer cell line. Figure 7 provides a typical example for the dose response curves for Compound 2 in the PC-3 cell line. On average, the potency for growth inhibition by Compound 2 as represented by the IC₅₀ value in the PC3 cell line was determined to be 0.14 µM ± 0.019

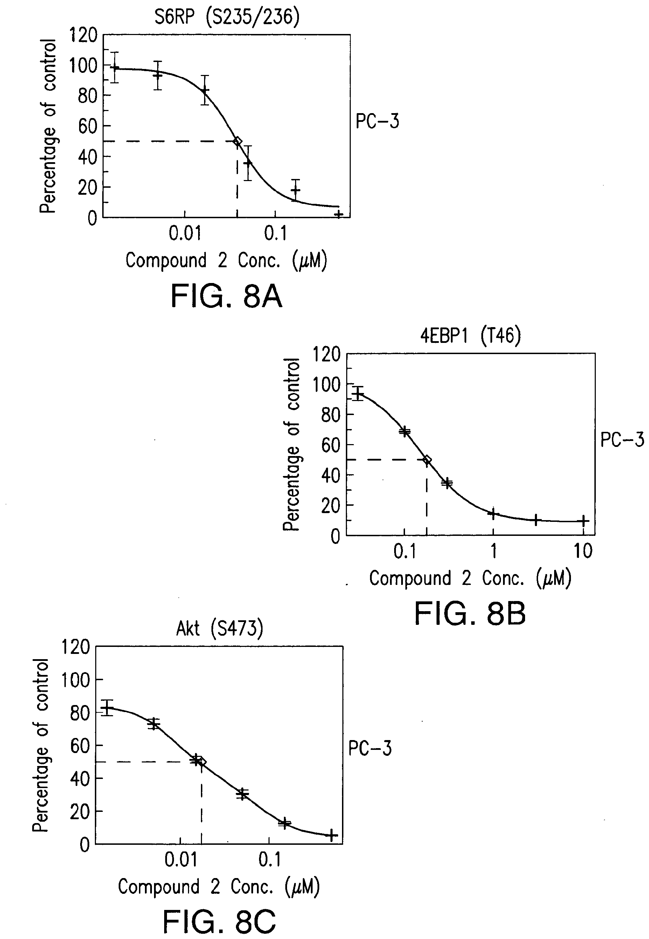

The activity of the mTORC1 complex was followed by assessing the phosphorylation status of 4EBP1 (T46), a direct substrate of mTOR kinase (Figure 8 B), and S6RP (S235/S236), a substrate of the p70S6 kinase that is directly activated by the TORC1 complex (Figure 8 A). The activity of the mTORC2 complex was followed directly by assessing the phosphorylation status of AKT (S473) (Figure 8 C) and indirectly by assessing the phosphorylation status of the AKT substrates GSK3β (S9) and PRAS40 (T246). Additionally, the phosphorylation status of AKT (T308), a phosphorylation site for PDK1, was monitored.

Compound 2 was demonstrated to inhibit the mTOR kinase target in both complexes in intact cells (Table 4). Typical dose response curves for Compound 2 inhibition of these substrates are shown in Figure 8. Inhibition of the direct mTORC1 and mTORC2 substrates 4E-BP1 and AKT was submicromolar. Inhibition of the downstream substrates p-S6RP (S235/S236) and p-PRAS40 (T246) was also submicromolar. The inhibition of p-GSK3β by Compound 2 was less potent than the inhibition of the direct substrate p-AKT (S473) or the other indirect substrate p-PRAS40 (T246). Full activation of AKT requires an additional phosphorylation on T308 by PDK1 and is speculated to be co-dependent with the mTORC2 phosphorylation of AKT (S473).

Potent intracellular inhibition of the mTOR pathway and potent antiproliferative activity were demonstrated with Compound 2 in PC3.

**TABLE 4**

| | **Intracellular Inhibition of mTOR Pathway by Compound 2 (IC50, µM)** | | | | | |
|---|---|---|---|---|---|---|
| | **mTORC1** | | **mTORC2** | | | **PDK1** |
| **Cell Line** | **4EBP1 (T46)** | **S6RP (S235/236)** | **AKT (S473)** | P**RAS40 (T246)** | **GSK3β (S9)** | **AKT (T308)** |
| PC-3 | 0.13 (n = 2) | 0.023 ± 0.007 | 0.022 ± 0.003 | 0.049 (n = 2) | 0.017 (n = 2) | 0.048 (n = 2) |

*Conclusions.* Compound 2 demonstrated potent growth inhibition (IC₅₀ values: 0.14 to 1.31 µM) in prostate tumor cells. Using specific molecular phosphobiomarkers of mTORC1 activity (4E-BP1 (T46) directly and S6RP (S235/S236) indirectly) and mTORC2 activity (AKT (S473) directly and GSK3β (S9) and PRAS40 (T246) indirectly), Compound 2 was demonstrated to inhibit the mTOR kinase target in both of these complexes in the intact cell. Submicromolar inhibition of the direct mTORC1 and mTORC2 substrates 4E-BP1 and AKT (S473) and the downstream substrates

### ANTITUMOR ACTIVITY OF COMPOUND 1 IN THE PC3 HUMAN PROSTATE CANCER XENOGRAFT MODEL (disclosed as reference)

*Tolerability Studies.* Four groups of female SCID mice bearing PC3 tumors (n = 4 per group) were dosed orally with vehicle or Compound 1 (10, 25, or 50 mg/kg) daily for 5 days. Plasma and tumor samples were collected 24 hours after the last dose in each dose group.

*Efficacy Studies.* Groups of female SCID mice bearing PC3 tumors (n = 5 to 10/group) were dosed orally with vehicle or Compound 1 (0.3 mg/kg to 50 mg/kg) either BID, QD, Q2D, Q3D, Q5D, or Q7D throughout the study starting when the tumor volumes reached approximately 100 mm³ or 500 mm³ (regression study). The BID dose groups were dosed with a 10-hour separation between the morning and evening doses. In the positive control group, rapamycin was administered Q3D via the intraperitoneal (IP) route. At the end of each study, plasma and/or tumor samples were collected.

The design of the experiments is shown in Table 5 below.

**TABLE 5**

| **Dose Group (n)** | **Dosing Schedule** | **Dosing Duration** | **Sample Collection Time Points After Last Dose** |
|---|---|---|---|
| Vehicle (n = 9) | QD | 3 weeks | 24 h |
| Rapamycin 4 mg/kg (n=7) | QD | 3 weeks | Not collected |
| Compound 1 10 mg/kg (n=9) | QD | 3 weeks | 2 and 24 h |
| Compound 1 25 mg/kg (n=9) | QD | 2 × 3 weeks | 2 and 24 h |
| Vehicle (n=9) | QD | 3 weeks | 24 h |
| Vehicle (n=5) | QD | 5 days | 2 h after 6th dose for MOA studies on day 6 |
| Rapamycin 4 mg/kg (n=5) | Q3D | 3 weeks | 24 h |
| Compound 1 25 mg/kg (n=9) | QD | 3 weeks | 1, 2, 4, 8, and 24 h |
| Dose Group (n) | Dosing Schedule | Dosing Duration | Sample Collection Time Points After Last Dose |
| Compound 1 25 mg/kg (n=5) | QD | 6 doses, 6 days | 2 h after 6th dose for MOA studies on day 6 |
| Compound 1 25 mg/kg (n=9) | Q2D | 3 weeks | 1, 2, 4, 8, 24, and 48 h |
| Compound 1 25 mg/kg (n=9) | Q3D | 3 weeks | 2, 4, 8, 24, 48, and 72 h |
| Compound 1 50 mg/kg (n=9) | Q3D | 3 weeks | 2, 4, 8, 24, 48, and 72 h |
| Compound 1 50 mg/kg (n=9) | Q5D | 3 weeks | 2, 24, 48, 72, 96, and 120 h |
| Compound 1 50 mg/kg (n=9) | Q7D | 3 weeks | 48, 72, 96, 120, 144, and 168 h |
| Vehicle (n=10) | BID | 3 weeks | 8 h |
| Rapamycin 4 mg/kg (n=6) | Q3D | 3 weeks | 24 h |
| Compound 1 5 mg/kg (n=10) | BID | 3 weeks | 1, 2, 4, 6, 8, 10, and 24 h |
| Compound 1 10 mg/kg (n=10) | BID | 3 weeks | 1, 2, 4, 6, 8, 10, and 24 h |
| Vehicle (n=10) | BID | 3 weeks | 24 h |
| Rapamycin 4 mg/kg (n=10) | Q3D | 3 weeks | 24 h |
| Compound 1 1 mg/kg (n=10) | BID | 3 weeks | 1, 2, 4, 6, 8, 10, and 24 h |
| Compound 1 5 mg/kg (n=10) | BID | 3 weeks | 1, 2, 4, 6, 8, 10, and 24 h |
| Compound 1 10 mg/kg (n=10) | BID | 3 weeks | 1, 2, 4, 6, 8, 10, and 24 h |
| Compound 1 25 mg/kg (n=10) | QD | 3 weeks | None |
| Compound 1 25 mg/kg (n=10) | Q2D | 3 weeks | 1, 2, 4, 8, 24, and 48 h |

*Pharmacokinetic*/*Pharmacodynamic Studies.* Groups of female SCID mice bearing PC3 tumors (n = 4 per group) were dosed orally with a single dose of vehicle or Compound 1. The plasma and tumor samples were collected at various time points following compound administration as described.

Plasma and tumor collection time points were each terminal time points for PK/PD studies.
Compound 1 at 4, 8, and 24 hours; Vehicle control at 24 hours.
Compound 1 at 1, 3, 8, 16, 24, 48, and 72 hours; Vehicle control at 72 hours.
Compound 1 at 1, 3, 8, 16, 24, 48, and 72 hours; Vehicle control at 72 hours.
Compound 1 at 0.5, 2, 4, 6, 10, and 24 hours; Vehicle control at 24 hours.

### Experimental Procedures - Tolerability and Efficacy Studies.

Cell line and culture - PC3 cell line was obtained from American Tissue Culture Collection (ATCC) (Gaithersberg, MD) and grown in growth medium containing Ham's F12K medium with 2 mM L-glutamine adjusted to contain 1.5 g/L sodium bicarbonate and 10% of fetal bovine serum. The cells were detached from tissue culture flasks using trypsin-EDTA. After centrifugation, the cell pellets were suspended in phosphate buffered saline (PBS) and counted using a hemocytometer. Matrigel was added to the cell suspension to adjust the final volume to 2×106 cells/0.1 mL of 1:1 mixture of Matrigel:PBS.

Tumor cell inoculation - Mice were anesthetized with inhaled isoflurane and then inoculated with PC3 tumor cells subcutaneously on the right hind leg with 0.1 mL of single cell suspensions in Matrigel using a sterile 1 mL syringe fitted with a 26 gauge needle. Following inoculation, the mice were returned to microisolator cages.

Randomization of Animals - Following inoculation, the tumors were allowed to grow to about 100 mm³ or 500 mm³ prior to randomization. For efficacy studies, animals with tumors of approximately 100 mm³ were used. The typical number of days required for tumors to reach 100 mm³ was 9 to 11 days. The tumor of each animal was measured and animals with tumors ranging between 100 and 200 mm³ were included in the study. For tolerability and regression studies, animals with tumors of approximately 500 mm³ (range 300-500 mm³) were used. The typical number of days required for tumors to reach 500 mm³ was 20 to 22 days. The animals were then distributed randomly into various cages and the cages were randomly assigned to vehicle, positive control or test article groups. All of the mice were tagged with metal ear tags on the right ear. A typical efficacy study group consisted of 8 to 10 animals. Tolerability study group consisted of 4-6 animals.

Test Article Preparation and Administration - Suspensions of Compound 1 were prepared in aqueous 0.5% CMC and 0.25% Tween-80. The formulations were homogenized using a Teflon™ pestle and mortar (Potter-Elvehjem tissue grinder). Between the doses, the formulated compound was stored under constant stirring using a magnetic stirrer at 4 °C in the dark. The test article and vehicle were administered by oral gavage. The positive control (rapamycin) was prepared as solution in 2% ethanol, 45% polyethyleneglycol 400, and 53% saline and administered by IP injection. Sterile syringes and gavage needles were used for compound administration. All the procedures including injections were done in biosafety cabinets sprayed with 70% ethanol prior to use.

Tolerability Study - The mice were dosed with vehicle or Compound 1 once daily for the duration of the study. Mice were monitored daily for clinical signs and body weight changes. At the end of the study, mice were euthanized and plasma and tumor samples were collected for PK/PD determination. PD markers, pS6 and pAkt, were measured using MSD technology.

Tumor Measurements - Tumor volumes were determined prior to the initiation of treatment and were considered as the starting volumes. Thereafter, tumors were measured twice a week for the duration of the study. The long and short axes of each tumor were measured using a digital caliper in millimeters. The tumor volumes were calculated using the formula: width² × length/2. The tumor volumes were expressed in cubic millimeters (mm³).

Body Weight Meansurements - Initial body weights were recorded prior to the initiation of treatment using a digital scale. The percent body weight change during the course of the study was calculated using initial body weight measurements. Body weights of each animal were measured twice a week at the same time that tumor measurements were taken. Body weights were measured more frequently if significant decreases were noted during the course of the study.

Termination of Experiments - After the last measurement, the experiment was terminated by taking the plasma and tumor samples for PK and PD measurements. Compound concentrations in plasma and tumor were determined by LC-MS/MS. PD markers, pS6 and pAkt, were measured using MSD technology.

Collection of Plasma Samples - Following the last dose of test article, blood samples were collected via retro-orbital bleeds at predetermined time points. Plasma samples were taken over the dosing period (*e.g.,* for Q2D dosed group, plasma samples were taken over 48 hours post dose). The blood samples were processed to plasma using plasma separation tubes with heparin.

Tissue Sample Collection - After the last bleed, the animals were euthanized via CO₂ asphyxiation and the tumors were dissected out. A small section of tumor (about 100 mg) was placed in a pre-weighed vial and snap frozen in liquid nitrogen for compound determination. The remaining tissues were either snap frozen in liquid nitrogen and/or fixed in buffered formalin for PD measurements.

### Experimental Procedures - Pharmacokinetic and Pharmacodynamic Studies

Female SCID mice bearing PC3 tumor ranging between 300 and 500 mm³ were pooled together and randomly distributed to vehicle or Compound 1 treatment groups. At predetermined time points following dosing, mice were euthanized and plasma and tumor samples were collected for PK/PD determination. Compound concentrations in plasma and tumor were determined by LC-MS/MS. PD markers, pS6 and pAkt, were measured using Mesoscale technology.

For Mesoscale discovery assays, tissue samples were homogenized in lysis buffer containing protease and phosphatase inhibitors and centrifuged for 10 minutes at 1200 rpm at 4 °C. Supernatants were sonicated for 1 minute at 4 °C, aliquoted and stored at -80 °C. Protein concentrations in samples were determined using BCA Protein Assay Reagent Kit (Thermo Scientific). Meso Scale Discovery kits and protocol for measuring the pS6 and pAkt/Total Akt kits were used. MSD 96-well plates spotted with pS6, pAkt/total Akt were blocked with blocking solution for 5 minutes followed by a wash with wash buffer. Twenty-five milligrams of tumor lysates were added to each well and incubated for 2 hours followed by a 4X wash with a wash buffer. Detection antibody was added to each well and incubated in the dark for 1 hour at 4 °C. The plates were then washed and bound antigen was read using an MSD SECTOR™ instrument.

### Experimental Procedures - Mechanism of Action Studies

To determine the mechanism of action of Compound 1, mice bearing PC3 tumors were dosed orally with vehicle or Compound 1 at 25 mg/kg QD for 6 days. The positive control, rapamycin, was dosed IP at 4 mg/kg Q3D for 6 days. Two hours after the last dose of either vehicle, Compound 1, or rapamycin, the mice were euthanized and the tumors were dissected. The tumors were snap frozen in liquid nitrogen and processed for immunohistochemistry (IHC) and TUNEL.

Immunohistochemistry - Five to ten micron thick cryostat sections of tumor samples were used for IHC. The expression of a cell proliferation marker Ki-67 was evaluated by IHC using anti-Ki-67 antibody. Anti-CD-31 antibody was used to determine blood vessel density and is a measurement of tumor angiogenesis. Frozen sections were fixed in 4% paraformaldehyde for 10 minutes at room temperature, washed in PBS, blocked and permeabilized with normal goat serum and triton x-100. Sections were then incubated with primary antibody (overnight) followed by incubation with secondary antibody (60 minutes). The sections were washed, counterstained with Hoechst stain and mounted with antifade reagent. For double labeling methods (Ki-67 and CD-31), cocktails of primary and secondary antibodies were used for incubation. Positive and negative controls were included in each assay. Positive controls included the sections that were known to be reactive with the antibody. Negative controls included omission of primary or secondary antibody. The sections were visualized with a Nikon E800 microscope equipped with fluorescence detection equipment and a digital camera attached to a computer.

Apoptosis TUNEL Assay - To detect the apoptotic cells, fluorescence in situ cell death detection kit (Roche Biosciences) was used. Five to ten micron thick cryostat sections were fixed in 4% paraformaldehyde for 15 minutes at room temperature, washed, permeabilized with 0.3% triton X-100 and 0.1% sodium citrate in PBS for 10 minutes. Sections were then washed in PBS and incubated with a labeling solution containing TdT enzyme for 1 hour at 37 °C in the dark. The sections were washed in PBS, counterstained with Hoechst dye (0.4 µg/mL) at room temperature for 10 minutes and mounted in Prolong Gold antifade reagent.

Quantitation of Immunohistochemistry - The tissues sections processed for apoptosis or immunostained for proliferating cells (Ki-67) or blood vessels were quantitated using Metamorph software. Using 20X objective, 5 different fields from each section, 2 to 4 sections from each tumor, and 3 to 4 tumors from each treatment group or control were used for quantitation. The area of interest was expressed as the percent threshold area of the total area.

*Results* - *Tolerability of Compound 1 in PC3 Tumor-bearing Mice.* In preparation for xenograft studies, a 5-day tolerability study was performed. Compound 1 was orally dosed at 10, 25, or 50 mg/kg, QD in PC3 tumor-bearing SCID mice. The animals in the 50 mg/kg dose group lost >17% of the starting body weight by Day 5 (24 hours after the 4th dose). The animals in this group were euthanized. By Day 6 (24 hours after the 5th dose), the animals in the 25 mg/kg and 10 mg/kg groups lost 9% and 4%, respectively, of the starting body weight. Based on these data, 10 and 25 mg/kg doses were selected for the xenograft study.

At the end of the tolerability study, plasma and tumor samples were collected and processed for PK/PD analysis. A high concentration of Compound 1 was observed in the plasma (7.3 ± 2.4 µM) and tumor (4.7 ± 1.3 µM) of mice dosed with 50 mg/kg on day 5, compared to the 10 and 25 mg/kg groups (plasma levels: 0.08 ± 0.06 and 0.84 ± 0.41 µM, respectively). This higher than expected Compound 1 concentration in plasma and tumor is probably due to accumulation of the compound with repeated dosing. At 10 mg/kg dose level, there was a moderate inhibition of pS6 (22%) and pAKT (28%) in the tumors collected 24 hours after the last dose; however, both PD markers were significantly inhibited in the tumors of animals dosed at 25 and 50 mg/kg (data not shown) at the 24 hr timepoint.

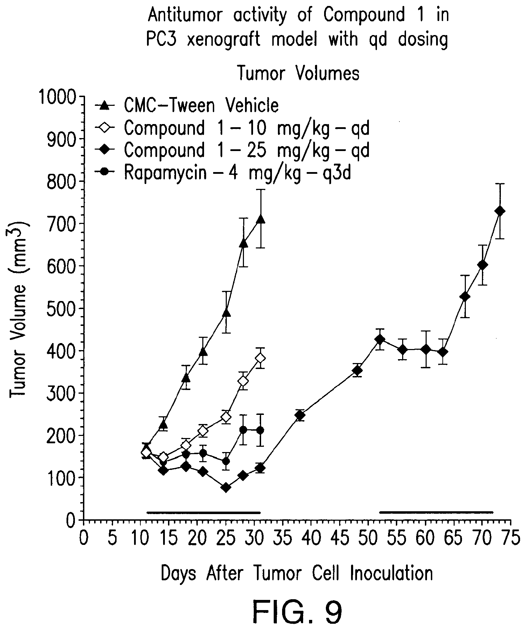

*Results* - *Antitumor Activity of Compound 1 in PC3 Xenograft Model.* The antitumor activity of Compound 1 was initially tested at 10 and 25 mg/kg in a once daily dosing paradigm. Dosing started on Day 11 when the tumor volumes ranged between 158 and 172 mm³. By Day 31, tumors in the vehicle-treated group measured 712 ± 68 mm³. All animals in the positive control group (rapamycin 4 mg/kg Q3D) showed significantly (p < 0.001) smaller tumors when compared with vehicle on Day 31. The % tumor inhibition expressed in Figure 9 for each treatment group is the tumor volume reduction on day 31 when compared with the vehicle control. The Compound 1-treated groups showed dose-dependent tumor inhibition (Figure 9). Tumor regression with Compound 1 at 25 mg/kg was evidenced by a smaller average tumor volume on Day 31 compared with the average starting tumor volume on Day 11 (123 mm³ on Day 31 vs. 158 mm³ on Day 11). On day 31, the tumor volumes from the 10 and 25 mg/kg Compound 1 treated animals were reduced by 46.1% and 86.7%, respectively, compared to the vehicle control group. Both dose levels were significantly different (p<0.001) from the control (712 mm³ vs. 383 mm³, 10 mg/kg and 123 mm³, 25 mg/kg).

On Day 31 all study groups were euthanized with the exception of the 25 mg/kg Compound 1 group. The animals in the 25 mg/kg Compound 1 dose group were allowed to survive without dosing for an additional 3 weeks (bars in Figure 9 indicate the duration of compound treatment). During the absence of dosing, tumor growth resumed. On Day 52 when the average tumor growth reached about 427 mm³, a second daily dosing cycle of Compound 1 (25 mg/kg) was administered for an additional 3 weeks. During the second dosing cycle, tumor stasis was observed for about 11 days. Average tumor volumes remained between 427 mm³ and 397 mm³. Thereafter, tumor re-growth was observed. At the end of the second cycle the tumor volumes were approximately 729 mm³.

No significant change in body weight was observed in the groups dosed with vehicle, Compound 1 at 10 mg/kg, or positive control (data not shown). Mice in the 25 mg/kg dose group lost approximately 10% of their initial body mass by the end of the first cycle. As soon as dosing ceased the animals immediately gained weight. During the second dosing cycle, the body weight change was about 5%.

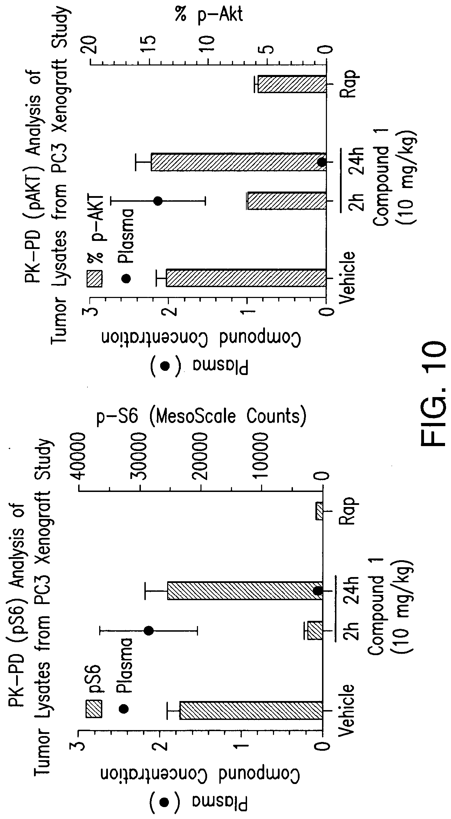

At the end of the first dosing cycle, plasma and tumor samples were collected at 2 and 24 hours after the last dose in the 10 mg/kg group and analyzed for compound levels and PD markers. Approximately 2 µM of Compound 1 was detected at 2 hours post-last dose, whereas by 24 hours, the compound amount was negligible in the plasma. Concurrently, both PD markers, pS6 and pAkt, were inhibited at 2 hours but increased to control levels by 24 hours (Figure 10).

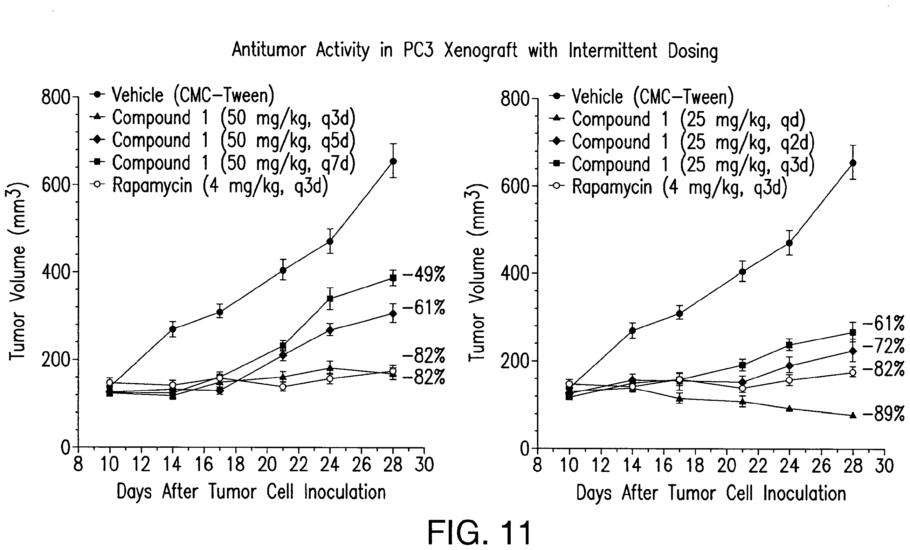

Another study was designed to determine the antitumor activity of Compound 1 in the PC3 xenograft model with intermittent dosing (*i.e.,* Q2D, Q3D, Q5D and Q7D) at 25 and 50 mg/kg dose levels (Figure 11). Dosing was initiated on Day 10 when the average tumor volumes ranged between 116 and 146 mm³. By the end of the 3-week dosing period on Day 31, the vehicle-treated tumors reached an average volume of 813 ± 60 mm³. The positive control rapamycin significantly inhibited tumors (p < 0.001) on day 31. The % tumor inhibition expressed in Figure 11 for each treatment group represents the tumor volume reduction on day 31 when compared with vehicle control. At the 50 mg/kg dose level, the greatest efficacy (82% inhibition) was achieved on the Q3D schedule, whereas less frequent dosing on the Q5D and Q7D schedules produced less robust (61% and 49%, respectively) but statistically significant (p < 0.001) tumor inhibition when compared to vehicle control. The 25 mg/kg dose level was tested on QD, Q2D, and Q3D dosing schedules. Tumor regression was observed with Compound 1 at 25 mg/kg QD. Significant (p < 0.001) tumor inhibition of 72% and 61% was observed with Q2D and Q3D dosing, respectively.

With the exception of the Compound 1 at 25 mg/kg QD group, no significant change in body weight was observed in any of the treatment groups. The animals in the 25 mg/kg QD dose group lost about 10% body weight during the study period (data not shown).

Following the last administration of Compound 1, plasma samples were collected at various time points and analyzed for compound. The calculated plasma AUC value for each dose group was:
25 mg/kg QD: 122 µM•hr (AUC0-24h)
25 mg/kg Q2D: 99 µM•hr (AUC0-48h)
25 mg/kg Q3D: 77 µM•hr (AUC0-72h)
50 mg/kg Q3D: 228 µM•hr (AUC0-72h)
50 mg/kg Q5D: 359 µM•hr (AUC0-120h)

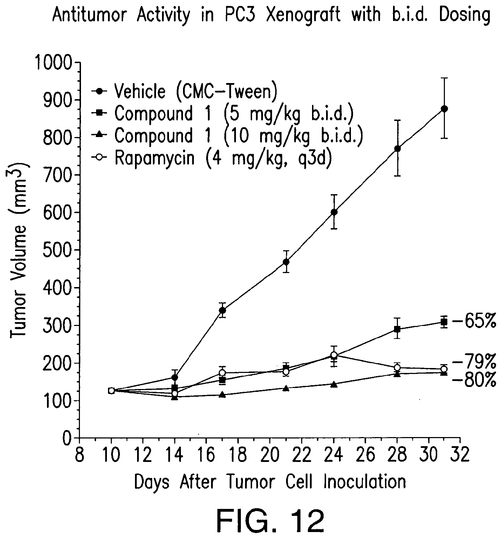

Another study was designed to test the antitumor activity of Compound 1 with BID dosing at 5 and 10 mg/kg in the PC3 xenograft model (Figure 12). Dosing was initiated on Day 10 when the average tumor volume ranged between 123 and 131 mm3. By the end of the 3-week dosing period on Day 31, vehicle-treated tumors reached an average volume of 875 ± 80 mm3. Significant (p < 0.001) inhibition of tumor growth was observed with rapamycin. Dose-dependent tumor inhibition was observed with Compound 1 treatment. The tumor volume reduction in Compound 1 treated groups at 5 and 10 mg/kg were 64.9% and 79.9%, respectively, when compared with vehicle control (Figure 12). The lowest efficacious dose as determined by 65% tumor volume inhibition was observed at the 5 mg/kg dose level of Compound 1.

No statistically significant change in body weight was observed in any of the groups in the study (data not shown).

Following the last dose of Compound 1, plasma samples were collected at various time points and analyzed for compound levels. Compound exposure was approximately dose-proportional. Total plasma AUC0-10 h values for the 5 and 10 mg/kg dose levels were 8.6 and 23.7 µM•hr, respectively, as shown below in Table 6. The calculated plasma free fraction AUC0-10 h values for 5 and 10 mg/kg were 1.0 and 2.8 µM•hr, respectively, (Compound 1 mouse plasma protein binding is 88%).

**TABLE 6**

| **Time (hr) 5 mg/kg** | **Plasma Concentration (µM)** |
|---|---|
| 1 | 2.91 ± 0.68 |
| 2 | 1.29 ± 0.28 |
| 4 | 0.57 ± 0.17 |
| 6 | 0.81 ± 0.51 |
| 8 | 0.41 ± 0.28 |
| 10 | 0.21 ± 0.13 |
| 24 | 0.60 ± 0.13 |

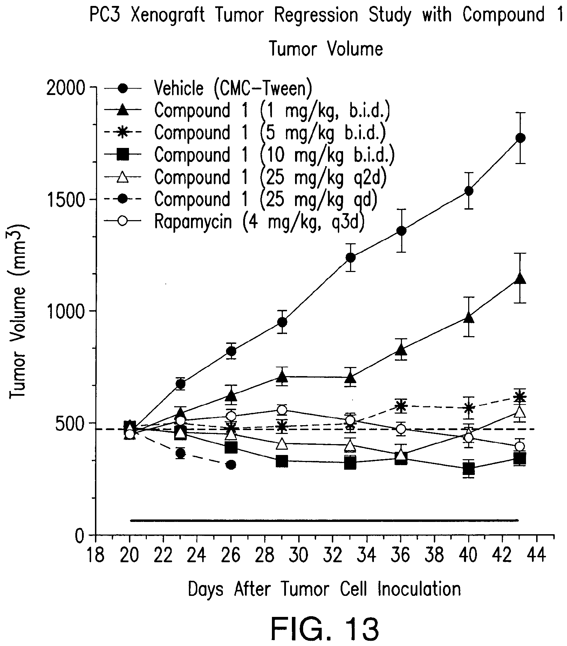

Another study was designed to test the antitumor activity of Compound 1 in large, established PC3 tumors to determine whether the compound causes tumor regression (Figure 13). Dosing was initiated on Day 20 when the average tumor volumes ranged between 450 and 486 mm3. By the end of the dosing period on Day 43, the vehicle-treated tumors reached an average volume of 1773 ± 113 mm³. The positive control rapamycin caused tumor growth stasis. The tumor volumes of the rapamycin-treated group were significantly (p < 0.001) smaller than the vehicle control group.

Compound 1 was tested at 3 doses levels (1, 5, and 10 mg/kg) BID. The % tumor inhibition expressed in Figure 13 for each treatment group represents the tumor volume reduction on day 43 when compared with vehicle control. At 10 mg/kg BID, the tumor inhibition was better than the positive control. At this dose level, Compound 1 caused the regression of PC3 tumors. The average tumor volume by the end of the dosing period on Day 43 was significantly (p < 0.01) smaller than the average starting volume on Day 20 at the beginning of dosing (343.6 ± 32.6 mm³ on day 43 vs. 482.2 ± 12.6 mm³ on day 20). Compound 1 at 5 mg/kg BID caused stasis of tumor growth, whereas 1 mg/kg BID significantly (p < 0.01) slowed tumor growth when compared with vehicle-treated animals. Compound 1 at 25 mg/kg Q2D caused regression of tumors initially during the first 2 weeks of dosing. By the end of the dosing period on Day 43, the tumor volumes were similar to the starting volumes on Day 20 (549.4 ± 45.7 mm³ on day 43 vs. 475.1 ± 19.6 mm³ on day 20). The animals in the Compound 1 25 mg/kg QD group were euthanized on Day 26 due to excessive weight loss (Figure 13).

With the exception of the Compound 1 25 mg/kg QD dose group, no statistically significant change in body weight was observed in any of the groups in the study (Figure 9). The animals in 25 mg/kg QD group lost over 19% of their initial body weight by Day 26 and were euthanized.

Following the last dose of Compound 1, plasma samples were collected at various time points and analyzed for compound levels. Approximately dose-proportional compound exposure was observed in the plasma between the 1 and 5 mg/kg dose groups but a greater than dose proportional increase was observed with 10 mg/kg treatment. The calculated total plasma AUC0-10 hr value with BID dosing for the 1, 5, and 10 mg/kg groups was 1.5 µM•hr, 8.7 µM•hr, and 26 µM•hr, respectively. The calculated free fraction AUC0-10 hr was 0.18 µM•hr, 1.0 µM•hr, and 3.1 µM•hr, respectively (Compound 1 mouse plasma protein binding is 88%). The calculated total plasma AUCO-48 hr value for the 25 mg/kg Q2D dose group was 112 µM•hr. The calculated plasma free fraction AUC0-48 hr value for the 25 mg/kg Q2D dose group was 13.4 µM•hr.

*Results* - *Pharmacokinetics*/*Pharmacodynamics of Compound 1 in PC3 Tumor-bearing Mice.* The PK/PD relationship between Compound 1 exposure in plasma and tumor and the inhibition of tumor mTOR pathway-related PD markers was determined after administration of a single dose of compound. pS6 (a PD marker for mTORC1) and pAkt (a PD marker for mTORC2) levels were measured in the tumor and correlated with compound exposure in the plasma and/or tumor. The PK/PD relationship was studied at various dose levels (0.3, 1, 10, 25, 30, 50 and 100 mg/kg). At 30 and 100 mg/kg, plasma and tumor samples were collected at 4, 8, and 24 hours post-dose. There was a dose and time dependent relationship between plasma and tumor compound levels and pS6 and pAkt levels in the tumor (data not shown). At both dose levels, pS6 and pAkt were significantly (p < 0.001) inhibited through 24 hours (pS6: 78% and 91% inhibition at 30 and 100 mg/kg, respectively; pAkt: 78% and 86% inhibition at 30 and 100 mg/kg, respectively). At both dose levels > 1µM Compound 1 was detected in plasma 24 hours after the dose (1.15±0.62 and 2.55 ±0.60 µM, for 30 and 100 mg/kg, respectively).

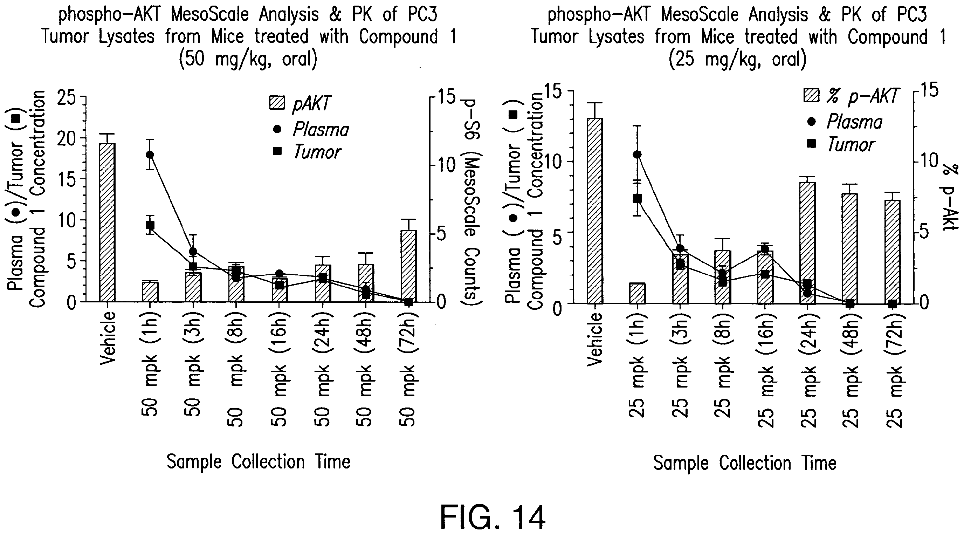

In another study, time points were extended further to 72 hours following the administration of Compound 1 at 25 and 50 mg/kg. The compound concentration in tumors mirrored the plasma concentrations at most time points. A dose and time dependent PK/PD relationship was observed at 25 (Figure 14 and Table 7) and 50 mg/kg (data not shown). At 25 mg/kg, >80% inhibition of pS6 levels persisted up to 24 hours, whereas >60% inhibition of pAkt was observed up to 16 hours. By 48 and 72 hours when the compound concentration in plasma and tumors was significantly lower (< 0.03 µM in both plasma and tumor at 48 and 72 hours), the PD marker inhibition was diminished.

**TABLE 7**

| | | **PD Marker** | **Inhibition (%)** |
|---|---|---|---|
| **Dose** | **Sample Collection time (h)** | **pS6** | **pAkt** |
| 25 mg/kg | 1 | 87.9 | 89.1 |
| | 3 | 95.1 | 73.6 |
| | 8 | 92.3 | 71.6 |
| | 16 | 92.1 | 71.7 |
| | 24 | 83.7 | 34.5 |
| | 48 | 47.2 | 40.5 |
| | 72 | 68.3 | 44.1 |
| 10 mg/kg | 1 | 79.5 | 88.6 |
| | 3 | 91.9 | 79.2 |
| | | PD Marker | Inhibition (%) |
| Dose | Sample Collection time (h) | pS6 | pAkt |
| 10 mg/kg | 8 | 91.2 | 64.1 |
| | 16 | 32.4 | 41.7 |
| | 24 | 32.7 | 46.1 |
| | 48 | 39.5 | 41.4 |
| | 72 | 47.9 | 38.6 |
| 1 mg/kg | 0.5 | 80.8 | 72.1 |
| | 2 | 82.8 | 66.7 |
| | 4 | 78.6 | 66.5 |
| | 6 | 53.1 | 39.2 |
| | 10 | 14.4 | 52.3 |
| | 24 | 20.6 | 55.9 |

| | | | |
|---|---|---|---|
| Key: BLQ = Below quantitation limits. | | | |

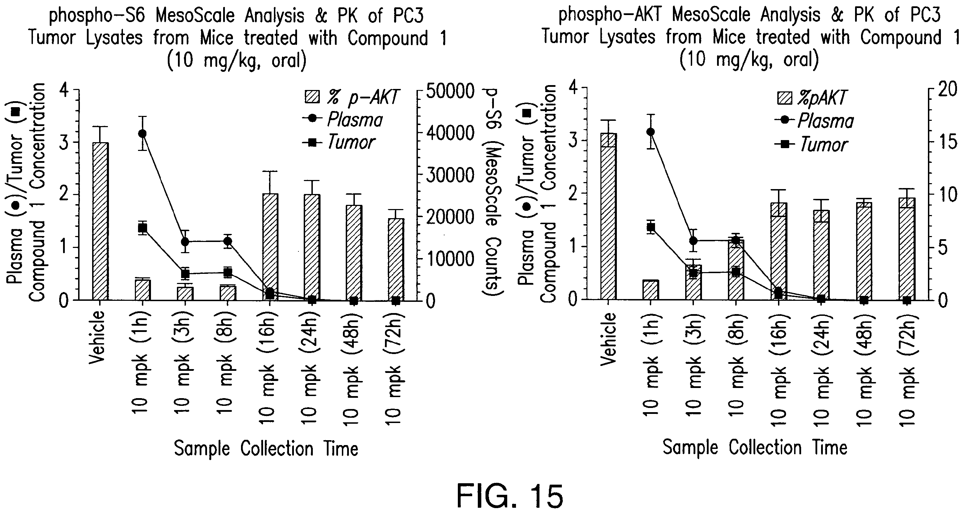

The PK/PD relationship was further determined at 10 mg/kg (Figure 15; Table 6). Significant (p < 0.001) inhibition of pS6 (>80%) and pAkt (> 60%) was observed up to 8 hours following administration of Compound 1 at plasma concentrations greater than 1 µM. Beyond 8 hours when the compound concentrations in the plasma and tumors were lower (0.17 µM plasma concentration), the PD marker inhibition was diminished but still significantly (P<0.01) lower than vehicle controls.

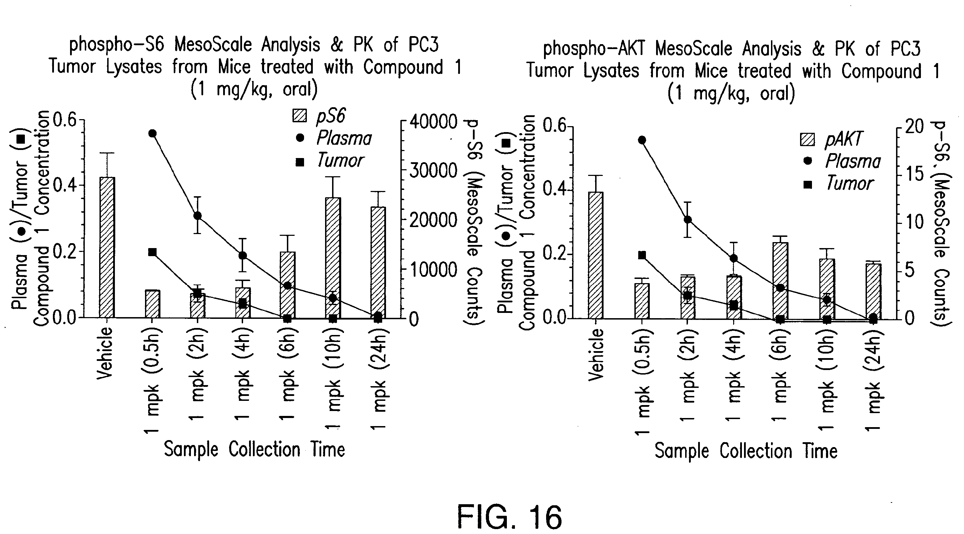

The PK/PD relationship was examined at low doses of 1 mg/kg (Figure 16) and 0.3 mg/kg (Table 8). Approximately 80% pS6 inhibition was observed up to 2 hours (data not shown) and 4 hours at 0.3 mg/kg and 1 mg/kg, respectively. Significant (p<0.01) pAkt inhibition was observed only at 1 mg/kg. The plasma concentration associated with >80% inhibition of pS6 and >60% inhibition of pAkt was 0.19 ± 0.1 µM at 1 mg/kg dose level. At 0.3 mg/kg dose level, the maximum Compound 1 plasma concentration was 0.23 ± 0.03 µM at 0.5 h time point. At this timepoint, 81% inhibition of pS6 but no inhibition of pAkt was observed (data not shown).

Data from Compound 1 PK/PD studies indicates that plasma concentrations >0.2 µM are needed to inhibit >80% pS6 and >60% pAkt. Plasma concentrations at the 5 mg/kg BID dose level (the minimal efficacious dose) were maintained above 0.2 µM for 8 hours and this lead to 65% tumor volume reduction in an efficacy study and tumor stasis in a regression study. Based on the PK/PD relationship of Compound 1 plasma concentration and PD biomarker inhibition as well as the relationship of Compound 1 plasma concentration and tumor volume reduction data in PC3 xenografts, maintenance of this level of exposure and degree of biomarker inhibition through 8 hours twice daily confers good antitumor efficacy in PC-3 tumors.

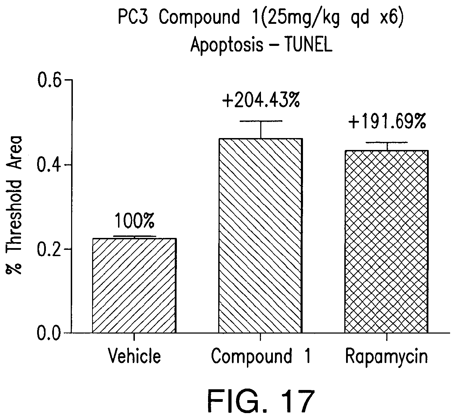

*Results* - *Mechanism of Action Studies in PC3 Tumors.* To determine if Compound 1 induced apoptosis in PC3 xenografts, tumors from vehicle (oral, QD for 6 days), Compound 1, (25 mg/kg, oral, QD for 6 days) and rapamycin-treated (4 mg/kg, IP, Q3D for 6 days) animals were harvested 2 hours after the last dose on Day 6 and processed for TUNEL which labels apoptotic cells. In this assay, terminal deoxynucleotidyl transferase (TdT) incorporates the FITC labeled nucleotides to the ends of DNA strand breaks in situ. The FITC labeled nucleotides (representing the cells with DNA strand breaks, a hallmark of apoptosis) can be detected using a microscope equipped with fluorescence attachment. Relatively few (0.2%) TUNEL-positive cells were observed in vehicle-treated PC3 tumors (Figure 17). The number of TUNEL-positive cells in the tumors treated with Compound 1 and rapamycin were comparable (Figure 17). There was about a two fold increase in TUNEL-positive cells observed in Compound 1 and rapamycin-treated tumors compared with the vehicle control. These data suggest that apoptosis appears to have some contribution to the observed antitumor activity of Compound 1 in vivo.

Tumors from animals treated with vehicle (oral, QD for 6 days), Compound 1 (25 mg/kg, oral, QD for 6 days) or rapamycin (4 mg/kg, IP, Q3D for 6 days) were harvested 2 hours after the last dose on Day 6 and processed for immunohistochemistry. Immunohistochemistry with anti-Ki-67 antibody was utilized to determine if Compound 1 inhibits tumor growth by blocking the proliferation of tumor cells in vivo. Ki-67 is a nuclear antigen expressed in cells. A strong correlation between the fraction of proliferating cells in S phase and the Ki67 index has been demonstrated. Tumor sections were co stained with anti-CD-31 antibody to determine the antiangiogenic activity of the compound. CD-31 (also called PECAM-1) antibody recognizes a CD-31 molecule expressed on the endothelial cell membranes and is involved in their adhesive interactions. Nuclei were counter-stained with Hoechst dye. The proliferating cells and microvessels were quantitated using Metamorph software and expressed as a percentage of the threshold area.

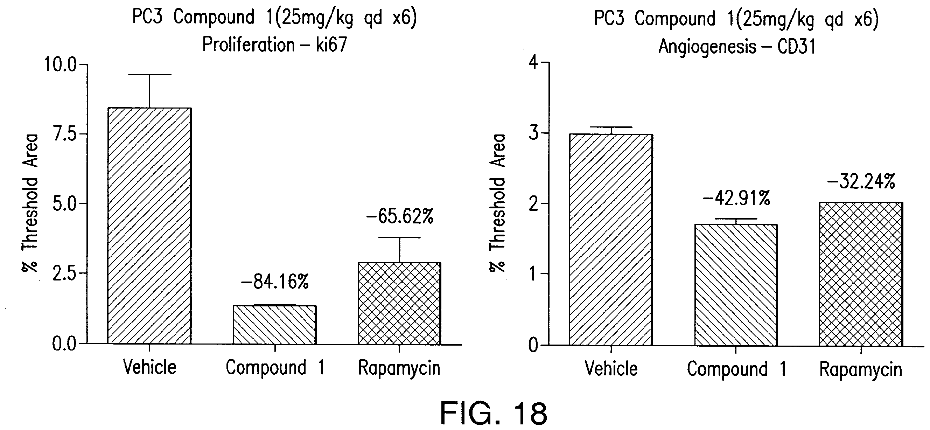

In vehicle-treated PC3 tumors there were a significant number of cells proliferating (about 8.5%, expressed as the Ki-67 positive threshold area) (Figure 18). There was an 84% reduction (p < 0.001) in the number of proliferating cells in the Compound 1-treated tumors compared with vehicle-treated tumors. About 3% of the threshold area comprised CD-31-positive vessels in the vehicle control PC3 tumor sections as determined by CD-31 immunohistochemistry. There were significantly (p < 0.01) fewer CD-31 positive blood vessels in the Compound 1-treated PC3 tumors compared with vehicle-treated tumors (Figure 18). These data suggest antiproliferative activity, apoptotic activity and anti-angiogenic activity of Compound 1 are the potential mechanisms underlying the antitumor activity of Compound 1. *Conclusions.* These studies demonstrated the following:
Treatment with mTOR kinase inhibitor Compound 1 significantly inhibited PC3 prostate tumor growth in a dose and schedule dependent manner.

The minimal efficacious dose required to inhibit PC3 tumor growth was 5 mg/kg BID. The total plasma AUC(0-10h) at the minimal efficacious dose was 8.6 µM•hr. The AUC(0-10h) of free faction in the plasma at the minimal efficacious dose was 1.0 µM•hr.

Significant inhibition of mTOR pathway markers pS6 and pAkt in Compound 1-treated PC3 tumors suggests that the observed antitumor activity of Compound 1 was mediated through the inhibition of both the mTORC1 and mTORC2 complexes of the mTOR pathway.

In PC-3 tumors the PK-PD relationship indicates that >80% inhibition of pS6 and >60% inhibition of pAKT was obtained for total drug plasma concentrations greater than 0.2 µM. Maintenance of plasma levels > 0.2 µM and this degree of biomarker inhibition through 8 hours twice daily confers good antitumor efficacy in PC-3 tumors.

Immunohistochemical data demonstrate that the observed antitumor activity of Compound 1 was not only due to the inhibition of tumor cell proliferation but also due to the increased apoptotic and antiangiogenic activities of Compound 1 in vivo.

### ANTI-TUMOR ACTIVITY OF COMPOUND 2 IN THE PC3 HUMAN PROSTATE CANCER XENOGRAFT MODEL

Suspensions of Compound 2 were prepared in aqueous 0.5% CMC and 0.25% Tween-80. Vehicle and the test article were dosed at a volume of 5 mL/kg. The positive control rapamycin was dosed at a volume of 10 mL/kg.

*Efficacy and Mechanism of Action Studies.* Groups of female SCID mice bearing PC3 tumors (n = 8 to 10/group) were dosed orally with vehicle or Compound 2 (doses ranged between 0.25 and 5 mg/kg) throughout the study, starting when tumor volumes reached approximately 150 mm³. The twice-daily (BID) dose groups were dosed with a 10-hour separation between morning and evening doses. In the positive control group, rapamycin was administered Q3D (every third day) via the intraperitoneal (IP) route. At the end of each study, plasma and/or tumor samples were collected.

The design of the experiments is shown in (Table 8) below.

**TABLE 8**

| **Dose Group (n)** | **Dosing Schedule** | **Dosing Duration** | **Sample Collection Time Points on the Last Day of Dosing** |
|---|---|---|---|
| Vehicle (n = 10) | BID | 3 weeks | 24 h |
| Rapamycin 4 mg/kg (n = 5) | Q3D | 3 weeks | 24 h |
| Compound 2 0.25 mg/kg (n = 10) | BID | 3 weeks | 1, 3, 6, 10, and 24 h |
| Compound 2 0.5 mg/kg (n = 10) | BID | 3 weeks | 1, 3, 6, 10, and 24 h |
| Compound 2 1 mg/kg (n = 10) | BID | 3 weeks | 1, 3, 6, 10, and 24 h |
| Compound 2 1 mg/kg (n =10) | QD | 3 weeks | 1, 3, 6, 10, and 24 h |
| Vehicle (n = 9) | QD | 3 weeks | 24 h |
| Rapamycin 4 mg/kg (n = 5) | Q3D | 3 weeks | 24 h |
| Compound 2 0.25 mg/kg (n = 9) | QD | 3 weeks | 1, 3, 6, 10, and 24 h |
| Compound 2 1 mg/kg (n = 9) | QD | 3 weeks | 1, 3, 6, 10, and 24 h |
| Compound 2 2 mg/kg (n = 9) | QD | 3 weeks | 1, 3, 6, 10, and 24 h |
| Compound 2 5 mg/kg (n = 9) | QD | 3 weeks | 1, 3, 6, 10, and 24 h |

*Pharmacokinetic*/*Pharmacodynamic Studies.* Groups of female SCID mice bearing PC3 tumors (n = 4 per group) were dosed orally with a single dose of vehicle or Compound 2. The plasma and tumor samples were collected at various time points following compound administration as described.

Plasma and tumor collection time points were each terminal time points for PK/PD studies.

Compound 2 (1 and 10 mg/kg) at 1, 3, 6, 10, and 24 hours; Vehicle control at 24 hours.

Compound 2(0.1 and 0.3 mg/kg) at 0.5, 1, 3, 6, 10, and 24 hours; Vehicle control at 24 hours.

*Kinetics of DNA-PK Inhibition.* Groups of PC3 tumor-bearing SCID mice (n = 4 per group) were dosed orally with 5 mg/kg Compound 2 or vehicle daily for 6 days (QDx6). The tumors were collected at 3, 6, 10, 24, 36, and 48 h after the last dose and processed for immunohistochemistry.

*Experimental Procedures.* PC3 cell line was obtained from American Tissue Culture Collection (ATCC) (Gaithersburg, MD) and grown in growth medium containing Ham's F12K medium with 2 mM L-glutamine adjusted to contain 1.5 g/L sodium bicarbonate and 10% fetal bovine serum. The cells were detached from tissue culture flasks using trypsin-EDTA. After centrifugation, the cell pellets were suspended in PBS and cells counted using a hemocytometer. Matrigel was added to the cell suspension to adjust the final volume to 2 × 106 cells/0.1 mL of 1:1 mixture of Matrigel: PBS.

Mice were anesthetized with inhaled isoflurane and then inoculated with PC3 tumor cells subcutaneously above the right hind leg with 0.1 mL of a single cell suspension in Matrigel using a sterile 1 mL syringe fitted with a 26-gauge needle. Following inoculation, the mice were returned to microisolator cages.

Following inoculation of animals, tumors were allowed to grow to approximately 150 mm³ prior to randomization of mice. The typical number of days required for tumors to reach 150 mm³ was 8 to 9 days. The tumor of each animal was measured and animals with tumors ranging between 130 and 155 mm³ were included in the study. Animals from the pool were then distributed randomly into various cages and the cages were randomly assigned to vehicle, positive control, or test article groups. All of the mice were tagged with metal ear tags on the right ear. A typical group consisted of 9 to 10 animals.

Compound 2 was formulated at 5 mg/kg in 0.5% CMC and 0.25% Tween 80 in water (as a suspension). The formulations were homogenized using a Teflon™ pestle and mortar (Potter-Elvehjem tissue grinder). Dilutions to accommodate lower doses were made directly from the 5 mg/kg (1 mg/mL) stock. Between the doses, the formulated compound was stored in the dark under constant stirring using a magnetic stirrer at 4 °C. Compound was formulated every 3 days. Test article and vehicle were administered by oral gavage. The positive control (rapamycin) was prepared as a solution in 2% ethanol, 45% polyethyleneglycol 400, and 53% saline and administered by IP injection. Sterile syringes and gavage needles were used for compound administration. All the procedures including injections were performed in biosafety cabinets sprayed with 70% ethanol prior to use.

Tumor volumes were determined prior to the initiation of treatment and were considered as the starting volumes. Thereafter, tumors were measured twice a week for the duration of the study. The long and short axes of each tumor were measured using a digital caliper in millimeters. Tumor volumes were calculated using the formula: width² x length / 2. The tumor volumes were expressed in cubic millimeters (mm3).

Initial body weights were recorded prior to the initiation of treatment using a digital scale. The percentage body weight change during the course of the study was calculated using initial body weight measurements. Body weights of each animal were measured twice a week at the same time as the tumor measurements. Body weights were measured more frequently if significant decreases were noted during the course of the study.

After the last measurement, the experiment was terminated by taking plasma and tumor samples for PK and PD measurements. Compound concentrations in plasma were determined by LC-MS/MS technology. PD markers, pS6RP and pAKT (S473), were measured using MSD technology.

Following the last dose of test article, blood samples were collected via retro-orbital bleeds at predetermined time points over 24 hours. The blood samples were processed to plasma using plasma separation tubes containing heparin.

After the last bleed, animals were euthanized via CO₂ asphyxiation and the tumors were dissected out. A small tumor piece (about 100 mg) was placed in a pre-weighed vial and snap frozen in liquid nitrogen for compound determination. The remaining tissues were either snap frozen in liquid nitrogen and/or fixed in buffered formalin for PD measurements.

Female SCID mice bearing PC3 tumor ranging between 300 and 500 mm³ were pooled together and randomly distributed to vehicle or Compound 2 treatment groups. At predetermined time points following dosing, mice were euthanized and plasma and tumor samples were collected for PK/PD determination. Compound concentrations in plasma and tumor were determined by LC-MS/MS. PD markers, pS6RP and pAKT (S473), were measured using MSD technology. Modulation of total DNA-PKcs and phospho-DNA-PK (pDNA-PK (S2056)) levels were measured using IHC.

*Meso Scale Discovery Assay.* Tissue samples were homogenized in lysis buffer (0.5 g/mL) containing para-nitrophenyl phosphate (pNPP), sodium metavanadate (NaVO₃), dithithreitol (DTT), and protease and phosphatase inhibitors, and centrifuged for 10 minutes at 1200 rpm at 4 °C. Supernatants were sonicated for 1 minute at 4 °C, divided into aliquots, and stored at -80 °C.

Protein concentrations in samples were determined using BCA Protein Assay Reagent Kit.

MSD electrochemiluminescent immunoassay kits and protocol were used for measuring the pS6RP and pAKT (S473)/Total AKT. MSD 96-well plates spotted with antibodies to pS6RP (S235/236), pAKT (S473) and Total AKT were blocked with blocking solution for 5-60 minutes followed by a wash with wash buffer. Then 25 µg of tumor lysates were added to each well and incubated for 2 hours followed by a washing 4 times with wash buffer. Detection antibody was added to each well and incubated in the dark for 1 hour at 4°C. The places were then washed and bound antigen was read using a MSD SECTOR™ instrument.

*Mechanism of Action Studies.* To determine potential mechanism of action of Compound 2, mice bearing PC3 tumors were dosed orally with vehicle or Compound 2 at 5 mg/kg QD for 6 days. The positive control, rapamycin, was dosed IP at 4 mg/kg on days 1, 4, and 6. Two hours after the last dose of vehicle, Compound 2, or rapamycin, the mice were euthanized and the tumors were dissected. The tumors were snap frozen in liquid nitrogen and processed for immunohistochemistry (IHC) and TUNEL.

*Immunohistochemistry* - Five to 10 micron (µm) thick cryostat sections were used for IHC. Modulation of total DNA-PKcs and phospho-DNA-PK (pDNA-PK (S2056)) levels were evaluated using respective antibodies specific for the total protein or the phosphorylation site at S2056. Cell proliferation marker Ki67 was evaluated by IHC using anti-Ki67 antibody. Anti-CD31 antibody was used to determine blood vessel density, which is a measurement of tumor angiogenesis. Frozen sections were fixed in 4% paraformaldehyde for 10 minutes at room temperature, washed in PBS, blocked, and permeabilized with 5% normal goat serum and 0.3% Triton X 100. Sections were then incubated with primary antibody (anti-DNA-PK and anti-pDNA-PK (S2056) [1 µg/mL for both] for 2 hours and overnight for anti-Ki67 [0.8 µg/mL] and anti-CD31 [15.6 µg/mL]) followed by incubation with 1:500 dilution of secondary antibodies (60 minutes). The sections were washed, counterstained with Hoechst dye (0.4 µg/ml), and mounted with antifade reagent. A double labeling method was used to detect DNA-PKcs and pDNA-PK (S2056) or Ki67 and CD31 in the same tissue section. For double labeling methods, cocktails of primary antibodies followed by a cocktail of secondary antibodies were used for incubation. Positive and negative controls were included in each assay. Positive controls included the sections that were known to be reactive with the antibody. Negative controls included omission of primary or secondary antibodies. The following specificity controls were performed for pDNA-PK (S2056) antibodies:
- Omission of primary or secondary antibody
- Blocking peptide control: pDNA-PK (S2056) primary antibody was preincubated with 50-fold excess of blocking peptide prior to addition to tissue sections
- Phosphatase digestion: To determine the specificity of pDNA-PK (S2056) phospho-antibody, tissue sections were incubated with 1000 units of λ protein phosphatase (to digest the phosphate groups on the proteins in the tissue section) prior to addition of primary antibody

The sections were visualized with a Nikon E800 microscope equipped with fluorescence detection equipment and a digital camera attached to a computer.

*Apoptosis TUNEL Assay* - To detect the apoptotic cells, fluorescence *in situ* cell death detection kit (Roche Biosciences) was used. Five to 10 µm thick cryostat sections were fixed in 4% paraformaldehyde for 15 minutes at room temperature, washed, permeabilized with 0.3% Triton X-100 and 0.1% sodium citrate in PBS for 10 minutes. Sections were then washed in PBS and incubated with a labeling solution containing TdT enzyme for 1 hour at 37 °C in the dark. The sections were washed in PBS, counterstained with Hoechst dye (0.4 µg/mL) at room temperature for 10 minutes, and mounted in Prolong Gold antifade reagent.

*Quantitation of Immunohistochemistry* - The tissue sections processed for apoptosis or immunostained for DNA-PK, proliferating cells (Ki67), or blood vessels were quantitated using Metamorph software. Using the 20X objective, 5 different fields from each section, 1 to 2 sections from each tumor, and 3 to 4 tumors from each treatment group or control group were used for quantitation. The area of interest was expressed as the percentage threshold area of the total area. The data from each individual animal were pooled together and the mean ± SEM for each group was calculated. For the DNA-PK kinetic inhibition study, the expression of pDNA-PK (S2056) was normalized to DNA-PKcs.

### Results

### Antitumor Activity, Body Weight Change, and Pharmacokinetics with Twice Daily and Once Daily Dosing.

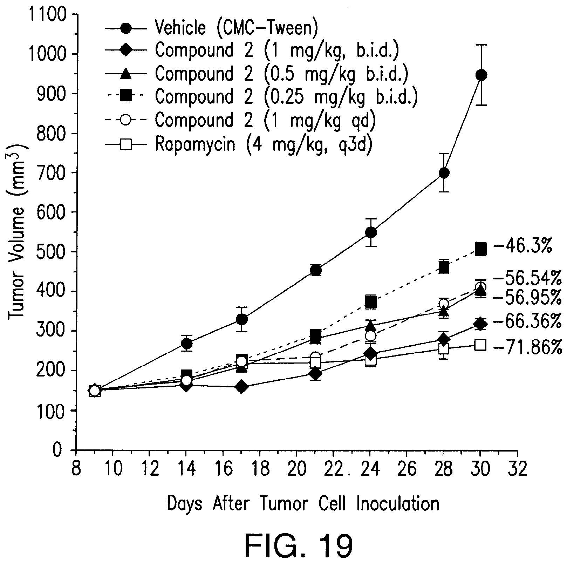

*Anti-tumor Acitivity* - The antitumor activity of Compound 2 was tested with BID (0.25, 0.5, and 1 mg/kg) and QD (1 mg/kg) dosing (Figure 19). Dosing started on Day 9 when tumor volumes ranged between 140 and 155 mm³ and continued until Day 30. By Day 30, the vehicle-treated group measured 947.6 ± 75.4 mm³. All animals in the positive control group that received rapamycin (4 mg/kg, Q3D) had significantly (p < 0.001) smaller tumors when compared with the vehicle group on Day 30. Tumor inhibition is shown as a percentage in Figure 1 for each treatment group and represents the difference in average tumor volume between Compound 2-treated mice and vehicle-treated mice on Day 30. Dose-dependent tumor inhibition was achieved with Compound 2 with BID dosing (46%, 57%, and 66% tumor volume reduction at 0.25, 0.5, and 1 mg/kg BID, respectively). The average tumor volumes of all Compound 2-treated groups were significantly smaller (p < 0.001) than in vehicle-treated control mice on Day 30. The lowest efficacious dose as determined by approximately 65% tumor volume inhibition was the 1 mg/kg BID dose level. In this study, Compound 2 was also tested with QD dosing at 1 mg/kg. The average tumor volumes of 1 mg/kg QD dosed groups were significantly smaller (p < 0.001) than in the vehicle-treated control mice on Day 30. The tumor inhibition observed with Compound 2 dosed at 1 mg/kg QD was the same as 0.5 mg/kg BID.

*Pharmacokinetics* - To determine plasma Compound 2 concentrations at the efficacious dose, plasma samples were collected at 1, 3, 6, 10, and 24 hours after the last dose and were analyzed (Table 9). Approximately dose-proportional compound exposure was observed. The calculated total plasma AUC0-10 h values for each BID dose group were 0.60, 1.42, and 3.27 µM•hr for 0.25, 0.5, and 1 mg/kg, respectively. The calculated total plasma AUC0-24 h value for 1 mg/kg QD was 2.95 µM•hr. The calculated plasma AUC0-10 h values of unbound fractions for each BID dose group were 0.2, 0.48, and 1.1 µM•hr for 0.25, 0.5, and 1 mg/kg, respectively (Compound 2 mouse plasma protein binding is 66% (PD1604)). The calculated plasma AUC0-24 h value of unbound fraction at 1 mg/kg QD was 1.0 µM•hr.

### Antitumor Activity, Body Weight Change, and Pharmacokinetics with Once Daily Dosing

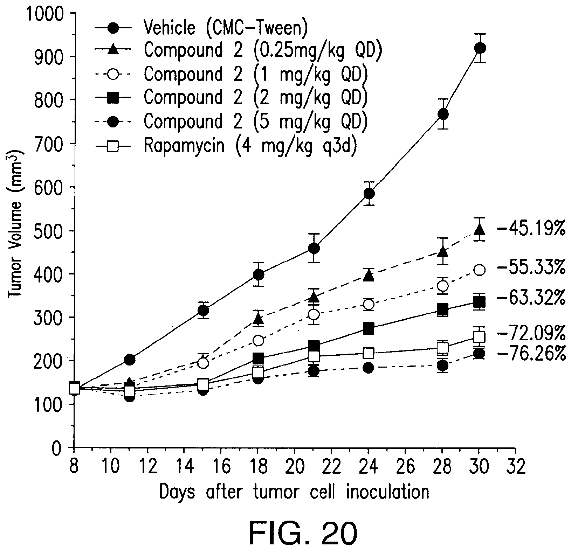

*Anti-tumor Activity* - The study was designed to determine the dose response antitumor activity of Compound 2 with QD dosing in the PC3 tumor xenograft model. Dosing was initiated on Day 8 when average tumor volumes ranged between 130 mm³ and 140 mm3. By the end of the 3-week dosing period on Day 30, vehicle-treated tumors reached an average volume of 919.7 ± 32.4 mm³. The positive control rapamycin significantly inhibited tumors (p < 0.001) on Day 30. Dose-dependent tumor inhibition was achieved with Compound 2 (45%, 55%, 63%, and 76% tumor volume reduction at 0.25, 1, 2, and 5 mg/kg, respectively; Figure 20). The average tumor volumes of all of Compound 2-treated groups were significantly smaller (p < 0.001) than in vehicle-treated control mice on Day 30. The lowest efficacious dose as determined by approximately 65% tumor volume inhibition was the 2 mg/kg QD dose level.

*Pharmacokinetics* - To determine plasma Compound 2 concentrations at the efficacious dose, plasma samples were collected at 1, 3, 6, 10, and 24 hours on the last day and were analyzed (Table 11). Approximately dose-proportional compound exposure was observed. The calculated total plasma AUC_{0-24 h} values for each dose group were 0.88, 2.33, 5.93, and 17.0 µM•hr for 0.25, 1, 2, and 5 mg/kg, respectively. The calculated plasma AUC_{0-24 h} values of unbound fractions for each dose group were 0.30, 0.79, 2.0, and 5.7 µM•hr for 0.25, 1, 2, and 5 mg/kg, respectively (Compound 2 mouse plasma protein binding is 66%).

**TABLE 9**

| **Dose & Schedule** | **Time (h)** | **Plasma Concentration (µM) Mean ± SEM** |
|---|---|---|
| 1 mg/kg BID | 1 | 0.73 ± 0.14 |
| 1 mg/kg BID | 3 | 0.48 ± 0.04 |
| 1 mg/kg BID | 6 | 0.22 ± 0.04 |
| 1 mg/kg BID | 10 | 0.11 ± 0.01 |
| 1 mg/kg BID | 24 | 0.02 ± 0.00 |
| 2 mg/kg QD | 1 | 1.02 ± 0.20 |
| 2 mg/kg QD | 3 | 0.59 ± 0.08 |
| 2 mg/kg QD | 6 | 0.27 ± 0.08 |
| 2 mg/kg QD | 10 | 0.21 ± 0.05 |
| 2 mg/kg QD | 24 | 0.02 ± 0.01 |

### Pharmacokinetics/Pharmacodynamics of Compound 2 in PC3 Tumor-bearing Mice

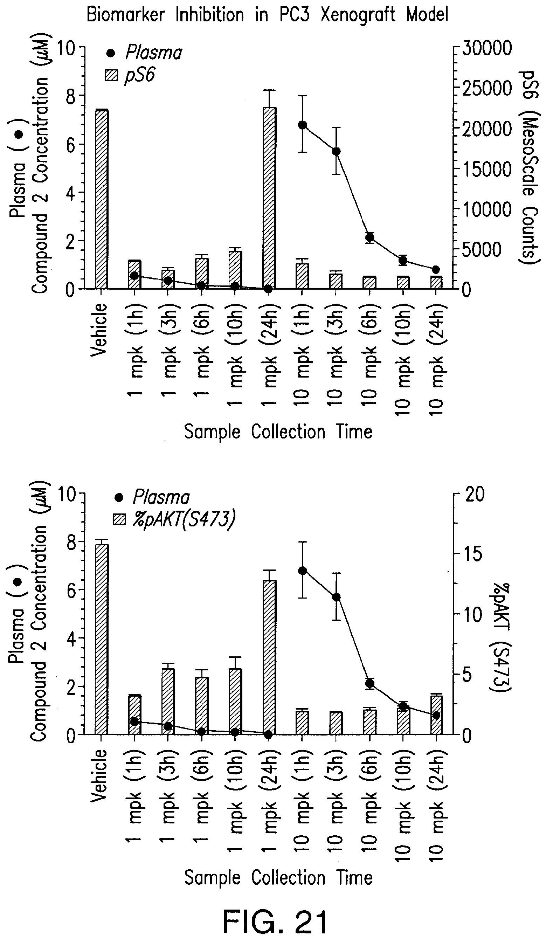

*mTOR Pathway-related PD Markers* - The PK/PD relationship between Compound 2 exposures in plasma and the inhibition of tumor mTOR pathway-related PD markers was determined after administration of a single dose of compound. The levels of pS6RP (a PD marker for mTORC1) and pAKT (S473) (a PD marker for mTORC2) were measured in the tumor and correlated with compound exposure in the plasma. The PK/PD relationship was studied at various dose levels (0.1, 0.3, 1, and 10 mg/kg). In the study, the PK/PD relationship was studied at 1 and 10 mg/kg. There was a dose- and time-dependent relationship between plasma Compound 2 exposure and pS6RP and pAKT (S473) levels in the tumor (Figure 21). At the 1 mg/kg dose level, pS6RP and pAKT (S473) were significantly (p < 0.001) inhibited through 10 hours (approximately 80% and 65% inhibition for pS6RP and pAKT (S473), respectively) at a plasma concentration of 0.12 ± 0.003 µM. At the 10 mg/kg dose level, ≥ 80% inhibition of both PD markers persisted through 24 hours (Figure 21; Table 10).

**TABLE 10**

| **Dose** | **Sample Collection Time (h)** | **Plasma Compound 2 Concentration (µM) Mean ± SEM** | **PD Marker Inhibition (%)** | |
|---|---|---|---|---|
| | | | **pS6RP** | **pAKT (S473)** |
| 10 mg/kg | 1 | 6.78 ± 1.16 | 85.3 | 87.6 |
| | 3 | 5.69 ± 0.96 | 91.3 | 88.3 |
| | 6 | 2.10 ± 0.22 | 93.5 | 86.9 |
| | 10 | 1.18 ± 0.20 | 93.7 | 86.0 |
| | 24 | 0.80 ± 0.09 | 93.2 | 79.8 |
| 1 mg/kg | 1 | 0.53 ± 0.02 | 84.6 | 79.6 |
| | 3 | 0.34 ± 0.01 | 89.5 | 65.1 |
| | 6 | 0.13 ± 0.01 | 82.8 | 69.9 |
| | 10 | 0.12 ± 0.00 | 78.7 | 65.5 |
| | 24 | 0.005 ± 0.00 | 0.0 | 19.0 |
| 0.3 mg/kg | 0.5 | 0.16 ± 0.03 | 69.8 | 77.2 |
| | 1 | 0.16 ± 0.01 | 79.6 | 70.7 |
| | 3 | 0.07 ± 0.01 | 73.5 | 52.9 |
| | 6 | 0.07 ± 0.01 | 67.3 | 42.2 |
| | 10 | 0.04 ± 0.01 | 57.9 | 23.0 |
| | 24 | 0.00 ± 0.00 | 33.3 | 9.6 |
| 0.1 mg/kg | 0.5 | 0.05 ± 0.00 | 45.7 | 50.0 |
| | 1 | 0.04 ± 0.00 | 38.2 | 25.1 |
| | 3 | 0.02 ± 0.00 | 27.2 | 15.3 |
| | 6 | 0.02 ± 0.00 | 23.3 | 14.2 |
| | 10 | 0.01 ± 0.00 | 17.6 | 2.5 |
| | 24 | BLQ | 00.0 | 0.0 |

In the study, the PK/PD relationship was examined at low doses of 0.3 and 0.1 mg/kg (Table 10). At 0.3 mg/kg, significant inhibition of pS6RP (p < 0.001) and pAKT (S473) (p < 0.05) was observed up to 10 hours and 6 hours, respectively; however, significant inhibition (p < 0.05) of both PD markers was observed only at the 0.5 hour time point for the 0.1 mg/kg dose level.

Data from Compound 2 PK/PD studies indicate that, in general, plasma concentrations > 0.12 µM are needed to inhibit ≥ 80% pS6RP and ≥ 65% pAKT (S473). At the 1 mg/kg BID dose level (the minimal efficacious dose), plasma concentrations above 0.12 µM were maintained for 6 hours twice daily and this led to 65% tumor volume reduction. With QD dosing, plasma concentrations > 0.12 µM were maintained through at least 10 hours (0.21 µM at 10 hours) at 2 mg/kg that led to approximately 65% tumor volume reduction. Based on the PK/PD relationship between Compound 2 plasma concentration and PD biomarker inhibition as well as the relationship between Compound 2 plasma concentration and tumor volume reduction data in PC3 xenografts, maintenance of this level of exposure (> 0.12 µM) and degree of biomarker inhibition through 10 hours with once daily dosing or at least 6 hours twice daily (BID dosing) confers good antitumor efficacy in PC-3 tumors.

*pDNA-PK (S2056)* - This study was designed to demonstrate the inhibition of pDNA-PK (S2056) in PC3 tumors treated with Compound 2. To determine the extent of pDNA-PK (S2056) inhibition in PC3 tumors, tumor-bearing mice were administered vehicle or Compound 2 at 5 mg/kg (QD for 6 days); tumors were harvested 2 hours after the last dose on Day 6 and processed for immuno-histochemistry to detect total (DNA-PKcs) and phospho (pDNA-PK (S2056)) proteins. In vehicle-treated PC3 tumors, there were numerous cells that exhibited a strong immunofluorescent signal for DNA-PKcs and pDNA-PK (S2056). There was a 98% reduction (p < 0.0001) in the threshold area that was positive for pDNA-PK (S2056) in the Compound 2-treated tumors compared with vehicle-treated tumors. There was no significant difference in the positive threshold area stained with an antibody to DNA-PKcs in vehicle- and Compound 2-treated tumors. These data suggest that Compound 2 inhibits pDNA-PK (S2056) in PC3 tumors.

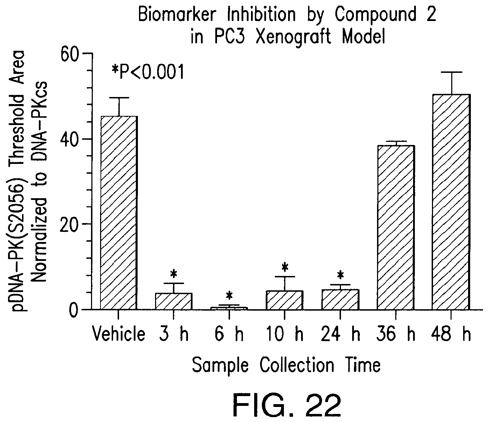

To determine the inhibition kinetics for pDNA-PK (S2056) following treatment with Compound 2 in PC3 tumors, tumor-bearing mice were dosed with vehicle or Compound 2 (5 mg/kg, QD) for 6 days and the tumors were collected at 3, 6, 10, 24, 36, and 48 hours after last dose and processed for immunohistochemistry to detect DNA-PKcs and pDNA-PK (S2056). The expression of pDNA-PK (S2056) was normalized to the expression of total DNA-PKcs (Figure 22). Significant (p < 0.001) pDNA-PK (S2056) inhibition (approximately 90%) was observed through 24 hours after the last dose of Compound 2. Thereafter, pDNA-PK (S2056) immunofluorescence gradually returned to control levels by 48 hours (Figure 22). As the plasma samples were not collected from this study, a direct PK/PD relationship between pDNA-PK (S2056) inhibition and plasma Compound 2 exposure could not be determined. However, plasma exposure data from the previous efficacy study dosed at 5 mg/kg showed that there was 0.13 µM of Compound 2 in the plasma at 24 hours after the last dose. These data together indicate that approximately 90% inhibition of pDNA-PK (S2056) could be achieved with plasma Compound 2 concentrations ≥ 0.13 µM.

### Mechanism of Action Studies

*Antiproliferative and Antiangiogenic Activity of Compound 2* - To determine the potential mechanism of action of Compound 2, tumors from animals treated with vehicle (oral, QD for 6 days) or Compound 2 (oral 5 mg/kg, QD for 6 days) were harvested 2 hours after the last dose on Day 6 and processed for immunohistochemistry. Immunohistochemistry with anti-Ki67 antibody was utilized to determine if Compound 2 inhibits tumor growth by blocking the proliferation of tumor cells in vivo. Ki67 is a nuclear antigen expressed in cells. A strong correlation between the fraction of proliferating cells in S phase and the Ki67 index has been demonstrated. Tumor sections were co stained with anti-CD31 antibody to determine the antiangiogenic activity of the compound. CD31 (also called PECAM-1) antibody recognizes a CD31 molecule expressed on the endothelial cell membranes and is involved in their adhesive interactions. Nuclei were counterstained with Hoechst dye. The proliferating cells and microvessels were quantitated using Metamorph software and expressed as a percentage of the threshold area.

In vehicle-treated PC3 tumors, approximately 35% of the threshold area was positive for Ki67. There was a 93% reduction (p < 0.001) in the number of proliferating cells in the Compound 2-treated tumors compared with vehicle-treated tumors. Over 3% of the threshold area comprised CD31-positive vessels in the vehicle control PC3 tumor sections as determined by CD31 immunohistochemistry. There were significantly (50% reduction, p < 0.05) fewer CD31 positive blood vessels in the Compound 2-treated PC3 tumors compared with vehicle-treated tumors. These data suggest that antiproliferative activity and antiangiogenic activity of Compound 2 are the potential mechanisms underlying the antitumor activity of Compound 2.

*Apoptotic Activity of Compound 2* - To determine if Compound 2 induced apoptosis in PC3 xenografts, tumors from vehicle-treated (oral, QD for 6 days) and Compound 2-treated (5 mg/kg, oral, QD for 6 days) animals were harvested 2 hours after the last dose on Day 6 and processed for TUNEL, which labels apoptotic cells. In this assay, terminal deoxynucleotidyl transferase (TdT) incorporates the FITC labeled nucleotides to the ends of DNA strand breaks in situ (Gavrieli, 1992). The FITC labeled nucleotides (representing the cells with DNA strand breaks, a hallmark of apoptosis) can be detected using a microscope equipped with fluorescence attachment. Relatively few (0.2%) TUNEL-positive cells were observed in vehicle-treated PC3 tumors. No significant change in the number of TUNEL-positive cells was observed between vehicle- and Compound 2-treated tumors at 5 mg/kg dose level (data not shown).

*Conclusions.* These studies demonstrated the following:
Treatment with Compound 2, a dual DNA-PKi/TORKi, significantly inhibited PC3 prostate tumor growth in a dose-dependent manner.

The minimal efficacious dose required to inhibit PC3 tumor growth was 2 mg/kg QD. The total plasma AUC0-24 h at the minimal efficacious dose was 5.93 µM•hr. The AUC0-24 h of free faction in the plasma at the minimal efficacious dose was 2.0 µM•hr.

Significant inhibition of mTOR pathway markers pS6RP and pAKT (S473) in Compound 2-treated PC3 tumors suggests that the observed antitumor activity of Compound 2 was mediated through the inhibition of both the mTORC1 and mTORC2. Significant inhibition of pDNA-PK (S2056) was also observed in PC3 tumors treated with Compound 2. As the PC3 xenograft model is sensitive to mTOR inhibition, the contribution of pDNA-PK (S2056) inhibition with Compound 2 in the observed antitumor activity could not be determined in this model.

In PC3 tumors, the PK/PD relationship indicates that ≥ 80% inhibition of pS6RP and ≥ 65% inhibition of pAKT (S473) was obtained for total drug plasma concentrations greater than 0.12 µM. Maintenance of plasma levels > 0.12 µM and this degree of biomarker inhibition through 10 hours with once daily dosing or through 6 hours twice daily (BID dosing) confers good antitumor efficacy in PC3 tumors.

Immunohistochemical data demonstrate that the observed antitumor activity of Compound 2 was not only due to the inhibition of tumor cell proliferation but also due to the antiangiogenic activities of Compound 2 in vivo.

### ANTI-TUMOR ACTIVITY OF COMPOUND 1 IN CASTRATION RESISTANT LNCAP (LNCAP-HR) PROSTATE CANCER MODEL (disclosed as reference)

Xenograft study is conducted with castration resistant LNCaP-HR tumor-bearing mice. Castrated male SCID mice are inoculated subcutaneously with LNCaP-HR cells in the flank region above the right hind leg. Following inoculation of animals, the tumors are allowed to grow to about 300 mm³ prior to randomization. Three weeks following tumor cell inoculation, the mice bearing LNCaP-HR tumors ranging between 100 and 400 mm³ are pooled together and randomized into various treatment groups. Compound 1 is formulated in 0.5% CMC and 0.25% Tween 80 in water (as a suspension). The animals are orally administered vehicle (CMC-Tween) or Compound 1 once daily (QD) for up to 15 days. Doses of Compound 1 range between 5 and 20 mg/kg. The positive control MDV-3100 (50 mg/kg, Q4D) is administered via oral route. MDV-3100 is formulated in 1% CMC, 0.1% Tween 80 and 5% dimethyl sulfoxide (DMSO) in water (as a suspension). Tumors are measured twice a week using calipers and tumor volumes are calculated using the formula of W2 x L / 2. Statistical analysis is performed using a one-way analysis of variance (ANOVA) followed by Dunnett's post-hoc comparison with the vehicle-treated control group.

## Claims

1. Compound 1-ethyl-7-(2-methyl-6-(1H-1,2,4-triazol-3-yl)pyridin-3-yl)-3,4-dihydropyrazino[2,3-b]pyrazin-2(1H)-one for use in a method for treating prostate cancer, wherein the method comprises administering to a patient having prostate cancer 0.5 mg/day to 128 mg/day of said compound, and wherein the prostate cancer is not ETS overexpressing castration-resistant prostate cancer.

2. The compound for use of claim 1, wherein the method further comprises assessing inhibition of phosphorylation of S6RP, 4E-BP1 and/or AKT in a biological sample of the patient, and wherein the assessment comprises comparing the amount of phosphorylated S6RP, 4E-BP1 and/or AKT in a biological sample of said patient obtained prior to and after the administration of said compound, wherein less phosphorylated S6RP, 4E-BP1 and/or AKT in said biological sample obtained after said administration relative to the amount of phosphorylated S6RP, 4E-BP1 and/or AKT in said biological sample obtained prior to said administration indicates inhibition.

3. The compound for use of claim 1, wherein the method further comprises assessing inhibition of DNA-dependent protein kinase (DNA-PK) activity in a skin sample of the patient, and wherein the assessment comprises comparing the amount of phosphorylated DNA-PK in a skin sample of said patient obtained prior to and after the administration of said compound, wherein less phosphorylated DNA-PK in said skin sample obtained after said administration relative to the amount of phosphorylated DNA-PK in said skin sample obtained prior to said administration indicates inhibition.

4. The compound for use of claim 1, wherein the method further comprises measuring inhibition of phosphorylation of S6RP, 4E-BP1 or AKT in a biological sample of the patient, and wherein the measurement comprises measuring the amount of phosphorylated S6RP, 4E-BP1 or AKT in said patient after the administration of said compound and comparing said amount of phosphorylated S6RP, 4E-BP1 or AKT to that of said patient prior to said administration.

5. The compound for use of claim 1, wherein the method further comprises measuring inhibition of phosphorylation of DNA-PK S2056 in a skin sample of the patient, wherein the measurement comprises measuring the amount of phosphorylated DNA-PK S2056 present in the skin sample after the administration of said compound and comparing said amount of phosphorylated DNA-PK S2056 to that in a skin sample from said patient prior to said administration.

6. The compound for use of any one of claims 1 to 5, wherein the prostate cancer is that in which the PI3K/mTOR pathway is activated.

7. The compound for use of claim 6, wherein prostate cancer is that in which the PI3K/mTOR pathway is activated due to PTEN loss, a PIK3Ca mutation or EGFR overexpression, or a combination thereof.

## Patentansprüche

1. Verbindung 1-Ethyl-7-(2-methyl-6-(1H-1,2,4-triazol-3-yl)pyridin-3-yl)-3,4-dihydropyrazino[2,3-b]pyrazin-2(1H)-on zur Verwendung in einem Verfahren zur Behandlung von Prostatakrebs, wobei das Verfahren Verabreichen von 0,5 mg/Tag bis 128 mg/Tag der Verbindung an einen Patienten, der an Prostatakrebs leidet, umfasst und wobei der Prostatakrebs kein ETS-überexprimierender kastrationsresistenter Prostatakrebs ist.

2. Verbindung zur Verwendung nach Anspruch 1, wobei das Verfahren ferner das Beurteilen der Hemmung der Phosphorylierung von S6RP, 4E-BP1 und/oder AKT in einer biologischen Probe des Patienten umfasst und wobei die Beurteilung Vergleichen der Menge von phosphoryliertem S6RP, 4E-BP1 und/oder AKT in einer biologischen Probe des Patienten, die vor und nach der Verabreichung der Verbindung erhalten wurde, umfasst, wobei weniger phosphoryliertes S6RP, 4E-BP1 und/oder AKT in der biologischen Probe, die nach der Verabreichung erhalten wurde, relativ zu der Menge von phosphoryliertem S6RP, 4E-BP1 und/oder AKT in der biologischen Probe, die vor der Verabreichung erhalten wurde, eine Hemmung angibt.

3. Verbindung zur Verwendung nach Anspruch 1, wobei das Verfahren ferner das Beurteilen der Hemmung der Aktivität von DNA-abhängiger Proteinkinase (DNA-PK) in einer Hautprobe des Patienten umfasst und wobei die Beurteilung das Vergleichen der Menge von phosphorylierter DNA-PK in einer Hautprobe des Patienten, die vor und nach der Verabreichung der Verbindung erhalten wurde, umfasst, wobei weniger phosphorylierte DNA-PK in der Hautprobe, die nach der Verabreichung erhalten wurde, relativ zu der Menge von phosphorylierter DNA-PK in der Hautprobe, die vor der Verabreichung erhalten wurde, eine Hemmung angibt.

4. Verbindung zur Verwendung nach Anspruch 1, wobei das Verfahren ferner das Messen der Hemmung der Phosphorylierung von S6RP, 4E-BP1 oder AKT in einer biologischen Probe des Patienten umfasst und wobei die Messung das Messen der Menge von phosphoryliertem S6RP, 4E-BP1 oder AKT in dem Patienten nach der Verabreichung der Verbindung und Vergleichen der Menge von phosphoryliertem S6RP, 4E-BP1 oder AKT mit der des Patienten vor der Verabreichung umfasst.

5. Verbindung zur Verwendung nach Anspruch 1, wobei das Verfahren ferner das Messen der Hemmung der Phosphorylierung von DNA-PK S2056 in einer Hautprobe des Patienten umfasst, wobei die Messung das Messen der Menge von phosphorylierter DNA-PK S2056, die in der Hautprobe vorhanden ist, nach der Verabreichung der Verbindung und Vergleichen der Menge von phosphorylierter DNA-PK S2056 mit der in einer Hautprobe von dem Patienten vor der Verabreichung umfasst.

6. Verbindung zur Verwendung nach einem der Ansprüche 1 bis 5, wobei es sich bei dem Prostatakrebs um denjenigen handelt, in dem der PI3K/mTOR-Weg aktiviert ist.

7. Verbindung zur Verwendung nach Anspruch 6, wobei es sich bei dem Prostatakrebs um denjenigen handelt, in dem der PI3K/mTOR-Weg aufgrund eines PTEN-Verlusts, einer PIK3Ca-Mutation oder einer EGFR-Überexpression oder einer Kombination davon aktiviert ist.

## Revendications

1. Composé 1-éthyl-7-(2-méthyl-6-(1H-1,2,4-triazol-3-yl)pyridin-3-yl)-3,4-dihydropyrazino[2,3-b]pyrazin-2(1H)-one destiné à une utilisation dans un procédé de traitement du cancer de la prostate, dans lequel le procédé comprend l'administration à un patient atteint d'un cancer de la prostate de 0,5 mg/jour à 128 mg/jour dudit composé, et dans lequel le cancer de la prostate n'est pas un cancer de la prostate résistant à la castration surexprimant l'ETS.

2. Composé destiné à une utilisation selon la revendication 1, dans lequel le procédé comprend en outre l'évaluation de l'inhibition de la phosphorylation de S6RP, de 4E-BP1 et/ou d'AKT dans un échantillon biologique du patient, et dans lequel l'évaluation comprend la comparaison de la quantité de S6RP, de 4E-BP1 et/ou d'AKT phosphorylés dans un échantillon biologique dudit patient obtenu avant et après l'administration dudit composé, dans lequel une quantité inférieure de S6RP, de 4E-BP1 et/ou d'AKT phosphorylés dans ledit échantillon biologique obtenu après ladite administration par rapport à la quantité de S6RP, de 4E-BP1 et/ou d'AKT phosphorylés dans ledit échantillon biologique obtenu avant ladite administration indique une inhibition.

3. Composé destiné à une utilisation selon la revendication 1, dans lequel le procédé comprend en outre l'évaluation de l'inhibition de l'activité de la protéine kinase dépendant de l'ADN (ADN-PK) dans un échantillon de peau du patient, et dans lequel l'évaluation comprend la comparaison de la quantité d'ADN-PK phosphorylée dans un échantillon de peau dudit patient obtenu avant et après l'administration dudit composé, dans lequel une quantité inférieure d'ADN-PK phosphorylée dans ledit échantillon de peau obtenu après ladite administration par rapport à la quantité d'ADN-PK phosphorylée dans ledit échantillon de peau obtenu avant ladite administration indique une inhibition.

4. Composé destiné à une utilisation selon la revendication 1, dans lequel le procédé comprend en outre la mesure de l'inhibition de la phosphorylation de S6RP, de 4E-BP1 ou d'AKT dans un échantillon biologique du patient, et dans lequel la mesure comprend la mesure de la quantité de S6RP, de 4E-BP1 ou d'AKT phosphorylés chez ledit patient après l'administration dudit composé et la comparaison de ladite quantité de S6RP, de 4E-BP1 ou d'AKT phosphorylés à celle dudit patient avant ladite administration.

5. Composé destiné à une utilisation selon la revendication 1, dans lequel le procédé comprend en outre la mesure de l'inhibition de la phosphorylation de l'ADN-PK S2056 dans un échantillon de peau du patient, dans lequel la mesure comprend la mesure de la quantité d'ADN-PK S2056 phosphorylée présente dans l'échantillon de peau après l'administration dudit composé et la comparaison de ladite quantité d'ADN-PK S2056 phosphorylée à celle dans un échantillon de peau dudit patient avant ladite administration.

6. Composé destiné à une utilisation selon l'une quelconque des revendications 1 à 5, dans lequel le cancer de la prostate est celui dans lequel la voie de PI3K/mTOR est activée.

7. Composé destiné à une utilisation selon la revendication 6, dans lequel le cancer de la prostate est celui dans lequel la voie de PI3K/mTOR est activée en raison d'une perte de PTEN, d'une mutation de PIK3Ca ou d'une surexpression d'EGFR, ou d'une combinaison de celles-ci.
